# EUROPEAN PATENT APPLICATION

(11) **EP 4 782 538 A2**
(43) Date of publication of application: **29.07.2026**
(21) Application number: 26171673.2
(22) Date of filing: 30.09.2022
(51) Int. Cl.: C12N 15/09

(54) **HUMAN HEPATOCYTE CULTURE MEDIUM AND CONDITIONED MEDIUM OF IN VITRO CULTURED HUMAN HEPATOCYTES AND USES THEREOF**

(30) Priority: 30.09.2021 US 202163250529 P; 30.09.2021 US 202163250541 P
(62) Divisional of application: 22877601.9
(71) Applicant: THE UNIVERSITY OF SOUTHERN CALIFORNIA, Los Angeles, CA 90015 (US); PhoenixBio Co., Ltd., Hiroshima 739-0046 (JP)
(72) Inventor: SAITO, Takeshi, California, 90015 (US); ISHIDA, Yuji, Higashi-Hiroshima, 739-0046 (JP); SUGAHARA, Go, Higashi-Hiroshima, 739-0046 (JP); TATENO-MUKAIDANI, Chise, Higashi-Hiroshima, 739-0046 (JP); YAMASAKI, Chihiro, Higashi-Hiroshima, 739-0046 (JP)
(74) Representative: J A Kemp LLP

(57) **Abstract**

HH culture media and methods are provided to allow for recovery from injury or stress associated with cell isolation procedures and freeze/thaw cycle(s) by the use of a DMSO-supplemented medium, i.e., Phase 1 (recovery-phase). Moreover, HH culture media and methods are provided to allow for support of hepatic functionality of cultured HH at a comparable level to that of the human liver by the use of a DMSO, DMSO2, or TMSO-supplemented medium, i.e., Phase 2 (maintenance-application phase). These HH are suitable for the *in vitro* study of liver biology, diseases drug metabolism and pharmacokinetics. Furthermore, the culture supernatant of HH during Phase 2, namely, conditioned culture medium of human hepatocytes (CMHH), is provided, which facilitates iHeps maturation and supports the cell fate maintenance of hepatocytes. Methods of preparing CMHH are also provided, including culturing humanized liver chimeric animal derived-human hepatocytes and/or primary human hepatocytes in a medium to be collected.

## Description

### CROSS-REFERENCE TO RELATED APPLICATIONS

This Application claims benefit under 35 U.S.C. §119(e) of U.S. Provisional Application No. 63/250,529 filed on September 30, 2021 and U.S. Provisional Application No. 63/250,541 filed on September 30, 2021, the contents of each of which are incorporated herein by reference in their entireties.

### FIELD OF INVENTION

This invention relates to cell culture medium and uses thereof.

### BACKGROUND

In vitro cultured non-cancerous, terminally differentiated human hepatocytes (HH) have been the mainstay in vitro experimental platform for the study of liver biology as well as various liver diseases. HH has also been a fundamental tool for the preclinical assessment of drug metabolism and pharmacokinetics (DMPK), including but not limited to evaluating drug toxicity of newly developed clinical compounds. The global market size of HH for research use is valued at 50 million dollars and is projected to grow steadily.

To date, however, the usability of HH has not yet reached the level that satisfies the demand from researchers. The shortfall is primarily due to the lack of a sophisticated culturing or maintenance method of HH *in vitro,* given the injury and stress on the cells resulting from the cell procurement processes including the cold-warm ischemia, the isolation procedures with collagenase perfusion, and the freeze/thaw storage process, which leads to a rapid loss of the cell quality. As a result, there is undesirable performance/functionality of *in vitro* cultured HH in the study of liver cell biology, various liver diseases, and DMPK.

Furthermore, there is a lack of a definitive technology that supports long-term cell fate maintenance of matured HH *in vitro,* which impedes our understanding of the molecular/cellular biology of the liver. In addition, there is a lack of a definitive technology that supports the complete maturation of stem cell or iPS cell-derived HH, referred to as iHep. Improving long-term cell fate maintenance of matured HH in vitro and complete maturation of iHep would lead to successful establishment of hepatocyte transplantation therapy.

Currently, liver transplantation (LT) is the only therapy for end-stage liver diseases, such as decompensated cirrhosis and liver cancer that result from various types of chronic liver diseases such as chronic viral hepatitis (HBV and HCV), alcoholic liver disease (ALD), non-alcoholic fatty liver disease (NAFLD), as well as numerous genetic diseases of the liver. However, LT is an invasive procedure, and the nationwide organ shortage greatly limits its utility. In addition, LT recipients are required to receive lifelong immunosuppressive therapy, which is associated with significant morbidity, including the development of opportunistic infections and neoplasms.

Hepatocyte transplantation technology is expected to replace LT therapy in the future; however, there remain numerous technical hurdles to overcome. One of the most significant technical barriers is the lack of a definitive method that allows for the complete maturation iHeps. The degree of the maturation of currently available iHeps are still far below compared to that of primary human hepatocytes (PHH). Indeed, iHep is rather a heterogeneous cell population consist of both immature hepatocytes as well as cholangiocytes-like cells, indicating that the degree of iHep maturation is at the stage of hepatic bipotential progenitor cells. The degree of hepatocytes maturation can be assessed by the expression abundance of "hepatocyte marker genes" as well as its capacity to proliferate in the liver of immunodeficient mice expressing toxic gene in the endogenous murine hepatocytes (the mouse strain used for the production of humanized liver chimeric mice (HLCM)). Accordingly, currently available iHeps are incapable of proliferating in the liver of HLCM-host mice. Thus, there remains a significant unmet need for the technology that enables the final maturation process of liver progenitor cells including iHeps.

All publications herein are incorporated by reference to the same extent as if each individual publication or patent application was specifically and individually indicated to be incorporated by reference. The following description includes information that may be useful in understanding the present invention. It is not an admission that any of the information provided herein is prior art or relevant to the presently claimed invention, or that any publication specifically or implicitly referenced is prior art.

### BRIEF DESCRIPTION OF THE FIGURES

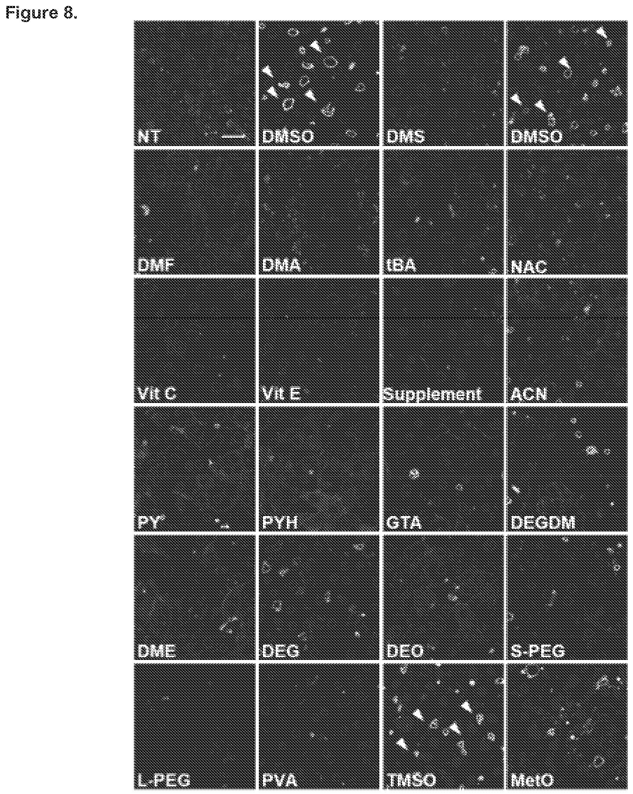

Exemplary embodiments are illustrated in referenced figures. It is intended that the embodiments and figures disclosed herein are to be considered illustrative rather than restrictive.

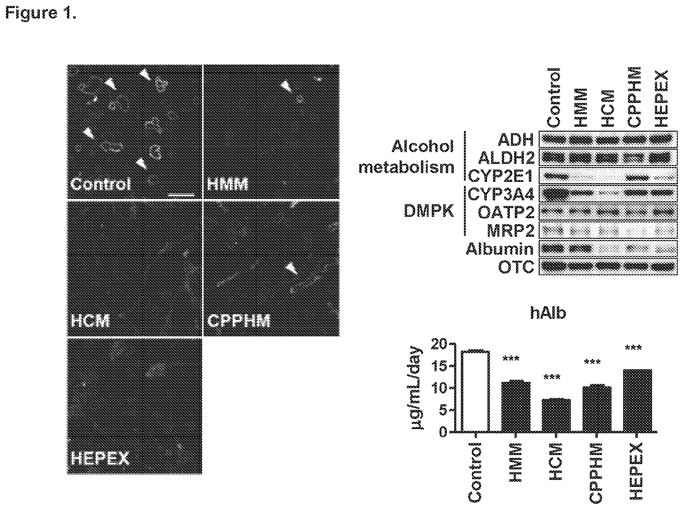

**Fig. 1** **shows a comparison of hepatocytes culture medium:** Freshly isolated HHs were cultured for 7 days with HH recovery medium (dHCGM: Control) or commercially available hepatocyte culture medium. Then, HH was subjected to: (Left) Immunofluorescent microscopic analysis for the detection of MRP2 (Arrowheads) (bar indicates 25 µm), (Right, Top) immunoblotting analysis for the detection of indicated hepatocyte marker genes, or (Right Bottom) quantification of human albumin in the culture supernatants. Graph bars represent mean ± SD; *** indicates p < 0.001 by one - way ANOVA.

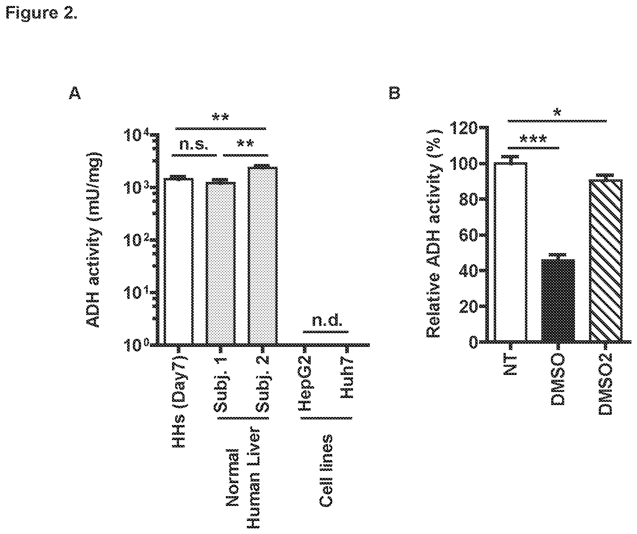

**Figs. 2A-2B** **shows a comparison of ADH activity in cultured HHs and normal human tissue, and DMSO inhibition of ADH activity:** (A) Cell/tissue homogenate of HH or human liver were subjected to the measurement of NADH production rate as a surrogate of ADH activity via the detection of absorbance at 340nm. Hepatoma cells (Huh7 and HepG2) were used as negative controls. (B) The relative ADH activity of HH were assessed in the absence of organic compounds (NT), as well as in the presence of DMSO or DMSO2 using the same method described in (A). Graph bars represent mean ± SD; *, ** and *** indicate *p* < 0.05 and *p* < 0.01 and *p* < 0.001 by one - way ANOVA; n.s., not significant; n.d., not detected.

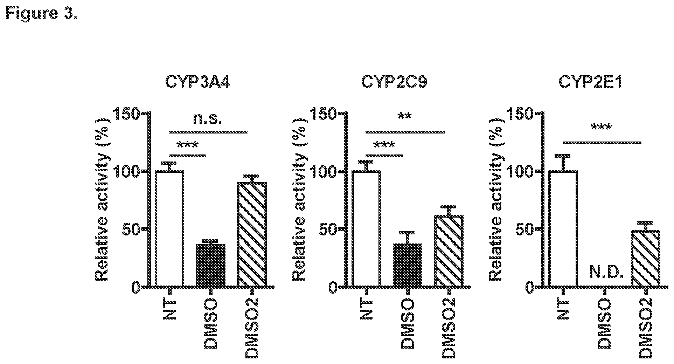

**Fig. 3** **shows DMSO inhibition of DMPK enzymes:** Freshly isolated HHs were cultured with DMSO containing medium (the recovery medium) for 7 days. Then, cells were used for the assessment of CYP3A4 (left), and CYP2A9 (middle) activity in the presence of organic compounds (DMSO or DMSO2) using P450-GloTM Assays (Promega). For the assessment of CYP2E1 activity (right) was assessed using VividTM CYP2E1 Blue Screening Kit (Thermo Fisher). Graph bars represent mean ± SD; *, ** and *** indicate *p* < 0.05 and *p* < 0.01 and *p* < 0.001 by one - way ANOVA; n.s., not significant; n.d., not detected.

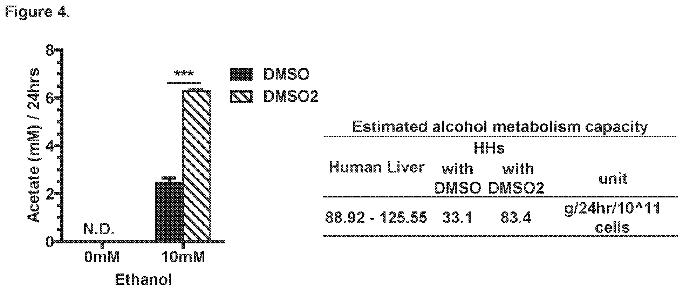

**Fig. 4** **shows DMSO inhibition of ethanol metabolism to the production of acetate:** Freshly isolated HHs were cultured for 7 days with HH recovery media. Then, HH were cultured with either DMSO or DMSO2 containing maintenance/application medium in the absence (0mM) or presence of 10mM ethanol for 24 hours. The sealing apparatus was applied throughout the course of ethanol treatment. At the end of the ethanol treatment, cell culture medium was harvested followed by the measurement of acetate concentration using Acetate Colorimetric Assay Kit (Biovision). Graph bars represent mean ± SD; *** indicates*p* < 0.001 by one - way ANOVA; n.d., not detected. The rate of acetate production was normalized by the cell number of the human liver in order to assess the comparability of EtOH metabolizing capacity of HH with each given condition to that of the health human liver (right).

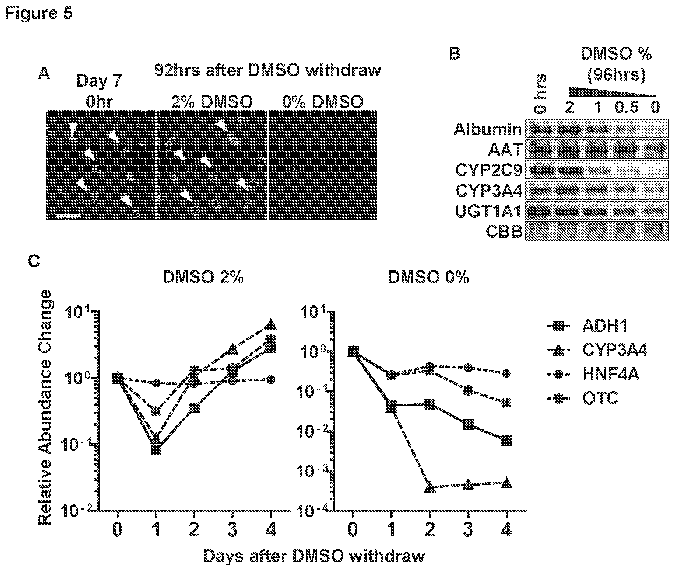

**Figs. 5A-5C** **shows withdrawal of DMSO promotes hepatocyte cell fate loss:** Freshly isolated HHs were cultured for 7 days with HH recovery media followed by the incubation with medium containing indicated concentration of DMSO for additional 4 days. Then, HH were subjected to (A) immunofluorescent analysis for the detection of MRP2 (Arrowheads), (B) immunoblotting and (C) qPCR analysis for the detection of indicated hepatocytes marker molecules. The bar indicates 25 µm.

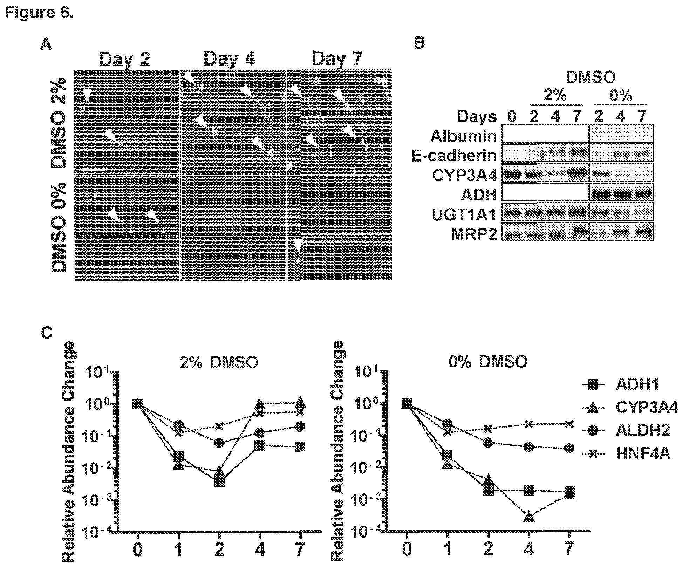

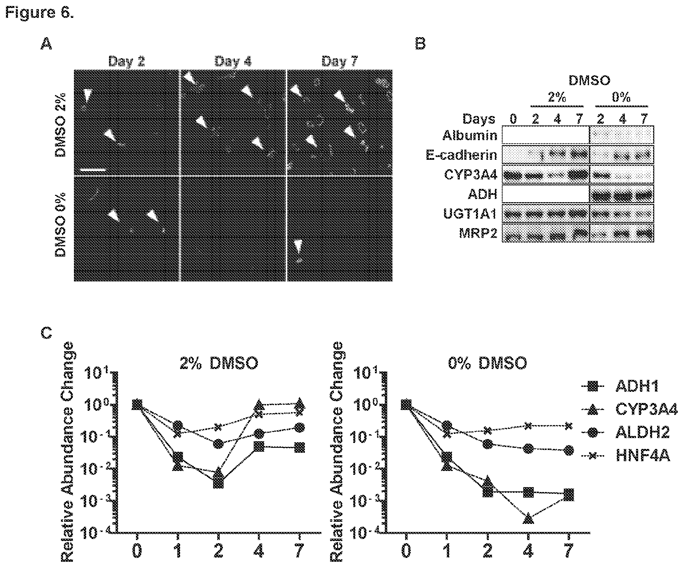

**Figs 6A-6C** **shows the role of DMSO in the cell fate recovery of HH:** Freshly isolated HHs were cultured for 7 days with medium containing indicated concentration of DMSO. Then, HH were subjected to (A) immunofluorescent analysis for the detection of MRP2 (Arrowheads), (B) immunoblotting and (C) qPCR analysis for the detection of indicated hepatocytes marker molecules at the indicated time points.

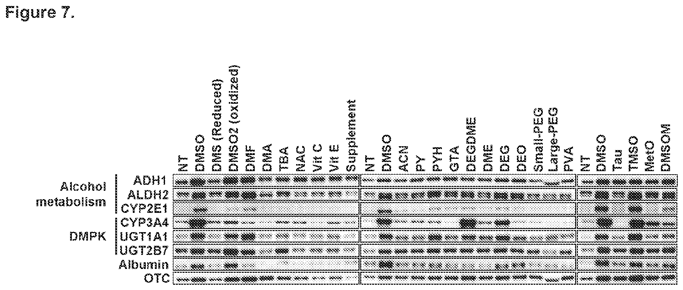

**Fig. 7** **shows the criticalness of organic compounds in the maintenance of hepatocyte marker genes expression:** Freshly isolated HHs were cultured for 7 days with HH recovery media followed by the maintenance/application medium containing indicated organic compound for another 7 days. Then, total cell lysates were subjected to immunoblotting analysis for the detection of indicated molecules. NT; non treatment.
[**0017]** **Fig. 8** **shows the criticalness of organic compounds in the morphological maintenance of human hepatocytes:** Freshly isolated HHs were cultured for 7 days with HH recovery media followed by the incubation with maintenance/application medium containing indicated organic compound for another 7 days. Then, HH was subjected to immunofluorescent analysis for the detection of MRP2 (Arrowheads), F-actin, DAPI. The bar indicates 25 µm.

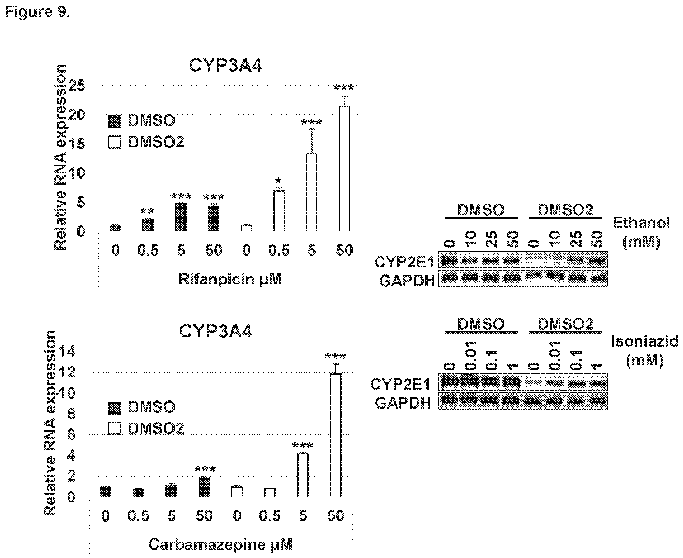

**Fig. 9** **show the impact of DMSO on the inducibility of CYP genes.** Freshly isolated HHs were cultured for 7 days with HH recovery media (dHCGM) followed by the maintenance/application medium containing DMSO or DMSO2 for another 7 days in the presence of indicated concentration of CYP3A4 inducers (rifanpicin and carbamazepine) (left) and CYP2E1 inducers (ethanol and isoniazid) (right). Then, and total RNA and cell lysates were subjected to immunoblotting analysis (left) and qPCR analysis (right) to determine the inducibility of indicated molecules. Graph bars represent mean ± SD; *, ** and *** indicate *p* < 0.05 and *p* < 0.01 and *p* < 0.001 by one - way ANOVA.

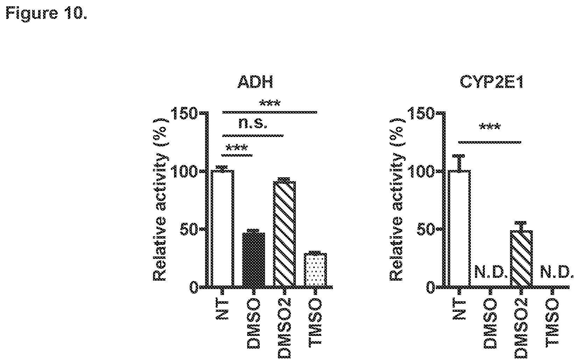

**Fig. 10** **shows TMSO inhibition of alcohol metabolism enzymes:** Cell homogenate of HH was subjected to the measurement of NADH production rate as a surrogate of ADH activity via the detection of absorbance at 340nm in the presence of organic compounds (DMSO, DMSO2 or TMSO). For the assessment of CYP2E1 activity (right) was assessed using VividTM CYP2E1 Blue Screening Kit (Thermo Fisher). Graph bars represent mean ± SD; *** indicates *p* < 0.001 by one - way ANOVA; n.s., not significant; n.d., not detected.

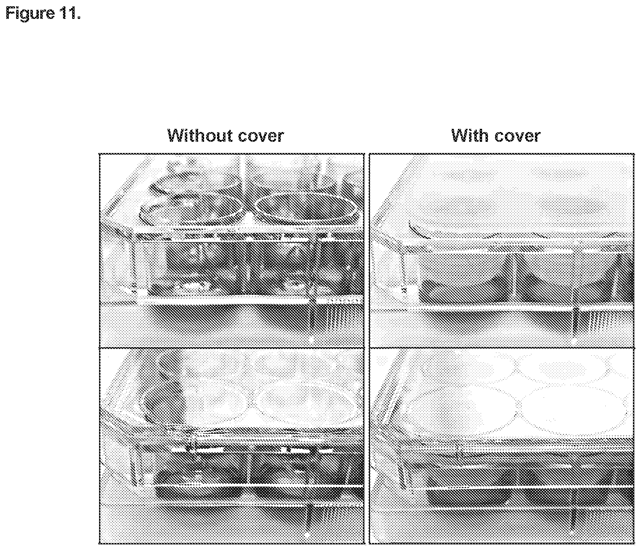

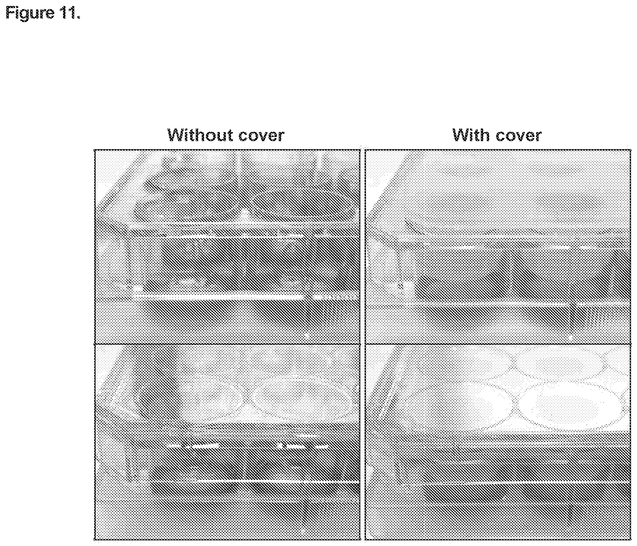

**Fig. 11** **shows application of tight sealing apparatus for the ethanol treatment of HH:** The tight silicone sealing apparatus was applied to the HH culture dish to minimize the evaporative loss of ethanol from culture medium. Left images are representative of a standard cell culture dish set up and the images on the right demonstrates the same cell culture dish upon application of the tight-sealing apparatus (top: without the lid, and bottom with the lid).

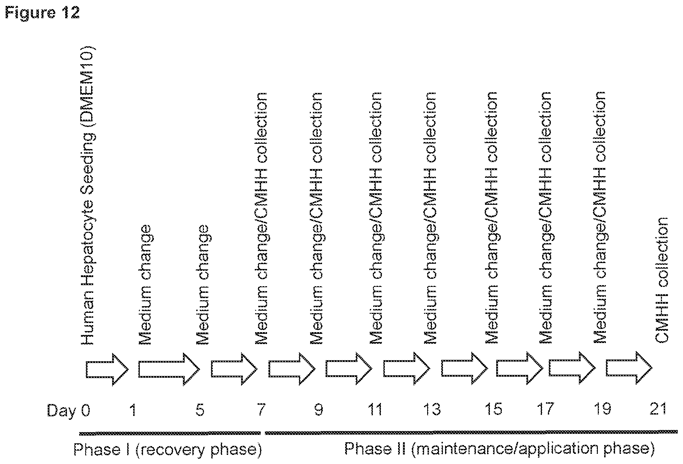

**Fig. 12** **shows CMHH Preparation Scheme.** Human hepatocytes (e.g., PHH and HLCM-HH) in suspension medium (e.g., DMEM10) are plated on to cell culture dish coated with type I collagen at the optimized seeding cell number to attain the adherent cell density > 0.5x10⁵ cells/cm². 24 hours after the seeding, the plating medium (e.g., DMEM10) will be replaced with the hepatocyte culture medium (DMSO-containing dHCGM), which will be replaced every 2-4 days for up to 7 days to complete the recovery phase culture. Then, during the maintenance/application phase, cells undergo repetitive medium change and collection of cell culture supernatant (CMHH) every 48 hours as shown above. The recovered CMHH will be refrigerated or frozen at - 20 C or - 80 C until use.

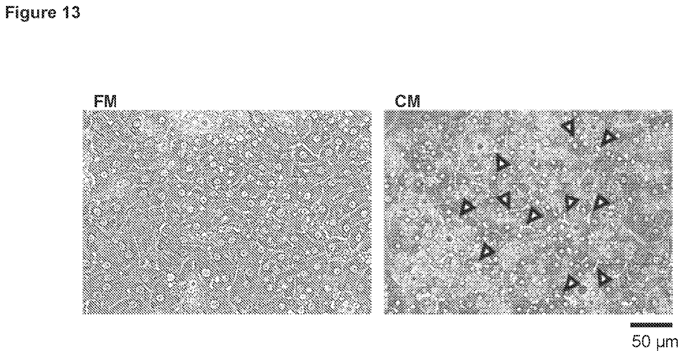

**Fig. 13** **shows biological effect of CMHH on the cellular architecture of HH.** Freshly isolated HLCM-HH (Lot: JFC) were plated on type I collagen-coated wells at 1.05x10⁵ cells/cm². The plating medium (DMEM10) was replaced with fresh dHCGM (FM) (left) or CMHH (right) 30-60 min after cell seeding followed by medium change (either FM or CMHH) for every 24 hours until day 7. Then cells were subjected to light microscopic analysis. The arrowheads point the bile canaliculi formed between the polygonal-cuboidal appearing hepatocytes, which are characteristics of terminally differentiated human hepatocytes.

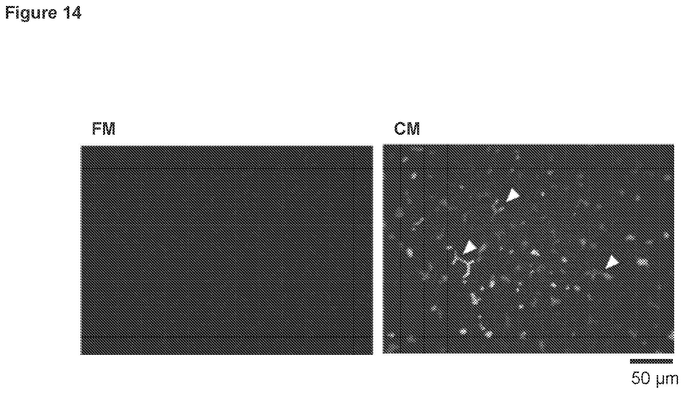

**Fig. 14** **shows functional assessment of bile secretion machinery.** Freshly isolated HLCM-HH (Lot: JFC) were cultured with FM or CMHH as described in Fig. 13. On 7 days post seeding, cells were washed with Hanks Balanced Salt Solution (HBSS) and incubated with HBSS containing 1.25 nM of Carboxy-DCFDA (5-(and-6)-Carboxy-2',7'-Dichlorofluorescein (CDFDA), a fluorescent substrate specific for MRP2, for 10 min at 37°C followed by wash with HBSS (3 times) and incubation with HBSS for 10 min at 37°C. Then cells were subjected to fluorescent microscopic analysis for the detection of bile canaliculi filled with CDFDA (arrowheads).

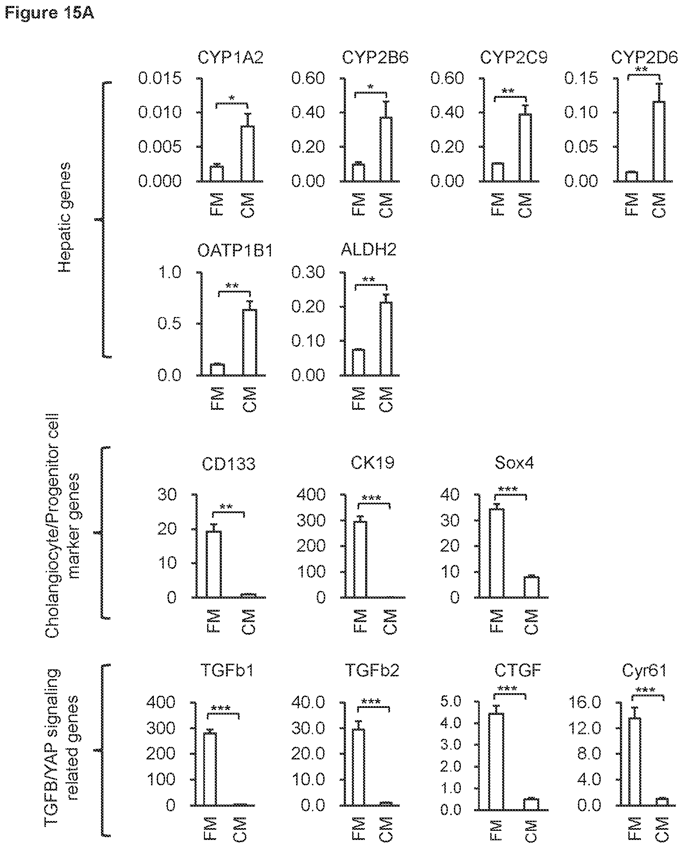

**Fig. 15A** **shows the biological impact of CMHH on the cell fate of HH.** Freshly isolated HLCM-HH (Lot: JFC) were cultured with fresh dHCGM (FM) or CMHH (CM) as described in Figure 13. On 7 days post seeding, total RNA was extracted using Quick RNA micro prep (ZymoReserach) followed by synthesis with cDNA superMix kit (Quanta Bio). cDNA was then subjected to Real-time qPCR for the relative quantification of indicated genes. Bars represent mean ± SD of relative expression levels to that of freshly isolated HLCM-HH; *, **, and *** indicate *p <* 0.01, *p <* 0.001, and *p <* 0.0001 by unpaired t test.

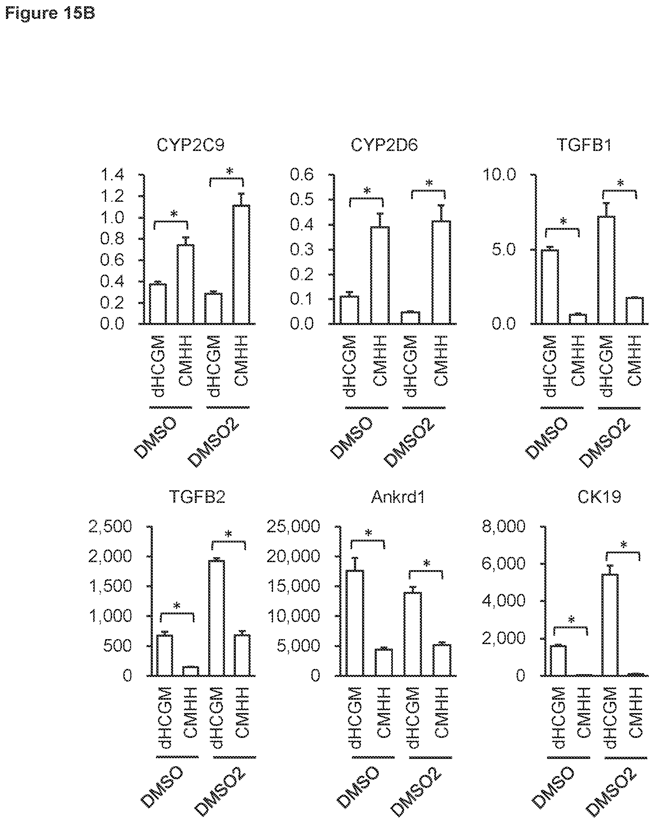

**Fig. 15B** **shows the biological impact of DMSO- and DMSO2-containing CMHH on the cell fate of HH** Freshly isolated HLCM-HH were cultured with either fresh dHCGM or CM containing DMSO or DMSO2 for 7 days. Subsequently, total RNA was extracted using Quick RNA micro prep (ZymoReserach) followed by synthesis with cDNA superMix kit (Quanta Bio). cDNA was then subjected to Real-time qPCR for the relative quantification of indicated genes. Bars represent mean ± SD of relative expression levels to that of freshly isolated HLCM-HH; Statistical analysis was performed with Tukey's multiple comparison test, *P <0.0001.

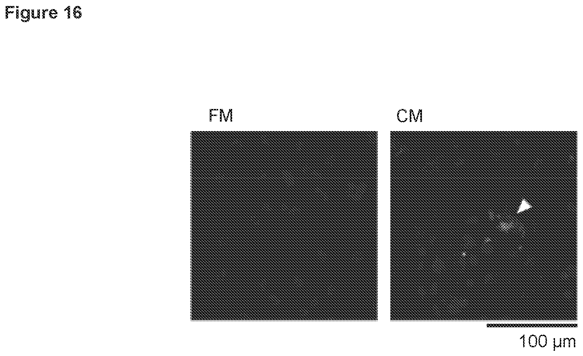

**Fig. 16** **shows the effect of CMHH on HH susceptibility to HBV infection.** Freshly isolated HLCM-HH (Lot: JFC) were cultured with dHCGM (FM) or CMHH (CM) as described in Figure 13. HLCM-HH incubated with either FM or CM for 7 days were then inoculated with hepatitis B virus (HBV) (genotype A) at 5 Geq/cells with the method established in previous study (PMID: 25791527). HBV-infected cells were cultured with FM or CM with medium change every 2 days for additional 14 days followed by immunofluorescent microscopic analysis for the detection of HBV surface antigen (HBsAg) using anti-HBsAg antibody and alexa-488 labeled anti-mouse antibody. An arrowhead indicated the active infection of HBV in CM treated HLCM-HH.

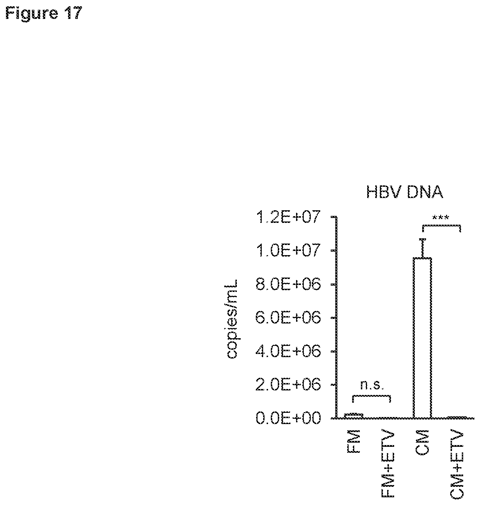

**Fig. 17** **shows the effect of antiviral therapy against HBV infection.** Freshly isolated HLCM-HH (Lot: JFC) were cultured with dHCGM (FM) or CMHH (CM) as described in Figure 13. HLCM-HH incubated with either FM or CM for 7 days were then inoculated with hepatitis B virus (HBV) (genotype A) at 5 Geq/cells with the method described in previous study (PMID: 25791527). HBV-infected cells were cultured with FM or CM with medium change every 2 days for additional 14 days in the presence or absence of entecavir (ETV) (250 nM). At the endpoint, the culture supernatant was subjected to viral DNA extraction using QIAamp MinElute Virus Spin Kit (Qiagen) followed by TaqMan probe based 1 step RT-qPCR analysis for the quantification of HBV genome. Bars represent mean ± SD; *** indicate *p* < 0.0001 by one-way ANOVA and Tukey's multiple comparison test.

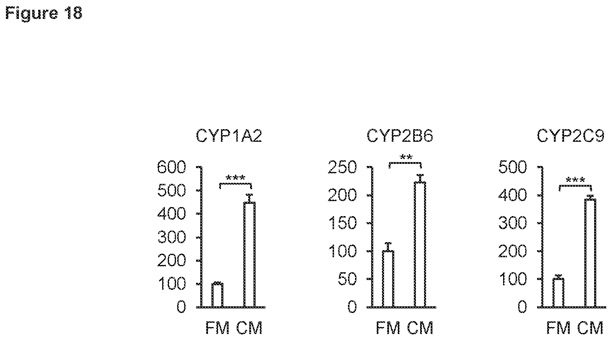

**Fig. 18** **shows the impact of CMHH on the xenobiotics metabolizing pathways of HH.** Freshly isolated HLCM-HH (Lot: JFC) were cultured with dHCGM (FM) or CMHH (CM) as described in Figure 13. HLCM-HH incubated with either FM or CM for 7 days were then subjected to the assessment of indicated CYP enzymes using P450-Glo assay (Promega). Bars represent mean ± SD of relative expression levels to that of freshly isolated HLCM-HH; **, and *** indicate *p* < 0.001, and *p* < 0.0001 by unpaired t test.

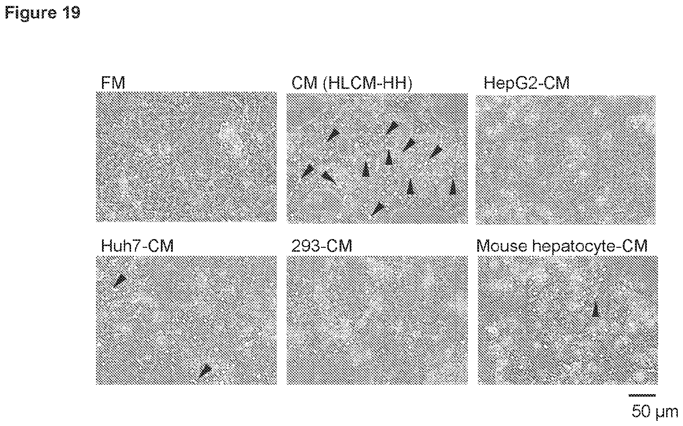

**Fig. 19** **shows the biological function of CM derived from various cell** types. CM propagated with HLCM-HH, HepG2, HuH7, 293 cells, and mouse primary hepatocytes with the schedule described in Figure 13, which were then applied for the culture of HLCM-HH for 7 days followed by light microscopic analysis. The arrows point to the bile canaliculi developed between the polygonal-cuboidal-appearing hepatocytes, which are a feature of terminally differentiated human hepatocytes.

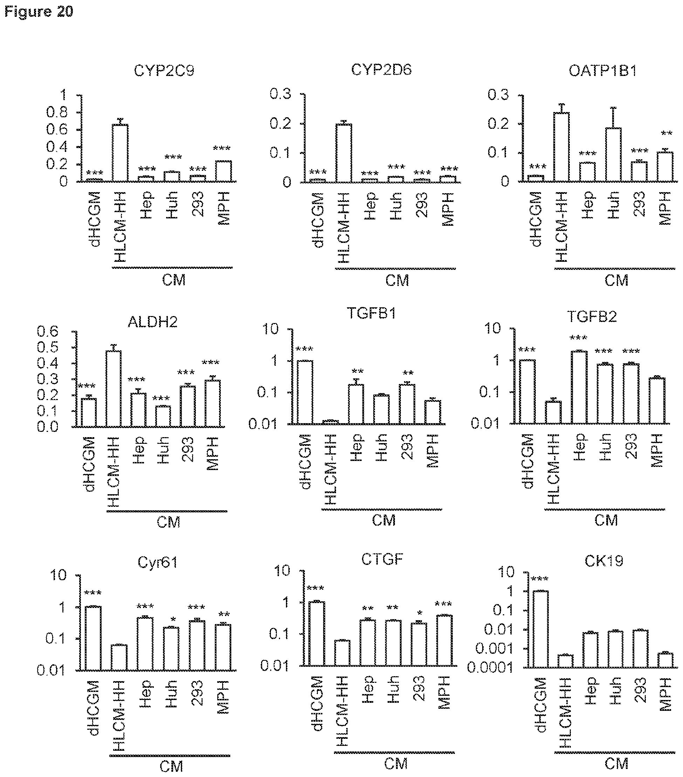

**Fig. 20** **shows the effect of CM on HLCM-HH.** HLCM-HH cultured with CM propagated with indicated cell types for 7 days were subjected to RT-qPCR analysis for the assessment of indicated genes. Bars represent relative expression levels to that in freshly isolated HLCM-HH (for CYP2C9, 2D6 ALDH2 and OATP1B1) or in HLCM-HH cultured with fresh dHCGM (FM) (for TGFB1, TGFB2, Cyr61, CTGF, and CK19). Statistical analysis was performed with Tukey's Multiple comparison test, vs CMHH, *P<0.01, **P,0.001, ***P<0.0001.

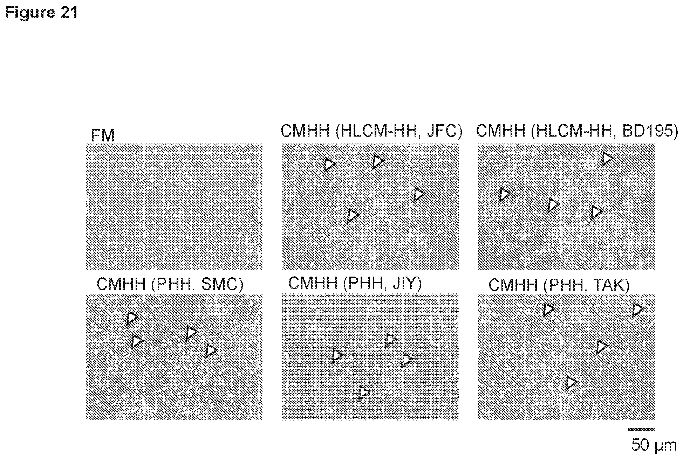

**Fig. 21** **shows the effect of CM propagated with variable cell types on the cell fate marker of HH.** CMHH propagated with HLCM-HH derived from two different donors (JFC and BD195) or with commercially available cryopreserved primary human hepatocytes from three different donors (SMC, JIY, and TAK) with the schedule described in Fig. 13 were used for the culture of freshly isolated HLCM-HH for 7 days. The morphological architecture of the cells was subsequently evaluated by light microscopy. The arrowheads indicate the bile canaliculi formed between the polygonal-cuboidal appearing human hepatocytes, which are characteristics of terminally differentiated human hepatocytes.

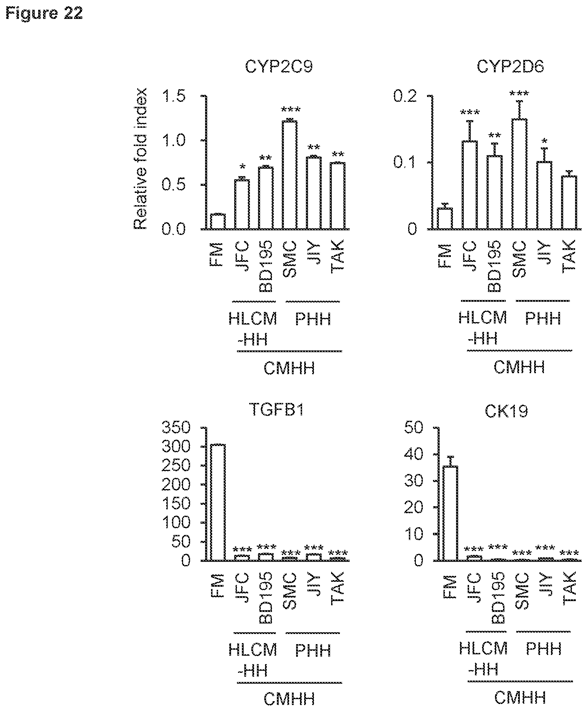

**Fig. 22** **shows the biological effect of CM propagated with HH from multiple donors.** Freshly isolated HLCM-HH cultured with either fresh dHCGM (FM), CMHH propagated with HLCM-HH (two different donors) or primary human hepatocytes (three different donors) for 7 days as described in Figure 13 followed by RT-qPCR analysis for the detection of indicated genes. The relative expression abundance was determined by normalization with the expression abundance in freshly isolated HLCM-HH. Statistical analysis was performed with Tukey's multiple comparison test. * P<0.01, **P<0.001, **P<0.0001.

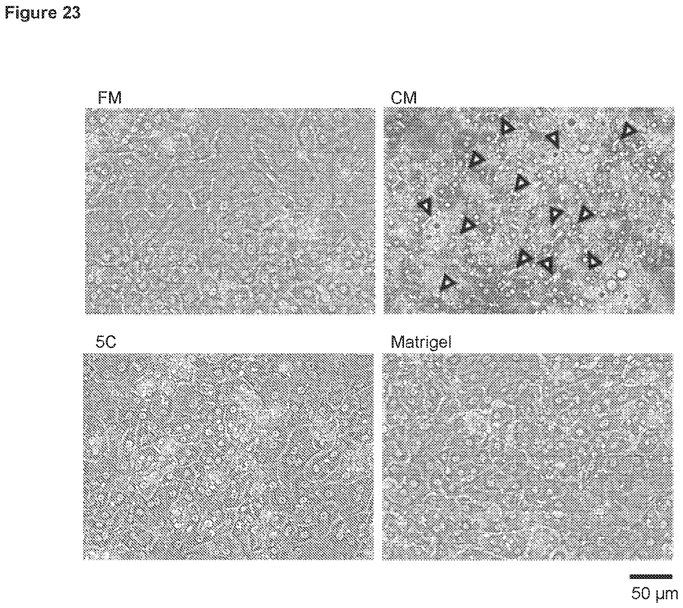

**Fig. 23** **shows the comparison of the biological effect of CM, 5Cn and Matrigel^{®} treatment.** Freshly isolated HLCM-HH (Lot: JFC) were plated as described in Figure 13 and cultured with dHCGM (FM) or CMHH (CM). Alternatively, cells were cultured with fresh dHCGM supplemented with 5C or **Matrigel^{®}** at concentrations described in previous reports (PMID: 31023926, 16469405). The arrowheads indicate the bile canaliculi formed between the polygonal-cuboidal appearing human hepatocytes, which are characteristics of terminally differentiated human hepatocytes.

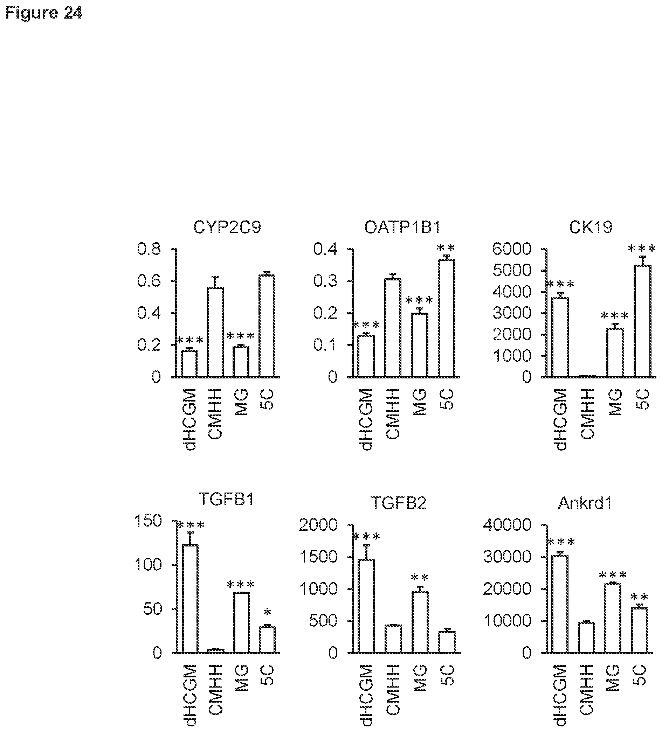

**Fig. 24** **shows the comparison of the biological effect of CM, 5Cn and Matrigel^{®} treatment.** Freshly isolated HLCM-HH (Lot: JFC) were plated and cultured with fresh dHCGM, CMHH, fresh dHCGM supplemented with 5C or Matrigel^{®}, as described in Figure 23, followed by RT-qPCR analysis for the relative quantification of indicated genes. Statistical analysis was performed with Tukey's multiple comparison test. * P<0.01, **P<0.001, **P<0.0001.

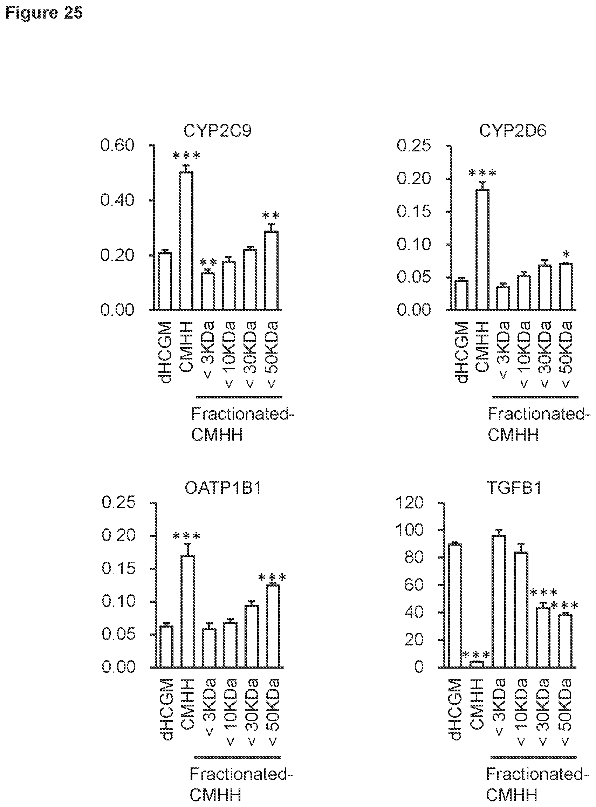

**Fig. 25** **shows the impact of size exclusion column filtration on biological effect of CMHH.** Freshly isolated HLCM-HH were cultured with either CMHH or CMHH filtered through an increasing cutoff-size exclusion column for 7 days, followed by RT-qPCR analysis for the detection of indicated genes. The relative abundance was determined by normalizing with the expression abundance of corresponding gene in freshly isolated HLCM-HH. Statistical analysis was performed with Tukey's multiple test, * P<0.01, ** P<0.001, **P<0.0001.

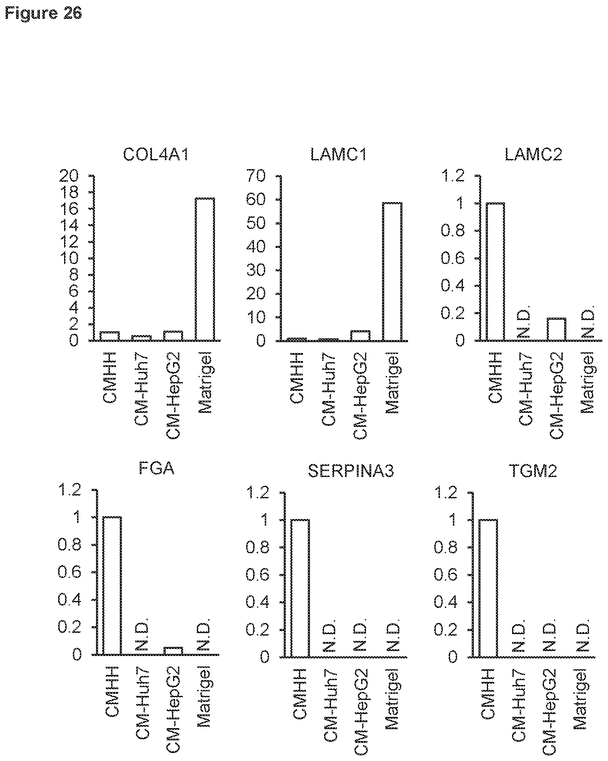

**Fig. 26** **shows the compositional analysis of CMHH.** dHCGM supplemented with Matrigel^{®} with the schedule described in Figure 13 were used for the relative or absolute quantification of specified humoral factors via the measurement of immunoblotting signal using Image J software and ELISA, respectively. The assessment of relative abundance was carried out using equal volume of CM propagated with indicated cell types or dHCGM supplemented with Matrigel^{®} containing the equal total protein quantity to that of CMHH. Bars represent relative protein levels to CMHH.

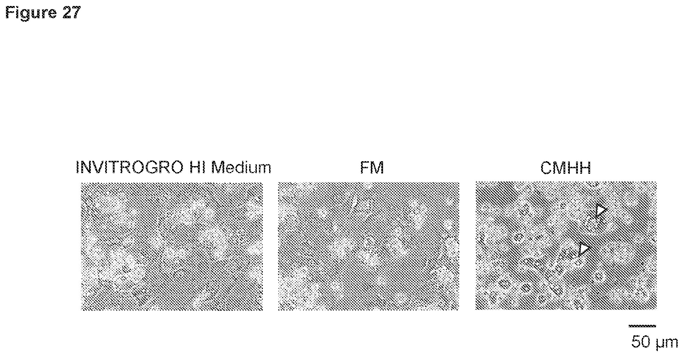

**Fig. 27** **shows the biological effect of CMHH on the morphology of cryopreserved primary human hepatocytes.** Commercially available primary human hepatocytes (Lot: GNA, BioIVT) were cultured with either commercially available hepatocyte culture medium (INVITROGRO HI Medium, BioIVT), fresh dHCGM, or CMHH for 7 days with the schedule described in Fig. 13. The morphological architecture of the cells was subsequently evaluated by light microscopy. The arrowheads indicate the bile canaliculi formed between the polygonal-cuboidal appearing human hepatocytes, which are characteristics of terminally differentiated human hepatocytes.

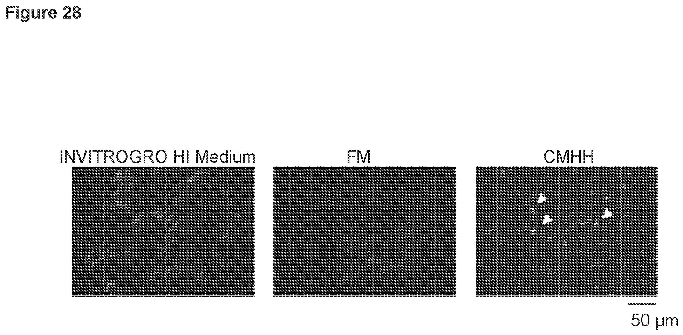

**Fig. 28** **shows the effect of CMHH on the bile secretion machinery of cryopreserved primary human hepatocytes** Commercially available primary human hepatocytes (Lot: GNA, BioIVT) were cultured with either commercially available hepatocyte culture medium (INVITROGRO HI Medium, BioIVT), fresh dHCGM, or CMHH for 7 days with the schedule described in Figure 13. On 7 days post seeding, cells were washed with Hanks Balanced Salt Solution (HBSS) and incubated with HBSS containing 1.25 nM of Carboxy-DCFDA (5-(and-6)-Carboxy-2',7'-Dichlorofluorescein (CDFDA), a fluorescent substrate specific for MRP2, for 10 min at 37°C followed by wash with HBSS (3 times) and incubation with HBSS for 10 min at 37°C. Then cells were subjected to fluorescent microscopic analysis for the detection of bile canaliculi filled with CDFDA (arrowheads).

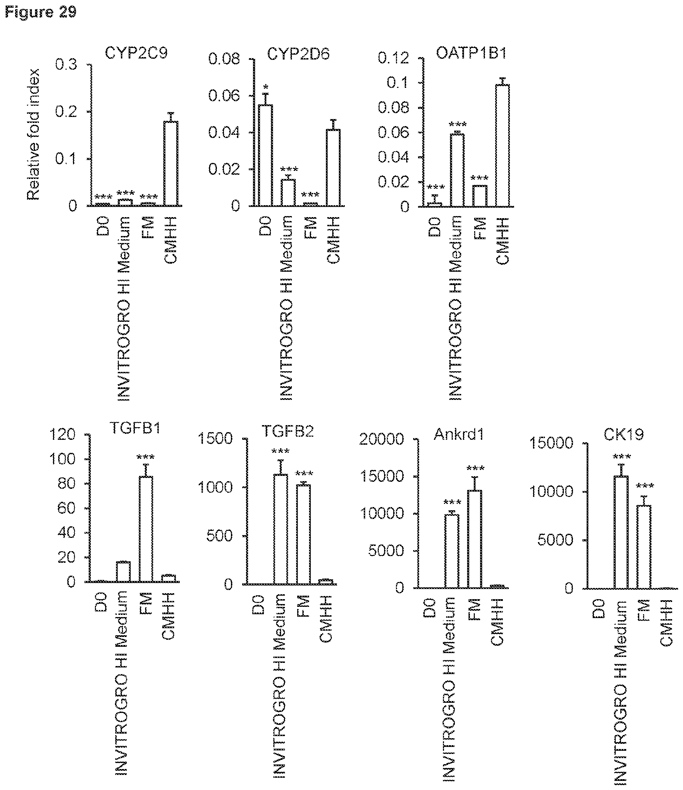

**Fig. 29** **shows the effect of CMHH on the recovery and fate maintenance of cryopreserved primary human hepatocytes.** Commercially available primary human hepatocytes (Lot: GNA, BioIVT) were cultured with either commercially available hepatocyte culture medium (INVITROGRO HI Medium, BioIVT), fresh dHCGM (FM), or CMHH (CM) for 7 days with the schedule described in Figure 13. On 7 days post seeding, total RNA was extracted for RT-qPCR analysis for the detection of indicated genes. The relative expression abundance was determined by normalization with the expression abundance in freshly isolated HLCM-HH. Statistical analysis was performed with Tukey's multiple comparison test, vs CMHH, *, **, and *** indicate *p <* 0.01, *p <* 0.001, and *p <* 0.0001.

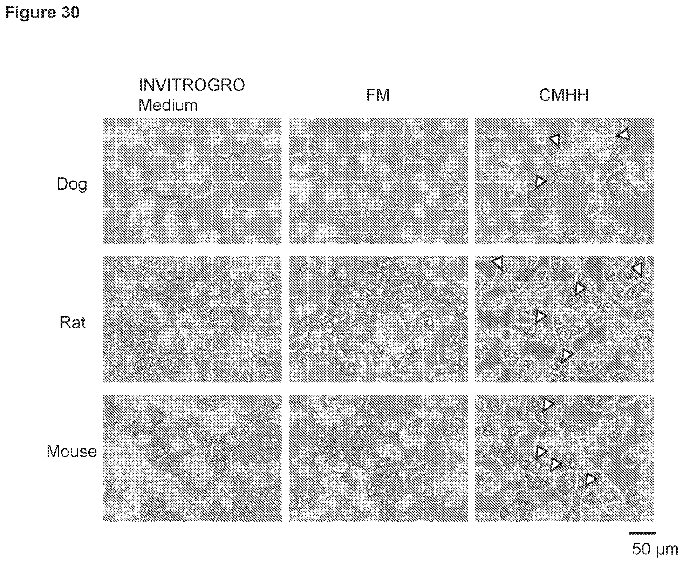

**Fig. 30** **shows the effect of CMHH on the morphology of primary hepatocytes of dog, mouse, rat.** Commercially available cryopreserved primary Dog, Rat and Mouse hepatocytes (BioIVT) were cultured with either commercially available hepatocyte culture medium (INVITROGRO HI Medium, BioIVT), fresh dHCGM (FM), or CMHH for 7 days with the schedule described in Figure 13. The morphological architecture of the cells was subsequently evaluated by light microscopy. The arrowheads indicate the bile canaliculi formed between the polygonal-cuboidal appearing human hepatocytes, which are characteristics of terminally differentiated hepatocytes.

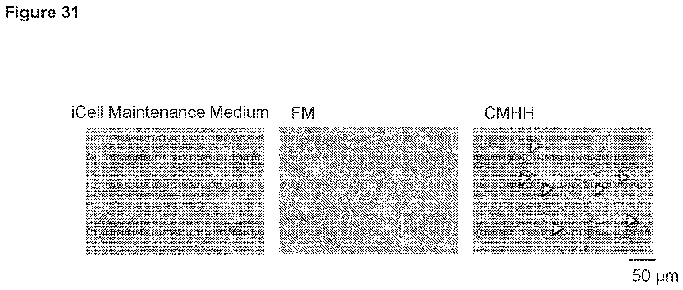

**Fig. 31** **shows the effect of CMHH on the morphology of iHeps.** Commercially available cryopreserved iHeps (iCell hepatocytes 2.0, FujiFilm) were thawed and cultured with iHep culture medium provided by the manufacture (iCell Plating Medium) for 5 days according to the manufacture's protocol followed by maintenance with either medium provided by the manufacture (iCell Maintenance Medium), fresh dHCGM (FM) or CMHH for 6 days with medium change every 2 days. The morphological architecture of the cells was subsequently evaluated by light microscopy. The arrowheads indicate the bile canaliculi formed between the polygonal-cuboidal appearing human hepatocytes, which are characteristics of terminally differentiated human hepatocytes.

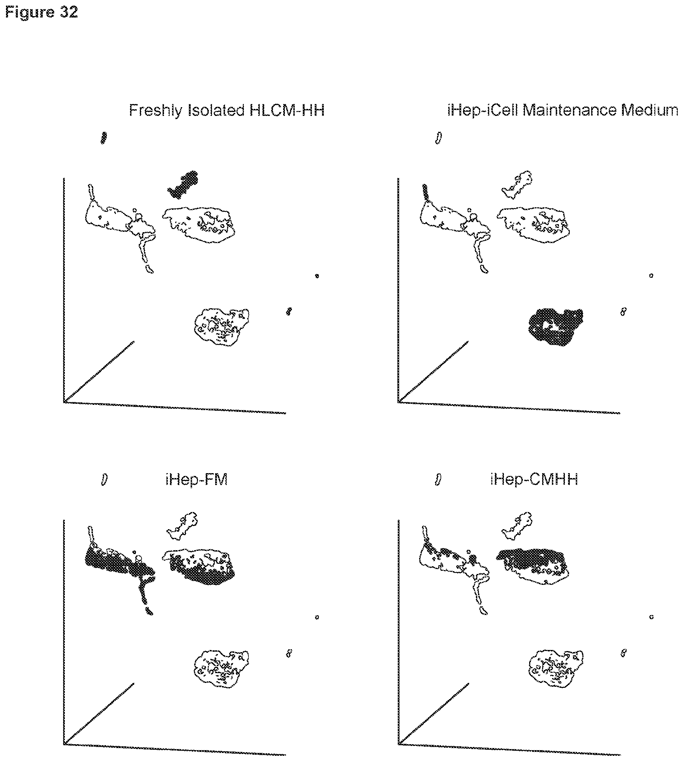

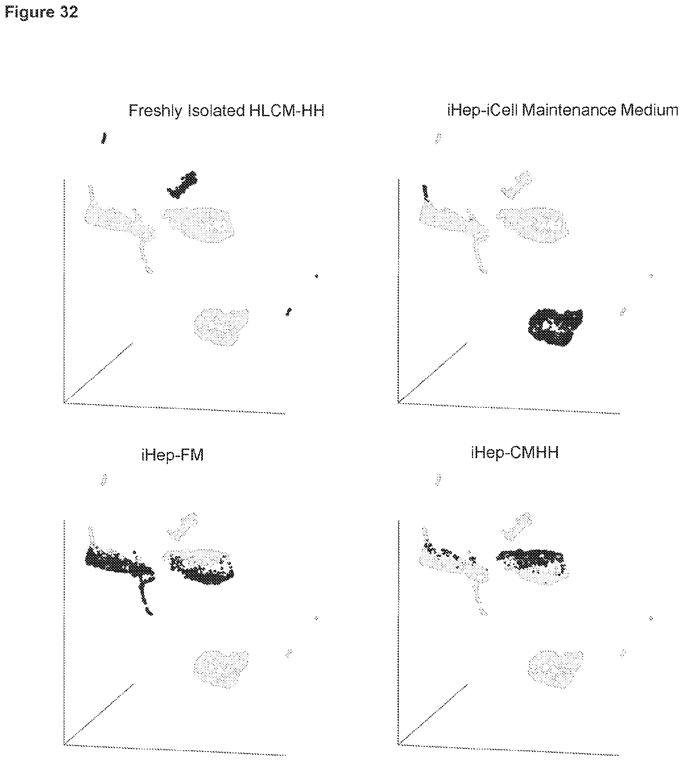

**Fig. 32** **shows effect of CMHH on iHeps.** Commercially available cryopreserved iHeps (iCell hepatocytes 2.0, FujiFilm) were thawed and cultured as described in Figure 31 followed by a single cell RNA sequencing analysis. The distance in the 3D scatter plot represents the variability between iHeps cultured with indicated medium. Freshly isolated HLCM-HH were included in the analysis as the representative of terminally differentiated human hepatocytes.

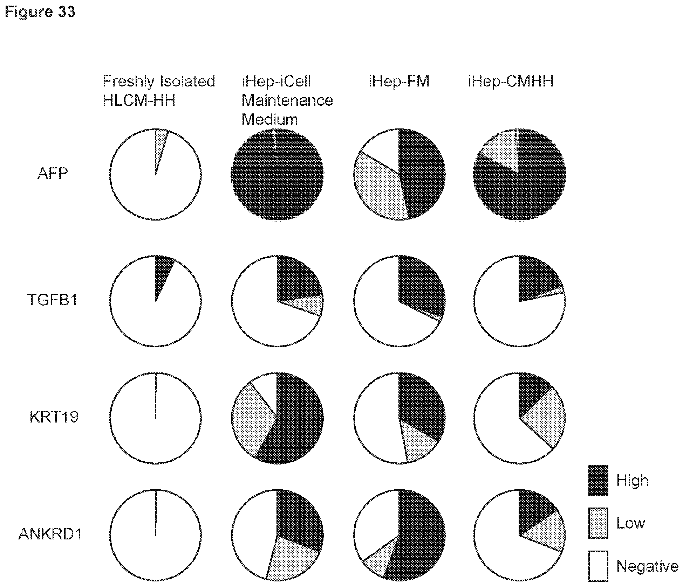

**Fig. 33** **shows the effect of CMHH on iHep gene expression.** Commercially available cryopreserved iHeps (iCell hepatocytes 2.0, FujiFilm) were thawed and cultured as described in Figure 31 followed by a single cell RNA sequencing analysis. The pie chart represents the proportion of cells with relative expression of indicated genes. Freshly isolated HLCM-HH were included in the analysis as the representative of terminally differentiated human hepatocytes.

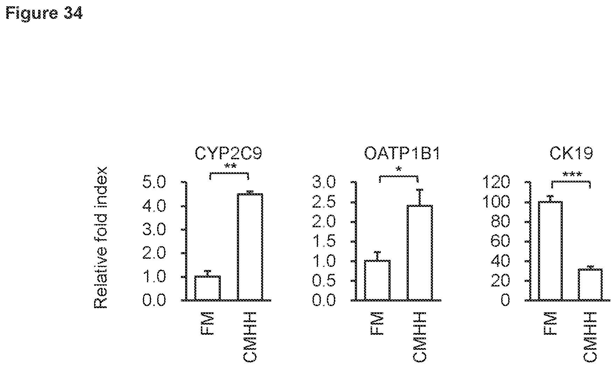

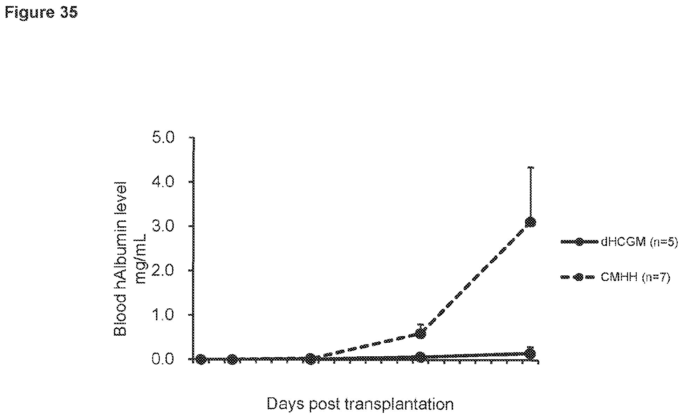

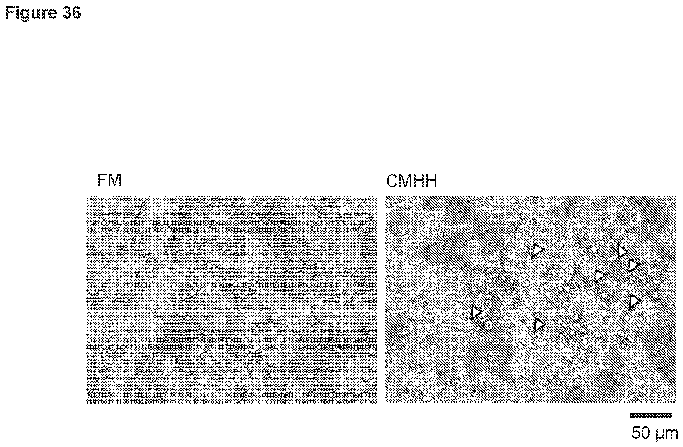

**Fig. 34** **shows effect of CMHH on CLiPs.** HLCM-HH derived CLiP cells were established as previously described (PMID: 31393263). After the first round of passage, CLiP cells were cultured with fresh dHCGM (FM) or CMHH for 7 days with the schedule described in Figure 13. The expression abundance of indicated genes was subsequently determined through RT-qPCR analysis. The relative expression abundance was determined by normalization with the expression abundance of corresponding genes in freshly isolated HLCM-HH. Paired test. *, **, and *** indicate *p* < 0.05, *p* < 0.01, *p <* 0.001 **Fig. 35** **shows the impact of CMHH pretreatment on HH proliferation in vivo.** Engraftment and proliferation of CMHH-treated human hepatocytes. Freshly isolated HLCM-HH were cultured with either fresh dHCGM (control) or CMHH for 14 days, followed by the in vivo implantation into the spleen of 3-weeks old cDNA-uPA/SCID mice (2.5×105 cells/animal). Then, serial measurement of human albumin levels in blood was performed at the indicated days after implantation with the previously described method (PMID: 17761892). **Fig. 36** **shows the effect of CMHH on the morphology of cryopreserved HLCM-HH.** Cryopreserved HLCM-HH were thawed and cultured with either fresh dHCGM (FM) or CMHH for 7 days with medium change every 2 days. The morphological architecture of the cells was subsequently evaluated by light microscopy. The arrowheads indicate the bile canaliculi formed between the polygonal-cuboidal appearing human hepatocytes, which are characteristics of terminally differentiated primary hepatocytes.

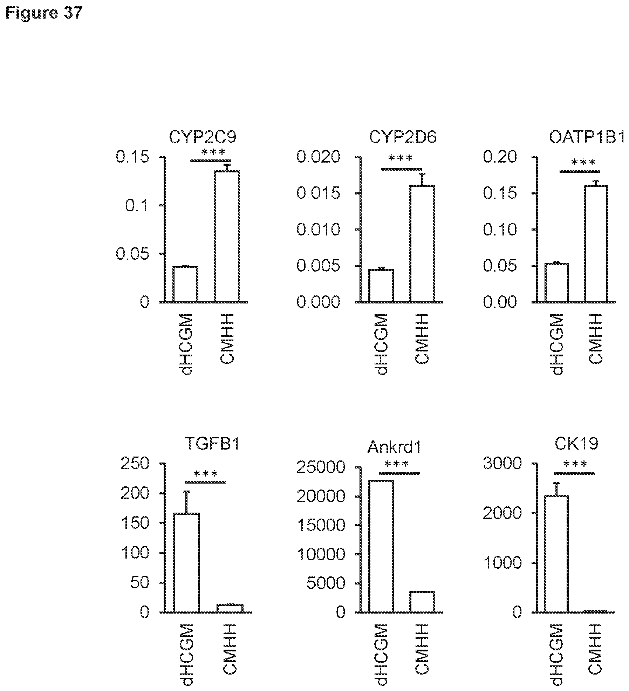

**Fig. 37** **shows the effect of CMHH on the recovery and fate maintenance of cryopreserved HLCM-HH.** Cryopreserved HLCM-HH were thawed and cultured with either fresh dHCGM (FM) or CMHH for 7 days with medium change every 2 days. On 7 days post seeding, total RNA was extracted for RT-qPCR analysis for the detection of indicated genes. The relative expression abundance was determined by normalization with the expression abundance in freshly isolated HLCM-HH. Statistical analysis was performed with student-t test, vs CMHH, *, **, and *** indicate *p <* 0.01, *p* < 0.001, and *p* < 0.0001.

### DESCRIPTION OF THE INVENTION

All references cited herein are incorporated by reference in their entirety as though fully set forth. Unless defined otherwise, technical and scientific terms used herein have the same meaning as commonly understood by one of ordinary skill in the art to which this invention belongs. Singleton et al., Dictionary of Microbiology and Molecular Biology 3rd ed., Revised, J. Wiley & Sons (New York, NY 2006); March, Advanced Organic Chemistry Reactions, Mechanisms and Structure 7th ed., J. Wiley & Sons (New York, NY 2013); and Sambrook and Russel, Molecular Cloning: A Laboratory Manual 4th ed., Cold Spring Harbor Laboratory Press (Cold Spring Harbor, NY 2012), provide one skilled in the art with a general guide to many of the terms used in the present application. For references on how to prepare antibodies, see D. Lane, Antibodies: A Laboratory Manual 2nd ed. Cold Spring Harbor Press, Cold Spring Harbor NY, 2013); Kohler and Milstein, (1976) Eur. J. Immunol. 6: 511; Queen et al. U. S. Patent No. 5,585,089; and Riechmann et al., Nature 332: 323 (1988); U.S. Pat. No. 4,946,778; Bird, Science 242:423-42 (1988); Huston et al., Proc. Natl. Acad. Sci. USA 85:5879-5883 (1988); Ward et al., Nature 334:544-54 (1989); Tomlinson I. and Holliger P. (2000) Methods Enzymol, 326, 461-479; Holliger P. (2005) Nat. Biotechnol. Sep;23(9):1126-36).

One skilled in the art will recognize many methods and materials similar or equivalent to those described herein, which could be used in the practice of the present invention. Indeed, the present invention is in no way limited to the methods and materials described. For purposes of the present invention, several terms are defined below.

The Applicants' discoveries can be used for the regenerative/stem cell biology field towards the establishment of hepatocyte transplantation therapy. Furthermore, the invention also provides a substantial degree of benefit for the basic research of liver biology and pathophysiology in general.

The Applicants discovered that the culture medium of terminally differentiated HH (hereafter conditioned medium of human hepatocytes; CMHH) contains numerous humoral factors (e.g., ECMs and plasma proteins, synthesized by hepatocytes) that cooperatively provide advantages for use in regenerative medicine and stem cell biology, e.g., to facilitate cell terminal maturation of iHeps and cell fate maintenance of fully matured iHeps. In addition, CMHH provides support for liver cell biology research, e.g., supporting the cell fate maintenance, function, and longevity of *in vitro* cultured HH, primary hepatocytes from other species (e.g., dog, murine rat, and monkey), hepatocytes derived from humanized liver chimeric animals, and nonparenchymal cells (NPC) of the liver.

In various embodiments, CMHH is propagated with high-quality HH. As used herein, the term "high-quality" refers to cells with high variability and platability. As used herein, "platability" refers to the percentage of viable cells that are capable of being adherent to the culture dish. Viable cells can be determined by techniques such as trypan blue staining. Cell quality is compromised if they are plated at low confluency; and therefore, determination of viability and platability are both important. In some embodiments, the hepatocytes cultured by the methods disclosed herein have a platability of at least 95%, 90%, 80%, 70%, 60%, and 50%. In addition, it is important to promote the recovery of high quality HH from the cell injury/stress resulting from the a) warm/cold ischemic injury during the organ procurement, b) cell damage due to the cell isolation procedure and freeze-thaw process. In some embodiments, it is important to optimize hepatocyte biological processes in order to better study hepatocyte function.

In some aspects of the application, the inventors describe HH culture media and methods that allow for 1) the rapid and robust recovery from the cell injury or stress associated with cell isolation procedures and freeze/thaw cycle(s), i.e., Phase 1 (recovery phase), and 2) cultured HH in vitro to exhibit a functionality at a comparable level to that of the human liver, thereby being suitable for a variety of experimental applications, i.e., Phase 2 (application phase).

In some embodiments, a first HH culture medium is provided for Phase 1 recovery. In some embodiments, this first medium is a DMSO-supplemented hepatocyte clonal growth medium (dHCGM), as shown in Tables 1-1 and 1-3. In some embodiments, dHCGM comprises, or consists essentially of, a standard cell culture base medium, L-proline, insulin, dexamethasone, EGF, and L-ascorbic acid 2-phosphate (Asc-2P). Particularly desired, the standard cell culture base medium is supplemented with HEPES, penicillin-streptomycin, and serum. In some embodiments the serum is fetal bovine serum (FBS) or human serum. In preferred embodiments, the standard cell culture base medium is Dulbecco's Modified Eagle's Medium (DMEM)-10.

Standard cell culture base media are well known to those skilled in the art. Examples of standard cell culture base media include but are not limited to DMEM, DMEM-10, Minimum Essential Media (MEM), RPMI-1640, Iscove's Modified Dulbecco's Medium (IMDM), or William's E Medium.

In some embodiments, the first HH culture medium is a DMSO-supplemented hepatocyte maintenance medium (dHMM), as shown in Tables 1-2 and 1-3). In some embodiments dHMM comprises, or consists essentially of, DMSO, a standard cell culture base medium, L-proline, insulin, dexamethasone, and L-ascorbic acid 2-phosphate (Asc-2P) Particularly desired, the standard cell culture base medium is supplemented with HEPES, penicillin-streptomycin, and serum. In some embodiments the serum is fetal bovine serum (FBS) or human serum. In preferred embodiments, the standard cell culture base medium is Dulbecco's Modified Eagle's Medium (DMEM)-10.

The medium used in the first phase of HH culture supports recovery of HH from stress associated with cell isolation and preservation processes. In some embodiments, HH plated at least 0.5x10⁵ cells/cm², preferably >1.2x10⁵ cells/cm², which corresponds to the confluency of >25% immediately after cell seeding, and maintained in the first HH culture medium, e.g., dHCGM or dHMM, will recover from injuries/stresses resulting from various isolation procedures and freeze/thaw storage process; and this recovery phase can take 4-7 days.

In some embodiments, the first HH culture medium, e.g., dHCGM or dHMM, is used for cell fate maintenance of HH. In some embodiments, the first HH culture medium, e.g., dHCGM or dHMM, is used for applications that are not confounded by the off-target effects of DMSO.

In some embodiments, a second HH culture medium is provided for Phase 2 application. In some embodiments, the second HH culture medium is provided for establishment and maintenance of hepatic functions of HH. In some embodiments, the application medium does not contain DMSO and allows for physiological levels of expression of CYP3A4 and CYP2E1. In some embodiments, rather than DMSO-supplemented, the second medium comprises a DMSO2-supplmented cell culture medium or a TMSO-supplemented cell culture medium.

In some aspects of the method, the quantity of HH cultured in the second phase has a physiological level of expression of CYP3A4 and a physiological level of expression of CYP2E1; or the quantity of HH cultured in the second phase metabolizes alcohol, xenobiotics, vitamin A, or a combination thereof.

In some aspects of the method, for use in assaying performance of a candidate agent in promoting alcohol metabolism by HH, promoting xenobiotics metabolism by HH, or toxicity of the candidate agent to HH, the method includes contacting the quantity of HH cultured in the second phase in the second cell culture medium, with the candidate agent, measuring a first level of metabolizing an alcohol or excretion of the candidate agent by the quantity of HH before the contact with the candidate agent, and measuring a second level of metabolizing the alcohol or excretion of the candidate agent by the quantity of HH in the presence of or after the contact with the candidate agent.

In some embodiments, the second HH culture medium is a DMSO2-supplemented hepatocyte clonal growth medium (d2HCGM), rather than DMSO-supplemented, as shown in Tables 2-1 and 2-3. In some embodiments, the second HH culture medium is a tetramethylene sulfoxide (TMSO)-supplemented hepatocyte clonal growth medium (tHCGM), rather than DMSO-supplemented.

In some embodiments, the second HH culture medium, e.g., d2HCGM, comprises, or consists essentially of DMSO2, a standard cell culture base medium, L-proline, insulin, dexamethasone, EGF, and Asc-2P. Particularly desired, the standard cell culture base medium is supplemented with HEPES, penicillin-streptomycin, and serum. In some embodiments the serum is fetal bovine serum (FBS) or human serum. In preferred embodiments, the standard cell culture base medium is Dulbecco's Modified Eagle's Medium (DMEM)-10.

In some embodiments, the second HH culture medium, e.g., tHCGM, comprises, or consists essentially of TMSO, a standard cell culture base medium, L-proline, insulin, dexamethasone, EGF, and Asc-2P. Particularly desired, the standard cell culture base medium is supplemented with HEPES, penicillin-streptomycin, and serum. In some embodiments the serum is fetal bovine serum (FBS) or human serum. In preferred embodiments, the standard cell culture base medium is Dulbecco's Modified Eagle's Medium (DMEM)-10.

In various embodiments, the second HH culture medium is a DMSO2-supplemented hepatocyte maintenance medium (d2HMM), rather than DMSO-supplemented, as shown in Tables 2-2 and 2-3. In some embodiments, the second HH culture medium is a TMSO-supplemented hepatocyte maintenance medium (tHMM), rather than DMSO-supplemented.

In some embodiments, the second HH culture medium, e.g., d2HMM, comprises, or consists essentially of DMSO2, a standard cell culture base medium, L-proline, insulin, dexamethasone, and Asc-2P. Particularly desired, the standard cell culture base medium is supplemented with HEPES, penicillin-streptomycin, and serum. In some embodiments the serum is fetal bovine serum (FBS) or human serum. In preferred embodiments, the standard cell culture base medium is Dulbecco's Modified Eagle's Medium (DMEM)-10.

In some embodiments, the second HH culture medium, e.g., tHMM, comprises, or consists essentially of TMSO, a standard cell culture base medium, L-proline, insulin, dexamethasone, and Asc-2P. Particularly desired, the standard cell culture base medium is supplemented with HEPES, penicillin-streptomycin, and serum. In some embodiments the serum is fetal bovine serum (FBS) or human serum. In preferred embodiments, the standard cell culture base medium is Dulbecco's Modified Eagle's Medium (DMEM)-10.

In some aspects of the method, the second HH culture medium contains the DMSO2 at a final concentration of about 140.8 mM.

In some aspects of the method, the HH culture medium, e.g., dHCGM, d2HCGM, or tHCGM, is supplemented with an amount of DMSO, DMSO2, or TMSO that is at least at a concentration of about 70 mM, 35 mM, and 35 mM, respectively in a standard cell culture base medium, wherein the standard cell culture base medium further includes one or more of L-proline, insulin, dexamethasone, EGF, Asc-2P, HEPES, penicillin-streptomycin, and serum. In some embodiments the serum is FBS or human serum. In preferred aspects, the standard cell culture base medium is DMEM-10.

In some aspects of the method, the HH culture medium, e.g., dHCGM, d2HCGM, or tHCGM, is supplemented with an amount of DMSO, DMSO2, or TMSO to reach a concentration of about 281.6mM, 140.8 mM, and 70.4mM, respectively in a standard cell culture base medium, wherein the standard cell culture base medium further includes one or more of L-proline, insulin, dexamethasone, EGF, Asc-2P, HEPES, penicillin-streptomycin, and serum. In some embodiments the serum is FBS or human serum. In preferred aspects, the standard cell culture base medium is DMEM-10.

In some aspects of the method, the HH culture medium, e.g., dHCGM, is supplemented with an amount of DMSO is supplemented to reach a concentration of about 2 (v/v) % in a standard cell culture base medium, wherein the standard cell culture base medium further includes one or more of L-proline, insulin, dexamethasone, EGF, Asc-2P, HEPES, penicillin-streptomycin, and serum. In some embodiments the serum is FBS or human serum. In preferred aspects, the standard cell culture base medium is DMEM-10.

In some aspects of the method, the HH culture medium, e.g., dHMM, d2HMM, or tHMM, is supplemented with an amount of DMSO, DMSO2, or TMSO to reach a concentration of about 281.6mM, 140.8 mM, and 70.4mM, respectively in a standard cell culture base medium, wherein the standard cell culture base medium further includes one or more of L-proline, insulin, dexamethasone, Asc-2P, HEPES, penicillin-streptomycin, and serum. In some embodiments the serum is FBS or human serum. In preferred aspects, the standard cell culture base medium is DMEM-10.

In some aspects of the method, the HH culture medium, e.g., dHMM, is supplemented with an amount of DMSO is supplemented to reach a concentration of about 2 (v/v) % in a standard cell culture base medium, wherein the standard cell culture base medium further includes one or more of L-proline, insulin, dexamethasone, Asc-2P, HEPES, penicillin-streptomycin, and serum. In some embodiments the serum is FBS or human serum. In preferred aspects, the standard cell culture base medium is DMEM-10.

A method is provided for culturing HH for recovery after isolation and/or preservation and establishment of hepatic function, and the method comprises culturing a quantity of HH in a first phase with a first cell culture medium for a first period of time, removing the first cell culture medium from the quantity of HH, followed by culturing the quantity of HH in a second phase with a second cell culture medium for a second period of time, wherein the first cell culture medium comprises a DMSO-supplemented cell culture medium, the second cell culture medium comprises a DMSO2-supplemented cell culture medium or a TMSO-supplemented cell culture medium, and wherein the second cell culture medium does not comprise DMSO.

In another aspect of the method, the first cell culture medium and the second cell culture medium both contain DMSO, e.g., dHCGM or dHMM.

In some aspects of the method, the first cell culture medium comprises the DMSO at 2% (v/v) and a standard cell culture base medium, and wherein the medium comprises one or more of L-proline at 15 µg/mL, insulin at 0.25 µg/mL, dexamethasone at 50 nM, EGF at 5 ng/mL, Asc-2P at 0.1 mM, HEPES at 20 mM, penicillin at 100 IU/mL, streptomycin at 100 µg/mL, and heat-inactivated FBS or human serum at 10%; and the second cell culture medium comprises the DMSO2 or TMSO at 140.8 mM or 70.4 mM, respectively and a standard cell culture base medium, and wherein the medium comprises one or more of L-proline at 15 µg/mL, insulin at 0.25 µg/mL, dexamethasone at 50 nM, EGF at 5 ng/mL, Asc-2P at 0.1 mM, HEPES at 20 mM, penicillin at 100 IU/mL, streptomycin at 100 µg/mL, and heat-inactivated FBS or human serum at 10%. In preferred aspects, the standard cell culture base medium is DMEM-10.

In some aspects of the method, the first cell culture medium comprises the DMSO at 2% (v/v) and a standard cell culture base medium, and wherein the medium comprises L-proline at 15 µg/mL, insulin at 0.25 µg/mL, dexamethasone at 50 nM, Asc-2P at 0.1 mM, HEPES at 20 mM, penicillin at 100 IU/mL, streptomycin at 100 µg/mL, and heat-inactivated FBS or human serum at 10%; and the second cell culture medium comprises the DMSO, DMSO2 or TMSO at 281.6mM, 140.8 mM or 70.4mM, respectively, and a standard cell culture base medium, and wherein the medium comprises L-proline at 15 µg/mL, insulin at 0.25 µg/mL, dexamethasone at 50 nM, EGF at 5 ng/mL, Asc-2P at 0.1 mM, HEPES at 20 mM, the penicillin at 100 IU/mL, streptomycin at 100 µg/mL, and heat-inactivated FBS or human serum at 10%. In preferred aspects, the standard cell culture base medium is DMEM-10.

In some aspects of the method, the first cell culture medium comprises the DMSO at 2% (v/v) and a standard cell culture base medium, and wherein the medium comprises L-proline at 15 µg/mL, insulin at 0.25 µg/mL, dexamethasone at 50 nM, EGF at 5ng/mL, Asc-2P at 0.1 mM, HEPES at 20 mM, penicillin at 100 IU/mL, streptomycin at 100 µg/mL, and heat-inactivated FBS or human serum at 10%; and the second cell culture medium comprises the DMSO, DMSO2 or TMSO at 281.6mM, 140.8 mM or 70.4 mM, respectively, and a standard cell culture base medium, and wherein the medium comprises L-proline at 15 µg/mL, insulin at 0.25 µg/mL, dexamethasone at 50 nM, Asc-2P at 0.1 mM, HEPES at 20 mM, the penicillin at 100 IU/mL, streptomycin at 100 µg/mL, and heat-inactivated FBS or human serum at 10%. In preferred aspects, the standard cell culture base medium is DMEM-10.

In some aspects of the method, the first cell culture medium comprises the DMSO at 2% (v/v) and a standard cell culture base medium, and wherein the medium comprises L-proline at 15 µg/mL, insulin at 0.25 µg/mL, dexamethasone at 50 nM, Asc-2P at 0.1 mM, HEPES at 20 mM, penicillin at 100 IU/mL, streptomycin at 100 µg/mL, and heat-inactivated FBS or human serum at 10%; and the second cell culture medium comprises the DMSO, DMSO2 or TMSO at 281.6mM, 140.8 mM or 70.4 mM, respectively, and a standard cell culture base medium, and wherein the medium comprises L-proline at 15 µg/mL, insulin at 0.25 µg/mL, dexamethasone at 50 nM, EGF at 5 ng/mL, Asc-2P at 0.1 mM, HEPES at 20 mM, the penicillin at 100 IU/mL, streptomycin at 100 µg/mL, and heat-inactivated FBS or human serum at 10%. In preferred aspects, the standard cell culture base medium is DMEM-10.

In some aspects of the method, the first cell culture medium comprises the DMSO at 0.5%-5% (v/v), the a standard cell culture base medium, the L-proline at 5-25 µg/mL, the insulin at 0.1-0.5 µg/mL, the dexamethasone at 10-100 nM, the EGF at 0-10 ng/mL, the Asc-2P at 0.01-1 mM, the HEPES at 10-50 mM, the penicillin at 10-300 IU/mL, the streptomycin at 10-300 µg/mL, and the heat-inactivated FBS or human serum at 2-20%; and the second cell culture medium comprises the DMSO, DMSO2, or TMSO at 35-281.6 mM, the a standard cell culture base medium, the L-proline at 5-25 µg/mL, the insulin at 0.1-0.5 µg/mL, the dexamethasone at 10-100 nM, the EGF at 0-10 ng/mL, the Asc-2P at 0.01-1 mM, the HEPES at 10-50 mM, the penicillin at 10-300 IU/mL, the streptomycin at 10-300 µg/mL, and the heat-inactivated FBS or human serum at 2-20%. In preferred aspects, the standard cell culture base medium is DMEM-10.

In some aspects of this method, the period of time HH are cultured in the first medium, e.g., recovery phase, is at least 4 days and up to 2 months, and the quantity of HH cultured in the first phase establishes cellular polarity, inter-cellular structure characterized by bile canaliculi, and/or a gene expression level equivalent to control HH exhibiting hepatic function.

In some aspects of this method, the first period of time, e.g., recovery phase, is about 7 days, and the first cell culture medium is optionally refreshed every 3 or 4 days.

In some embodiments, a kit is provided, containing the first HH culture medium, e.g., dHCGM or dHMM, and the second HH culture medium, e.g., d2HCGM, tHCGM, d2HMM or tHMM, which can be used sequentially for culturing HH obtained from isolation or thawed from preservation and subsequently for experimental assays.

A kit for culturing human hepatocytes is also provided, and the kit comprises a first container storing a first cell culture medium, said first cell culture medium is supplemented with DMSO, e.g., dHCGM or dHMM; and a second container storing a second cell culture medium, said second cell culture medium is supplemented with DMSO2 and/or TMSO and without DMSO e.g., d2HCGM, d2HMM, tHCGM, or tHMM.

A method is further provided for assaying toxicity of an agent to human hepatocytes (HH) or drug metabolism and pharmacokinetics (DMPK) of the agent, and the method comprises culturing a quantity of HH in a medium supplemented with DMSO2 or TMSO, without DMSO; contacting the agent with the quantity of HH cultured in the DMSO2 or TMSO-supplemented medium; and measuring a level of toxicity to the quantity of HH in the presence of, or after the contact with, the agent, or measuring a level of metabolism or excretion of the agent by the quantity of HH in the presence of, or after the contact with, the agent.

In some aspects of the assay method, the agent is an alcohol compound comprising ethanol, methanol, ethylene glycol, isopropanol, or a mixture thereof.

In some aspects of the assay method, the contacting and the measuring are performed in a tight-sealing apparatus.

In some aspects of the assay method, the level of toxicity is measured via quantifying expression or function of xenobiotics-metabolizing enzymes such as, but not limited to Cytochrome P450 (CYP) enzymes in the quantity of HH.

In some aspects of the assay method, the level of toxicity is measured via quantifying the toxic metabolites of alcohol such as acetaldehyde, or reduction of glutathione, the activation status of cell death pathways in the quantity of HH.

A method is further provided for assaying a candidate agent for promoting metabolism of alcohol or vitamin A, and the method comprises culturing a quantity of HH in a medium supplemented with DMSO2 or TMSO, without DMSO; contacting the quantity of HH cultured in the DMSO2 or TMSO-supplemented medium with the alcohol or the vitamin A in the presence of candidate agent; and measuring a level of metabolism of the alcohol or the vitamin A by the quantity of HH in the presence of, or after the contact with, the candidate agent.

Culturing HH first in a medium from the recovery phase (phase 1), e.g., dHCGM or dHMM and/or culturing HH in a medium from the application phase (phase 2), e.g., d2HCGM, tHCGM, d2HMM, or tHMM, supports high quality HH.

Accordingly, an aspect of the invention provides a method for preparing CMHH, comprising: culturing HH in a medium to generate a culture medium, and harvesting the culture medium having been incubated with the high-quality HH for at least 1 hour, as the conditioned medium.

In certain embodiments, the HH comprise humanized liver chimeric mice derived-human hepatocytes (HLCM-HH), primary human hepatocytes (PHH), hepatocytes derived from a chimeric animal with humanized liver, e.g., rat, mouse, sheep, pig, or monkey, or a combination thereof. In some embodiments, the HH are PHH. In some embodiments, the HH are HLCM-HH. In some embodiments, the HH are derived from a chimeric animal other than mouse with a humanized liver.

In certain embodiments, the HLCM-HH are obtained from a liver derived from a mouse injected with PHH or with previously isolated HLCM-HH into the spleen. In some embodiments, the HH are obtained from the liver derived from another animal (e.g., rat, mouse, sheep, pig, monkey, etc.) that was injected with PHH or with previous isolated HH from a humanized liver chimeric animal into the spleen.

In some embodiments, the mouse has a human hepatocyte replacement index (indicated by histological examination or blood concentration of HH derived factors (e.g., human albumin and human alpha 1 antitrypsin) of at least 10% before the HLCM-HH are obtained; or the chimeric animal with a humanized liver has a human hepatocyte replacement index at least 10% before the HH are isolated or obtained.

In a further embodiment, the HLCM-HH, the PHH, the hepatocytes derived from another chimeric animal with humanized liver, or a combination of any two or all three, are cultured at a cell density from 0.5×10⁵/cm² to 5×10⁵/cm².

In a further embodiment, the HH do not comprise any of HepG2 cells, Huh7 cells and murine hepatocytes. In another embodiment, the HH comprise HLCM-HH, which may include some mouse hepatocytes and mouse non-parenchymal cells in additional to HH.

In a further embodiment, the HH comprise HLCM-HH, and the method further comprises obtaining the HLCM-HH before the culturing step, wherein the obtaining of the HLCM-HH comprises isolating hepatocytes derived from a liver of a mouse injected with PHH or with previously isolated HLCM-HH into a spleen of the mouse, thereby obtaining the HLCM-HH.

In a further embodiment, the HH derived from the liver of the mouse are isolated via collagenase perfusion of the liver of the mouse, and the mouse has a human hepatocyte replacement index at least 10 % before the HH are isolated.

A further aspect of the invention provides CMHH, comprising one or more humoral factors secreted by HH and a hepatocyte clonal growth medium or a hepatocyte maintenance medium.

In further embodiments, the medium used to prepare CMHH is a dHCGM, d2HCGM, or tHCGM. In some embodiments, dHCGM comprises, or consists essentially of, DMSO, a standard cell culture base medium, L-proline, insulin, dexamethasone, EGF, and Asc-2P. Particularly desired the standard cell culture base medium is supplemented with HEPES, penicillin-streptomycin, and serum. In some embodiments, d2HCGM comprises or consists essentially of, DMSO2, a standard cell culture base medium, L-proline, insulin, dexamethasone, EGF, and Asc-2P. Particularly desired the standard cell culture base medium is supplemented with HEPES, penicillin-streptomycin, and serum. In some embodiments, tHCGM comprises or consists essentially of, TMSO, a standard cell culture base medium, L-proline, insulin, dexamethasone, EGF, and Asc-2P. Particularly desired the standard cell culture base medium is supplemented with HEPES, penicillin-streptomycin, and serum. In preferred embodiments, the standard cell culture base medium is DMEM-10. In some embodiments, the serum is FBS or human serum.

In some embodiments, the medium used to prepare CMHH is a dHMM, d2HMM or tHMM. In some embodiments, dHMM comprises, or consists essentially of, DMSO, a standard cell culture base medium, L-proline, insulin, dexamethasone, and Asc-2P. In some embodiments, d2HMM comprises or consists essentially of, DMSO2, standard cell culture base medium, L-proline, insulin, dexamethasone, and Asc-2P. In some embodiments, tHMM comprises or consists essentially of, TMSO, standard cell culture base medium, L-proline, insulin, dexamethasone, and Asc-2P. In preferred embodiments, the standard cell culture base medium is supplemented with HEPES, penicillin-streptomycin, and serum. Particularly desired the standard cell culture base medium is DMEM-10. In some embodiments, the serum is FBS or human serum.

In a further embodiment, the medium used to prepare CMHH consists of ingredients wherein the L-proline is 5-25 µg/mL, the insulin is 0.1-0.5 µg/mL, the dexamethasone is 10-100 nM, the EGF is 0-10 ng/mL, the Asc-2P is 0.01-1 mM, the DMSO, the DMSO2, or TMSO is 35-300mM, the HEPES is 10-50 mM, the penicillin is 10-300 IU/mL, the streptomycin is 10-300 µg/mL, and the serum is heat-inactivated FBS or human serum at 2-20%. Preferably, the L-proline is 15 µg/mL, the insulin is 0.25 µg/mL, the dexamethasone is 50 nM, the EGF is 5 ng/mL in the dHCGM, d2HCGM, or tHCGM or is 0 ng/mL in dHMM, d2HMM, or tHMM the Asc-2P is 0.1 mM, the DMSO, DMSO2, or TMSO is 281.6 mM, 140.8 mM, or 70.4 mM, respectively, the HEPES is 20 mM, the penicillin is 100 IU/mL, the streptomycin is 100 µg/mL, and the serum, e.g., FBS or human serum is at 10%.

In a further embodiment, the medium used to prepare CMHH is replaced with a fresh volume every 1 to 120 hours.

A further aspect of the invention provides CMHH, prepared by the method of the present invention.

In some embodiments, the CMHH comprises a dHCGM or dHMM, wherein the dHCGM or dHMM has been used to culture HLCM-HH, PHH, or a combination thereof for at least 1 hour.

In a further embodiment, dHCGM used to culture HLCM-HH, PHH, or a combination thereof for at least 1 hour comprises a standard cell culture base medium supplemented with L-proline, insulin, dexamethasone, EGF, Asc-2P, DMSO. Particularly desired the standard cell culture base medium is supplemented with HEPES, penicillin-streptomycin, and serum. In some embodiments, the serum is FBS or human serum. In preferred embodiments, the standard cell culture base medium is DMEM-10.

In a further embodiment, dHMM used to culture HLCM-HH, PHH, or a combination thereof for at least 1 hour comprises a standard cell culture base medium supplemented with L-proline, insulin, dexamethasone, Asc-2P, DMSO. Particularly desired the standard cell culture base medium is supplemented with HEPES, penicillin-streptomycin, and serum. In some embodiments, the serum is FBS or human serum. In preferred embodiments, the standard cell culture base medium is DMEM-10.

In some embodiments, the CMHH comprises a d2HCGM or d2HMM, wherein the d2HCGM or d2HMM has been used to culture HLCM-HH, PHH, or a combination thereof for at least 1 hour.

In a further embodiment, d2HCGM used to culture HLCM-HH, PHH, or a combination thereof for at least 1 hour comprises a standard cell culture base medium supplemented with L-proline, insulin, dexamethasone, EGF, Asc-2P, DMSO2. Particularly desired the standard cell culture base medium is supplemented with HEPES, penicillin-streptomycin, and serum. In some embodiments, the serum is FBS or human serum. In preferred embodiments, the standard cell culture base medium is DMEM-10.

In a further embodiment, d2HMM used to culture HLCM-HH, PHH, or a combination thereof for at least 1 hour comprises a standard cell culture base medium supplemented with L-proline, insulin, dexamethasone, Asc-2P, DMSO2. Particularly desired the standard cell culture base medium is supplemented with HEPES, penicillin-streptomycin, and serum. In some embodiments, the serum is FBS or human serum. In preferred embodiments, the standard cell culture base medium is DMEM-10.

In some embodiments, the CMHH comprises a tHCGM or tHMM, wherein the tHCGM or tHMM has been used to culture HLCM-HH, PHH, or a combination thereof for at least 1 hour.

In a further embodiment, tHCGM used to culture HLCM-HH, PHH, or a combination thereof for at least 1 hour comprises a standard cell culture base medium supplemented with L-proline, insulin, dexamethasone, EGF, Asc-2P, TMSO. Particularly desired the standard cell culture base medium is supplemented with HEPES, penicillin-streptomycin, and serum. In some embodiments, the serum is FBS or human serum. In preferred embodiments, the standard cell culture base medium is DMEM-10.

In a further embodiment, tHMM used to culture HLCM-HH, PHH, or a combination thereof for at least 1 hour comprises a standard cell culture base medium supplemented with L-proline, insulin, dexamethasone, Asc-2P, TMSO. Particularly desired the standard cell culture base medium is supplemented with HEPES, penicillin-streptomycin, and serum. In some embodiments, the serum is FBS or human serum. In preferred embodiments, the standard cell culture base medium is DMEM-10.

A further aspect of the invention provides a combination, comprising a CMHH and extracellular matrices. In some aspects, the extracellular matrices comprise MATRIGEL^{®}, a gelatinous protein mixture secreted by Engelbreth-Holm-Swarm (EHS) mouse sarcoma cells or individual extracellular matrices(es) such as but not limited to fibronectin, collagen, vitronectin, and laminin.

A further aspect of the invention provides a combination, comprising a CMHH of the present invention and five chemicals of forskolin, SB431542, DAPT, IWP2, and LDN193189.

In various embodiments, the CMHH is used for liver cell biology research. The current major limitation in the research of liver cell biology is the lack of stable in vitro culture system of terminally differentiated HH. For example, cryopreserved PHH is the mainstay tool that most pharmaceutical industry utilizes for the drug safety screening as well as drug metabolism and pharmacokinetics (DMPK) studies at the preclinical trial stage.

An aspect of the invention provides a CMHH for use in cultivating terminally differentiated PHH, HLCM-HH, hepatocytes derived from another chimeric animal (e.g., rat, sheep, pig, monkey) with humanized liver, primary hepatocytes from another species, e.g., dog, mouse, rat, and monkey, and/or cryopreserved PHH, HLCM-HH, hepatocytes derived from another chimeric animal (e.g., rat, sheep, pig, monkey) with humanized liver, and primary hepatocytes from another species, e.g., dog, mouse, rat, and monkey.

In some embodiments, the biological effect of CMHH supports the function of nonparenchymal cells (NPC) of the liver, such as hepatic stellate cells (HSC), liver sinusoidal endothelial cells (LSEC), and hepatic macrophage (Kupffer cells).

A further aspect of the invention provides a CMHH for use in cultivating terminally differentiated HH, HLCM-HH, hepatocytes derived from another chimeric animal (e.g., rat, sheep, pig, monkey) with humanized liver, PHH, and and/or primary hepatocytes from another species, e.g., dog, mouse, rat, and monkey. In some embodiments, the CMHH provided prevents the dedifferentiation of terminally differentiated in vitro cultured HH, HLCM-HH, hepatocytes derived from another chimeric animal (e.g., rat, sheep, pig, monkey) with humanized liver, PHH, and and/or primary hepatocytes from another species, e.g., dog, mouse, rat, and monkey.

A further aspect of the invention provides a CMHH for use in prohibiting the transdifferentiation of HH, HLCM-HH, hepatocytes derived from another chimeric animal (e.g., rat, sheep, pig, monkey) with humanized liver, PHH, and and/or primary hepatocytes from another species, e.g., dog, mouse, rat, and monkey.

A further aspect of the invention provides a CMHH for use in promoting the recovery of cryopreserved HH, HLCM-HH, hepatocytes derived from another chimeric animal (e.g., rat, sheep, pig, monkey) with humanized liver, PHH, and and/or primary hepatocytes from another species, e.g., dog, mouse, rat, and monkey.

A further aspect of the invention provides a CMHH for use in promoting the recovery of freshly isolated HH, HLCM-HH, hepatocytes derived from another chimeric animal (e.g., rat, sheep, pig, monkey) with humanized liver, PHH, and and/or primary hepatocytes from another species, e.g., dog, mouse, rat, and monkey from stresses/injuries associated with cell procurement processes.

A further aspect of the invention provides a CMHH for use in protecting HH, HLCM-HH, hepatocytes derived from another chimeric animal (e.g., rat, sheep, pig, monkey) with humanized liver, PHH, and and/or primary hepatocytes from another species, e.g., dog, mouse, rat, and monkey, from the cellular stress associated with the cryopreservation, which may further support the cell recovery upon thawing.

A further aspect of the invention provides a CMHH for use in supporting the maintenance and function of NPC function and morphology. In some embodiments, a CMHH is provided for use in co-culturing with NPCs, and/or for use in suspending human hepatocytes in cryopreservation.

A further aspect of the invention provides a CMHH for use in supporting/enhancing the functionality and longevity of HH, HLCM-HH, hepatocytes derived from another chimeric animal (e.g., rat, sheep, pig, monkey) with humanized liver, PHH, and and/or primary hepatocytes from another species, e.g., dog, mouse, rat, and monkey, in the format of spheroid, organoid, or 3D culture, as well as for the development of organ-chips format.

A further aspect of the invention provides a CMHH for use in enhancing the utility of HH, HLCM-HH, hepatocytes derived from another chimeric animal (e.g., rat, sheep, pig, monkey) with humanized liver, PHH, and and/or primary hepatocytes from another species, e.g., dog, mouse, rat, and monkey, for the production of HLCM.

A further aspect of the invention provides a CMHH for use in supporting the cell fate maintenance of HH, HLCM-HH, hepatocytes derived from another chimeric animal (e.g., rat, sheep, pig, monkey) with humanized liver, PHH, and and/or primary hepatocytes from another species, e.g., dog, mouse, rat, and monkey; thereby CMHH greatly enhances the utility of these cells for various types of liver cell biology studies, such as DMPK, drug screening, infectious diseases, and metabolic disease.

In various embodiments, the CMHH is used for cell fate maintenance of PHH and HLCM-HH and HH derived from another chimeric animal with humanized liver; thereby CMHH greatly enhances the utility of these cells for various types of liver cell biology studies, such as DMPK, drug screening, infectious diseases, and metabolic diseases.

A method is further provided for assaying toxicity of an agent to HH or DMPK of the agent, and the method comprises culturing a quantity of HH in a CMHH supplemented with DMSO2 or TMSO, without DMSO; contacting the agent with the quantity of HH cultured in the DMSO2 or TMSO-supplemented CMHH; and measuring a level of toxicity to the quantity of HH in the presence of, or after the contact with, the agent, or measuring a level of metabolism or excretion of the agent by the quantity of HH in the presence of, or after the contact with, the agent.

In some aspects of the assay method, the agent is an alcohol compound comprising ethanol, methanol, ethylene glycol, isopropanol, or a mixture thereof.

In some aspects of the assay method, the contacting and the measuring are performed in a tight-sealing apparatus.

In some aspects of the assay method, the level of toxicity is measured via quantifying expression or function of xenobiotics-metabolizing enzymes such as, but not limited to Cytochrome P450 (CYP) enzymes in the quantity of HH.

In some aspects of the assay method, the level of toxicity is measured via quantifying the toxic metabolites of alcohol such as acetaldehyde, or reduction of glutathione, the activation status of cell death pathways in the quantity of HH.

A method is further provided for assaying a candidate agent for promoting metabolism of alcohol or vitamin A, and the method comprises culturing a quantity of HH in a CMHH supplemented with DMSO2 or TMSO, without DMSO; contacting the quantity of HH cultured in the DMSO2 or TMSO-supplemented CMHH with the alcohol or the vitamin A in the presence of candidate agent; and measuring a level of metabolism of the alcohol or the vitamin A by the quantity of HH in the presence of, or after the contact with, the candidate agent.

In various embodiments, the CMHH is used in regenerative medicine and stem cell biology. CMHH can be an essential factor that enables the final stage of iHep maturation to the terminally differentiated human hepatocytes. In some embodiments, the CMHH is used in the preparation of a hepatocyte transplantation therapy, e.g., CMHH used in the mass production of iPS-cells derived matured human hepatocytes, which can be a substitution of LT.

A further aspect of the invention provides a CMHH for use in facilitating the further maturation of iHep (stem cell derived hepatic bipotential progenitor cells) to terminally differentiated hepatocytes.

A further aspect of the invention provides a CMHH for use in preventing the differentiation of iHep (stem cell derived hepatic bipotential progenitor cells) to cholangiocytes or other cell types.

A further aspect of the invention provides a CMHH for use in promoting the re-differentiation of Chemically Induced Liver Progenitors (CLiPs) to the matured hepatocytes.

A further aspect of the invention provides a CMHH for use in enhancing hepatic functions of hepatoma cells (e.g., HepaRG cells, HepG2, and Huh7 cells).

In some embodiments, the CMHH is used to enhance hepatic functions of HepaRG.

A further aspect of the invention provides a CMHH for use in allowing for the expansion/propagation of the CMHH treated iHep and CLiPs in the liver of HLCM-host mouse. In some embodiments, the invention provides a CMHH for use in allowing for the expansion/propagation of the CMHH treated PHH, and/or primary hepatocytes from another species, e.g., dog, mouse, rat, and monkey, in the liver of HLCM-host mouse.

A further aspect of the invention provides a CMHH for use in promoting the regression of chronic liver disease/liver fibrosis, as a result of hepatocyte transplantation therapy.

A further aspect of the invention provides a CMHH for use in adjuvant therapy for liver cancer.

A further aspect of the invention provides a CMHH for use in supporting the differentiation and cell fate maintenance of iHep, CliPs, and HepaRG, which allows the stable and long-term culture of these cells without compromising the characteristics of matured hepatocytes, therefore CMHH greatly enhances the utility of these cells for various types of liver cell biology studies, such as DMPK, drug screening, infectious diseases, and metabolic diseases

A further aspect of the invention provides a CMHH for use in differentiating Chemically Induced Liver Progenitors (Clip) cells.

In some embodiments, the CMHH is used to promote the re-differentiation of CLiPs to the matured hepatocytes.

In various embodiments, CHMM is used to cultivate and maintain hepatocytes that could include HH, PHH from other species, e.g., dog, mouse, rat, and monkey, hepatocytes derived from humanized liver chimeric mouse (HLCM), or hepatocytes derived from another chimeric animal with humanized liver (e.g., hepatocytes derived from rat, sheep, pig, or monkey).

In some embodiments, the CMHH is used for the hepatocytes transplantation (such as iHeps further differentiated by CMHH) to patients who suffer from any type liver diseases.

Various embodiments of the invention are described above in the Description of the Invention. While these descriptions directly describe the above embodiments, it is understood that those skilled in the art may conceive modifications and/or variations to the specific embodiments shown and described herein. Any such modifications or variations that fall within the purview of this description are intended to be included therein as well. Unless specifically noted, it is the intention of the inventors that the words and phrases in the specification and claims be given the ordinary and accustomed meanings to those of ordinary skill in the applicable art(s).

The foregoing description of various embodiments of the invention known to the applicant at this time of filing the application has been presented and is intended for the purposes of illustration and description. The present description is not intended to be exhaustive nor limit the invention to the precise form disclosed and many modifications and variations are possible in the light of the above teachings. The embodiments described serve to explain the principles of the invention and its practical application and to enable others skilled in the art to utilize the invention in various embodiments and with various modifications as are suited to the particular use contemplated. Therefore, it is intended that the invention not be limited to the particular embodiments disclosed for carrying out the invention.

While particular embodiments of the present invention have been shown and described, it will be obvious to those skilled in the art that, based upon the teachings herein, changes and modifications may be made without departing from this invention and its broader aspects and, therefore, the appended claims are to encompass within their scope all such changes and modifications as are within the true spirit and scope of this invention. It will be understood by those within the art that, in general, terms used herein are generally intended as "open" terms (*e.g.,* the term "including" should be interpreted as "including but not limited to," the term "having" should be interpreted as "having at least," the term "includes" should be interpreted as "includes but is not limited to," etc.).

As used herein the term "comprising" or "comprises" is used in reference to compositions, methods, and respective component(s) thereof, that are useful to an embodiment, yet open to the inclusion of unspecified elements, whether useful or not. It will be understood by those within the art that, in general, terms used herein are generally intended as "open" terms (e.g., the term "including" should be interpreted as "including but not limited to," the term "having" should be interpreted as "having at least," the term "includes" should be interpreted as "includes but is not limited to," etc.). Although the open-ended term "comprising," as a synonym of terms such as including, containing, or having, is used herein to describe and claim the invention, the present invention, or embodiments thereof, may alternatively be described using alternative terms such as "consisting of" or "consisting essentially of."

The singular terms "a," "an," and "the" include plural referents unless context clearly indicates otherwise. Similarly, the word "or" is intended to include "and" unless the context clearly indicates otherwise. Although methods and materials similar or equivalent to those described herein can be used in the practice or testing of this disclosure, suitable methods and materials are described below. The abbreviation, "e.g." is derived from the Latin exempli gratia, and is used herein to indicate a non-limiting example. Thus, the abbreviation "e.g." is synonymous with the term "for example."

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

Unless otherwise defined herein, scientific and technical terms used in connection with the present application shall have the meanings that are commonly understood by those of ordinary skill in the art to which this disclosure belongs. It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims. Definitions of common terms in immunology and molecular biology can be found in The Merck Manual of Diagnosis and Therapy, 19th Edition, published by Merck Sharp & Dohme Corp., 2011 (ISBN 978-0-911910-19-3); Robert S. Porter et al. (eds.), The Encyclopedia of Molecular Cell Biology and Molecular Medicine, published by Blackwell Science Ltd., 1999-2012 (ISBN 9783527600908); and Robert A. Meyers (ed.), Molecular Biology and Biotechnology: a Comprehensive Desk Reference, published by VCH Publishers, Inc., 1995 (ISBN 1-56081-569-8); Immunology by Werner Luttmann, published by Elsevier, 2006; Janeway's Immunobiology, Kenneth Murphy, Allan Mowat, Casey Weaver (eds.), Taylor & Francis Limited, 2014 (ISBN 0815345305, 9780815345305); Lewin's Genes XI, published by Jones & Bartlett Publishers, 2014 (ISBN-1449659055); Michael Richard Green and Joseph Sambrook, Molecular Cloning: A Laboratory Manual, 4th ed., Cold Spring Harbor Laboratory Press, Cold Spring Harbor, N.Y., USA (2012) (ISBN 1936113414); Davis et al., Basic Methods in Molecular Biology, Elsevier Science Publishing, Inc., New York, USA (2012) (ISBN 044460149X); Laboratory Methods in Enzymology: DNA, Jon Lorsch (ed.) Elsevier, 2013 (ISBN 0124199542); Current Protocols in Molecular Biology (CPMB), Frederick M. Ausubel (ed.), John Wiley and Sons, 2014 (ISBN 047150338X, 9780471503385), Current Protocols in Protein Science (CPPS), John E. Coligan (ed.), John Wiley and Sons, Inc., 2005; and Current Protocols in Immunology (CPI) (John E. Coligan, ADA M Kruisbeek, David H Margulies, Ethan M Shevach, Warren Strobe, (eds.) John Wiley and Sons, Inc., 2003 (ISBN 0471142735, 9780471142737), the contents of which are all incorporated by reference herein in their entireties.

One of skill in the art can readily identify a chemotherapeutic agent of use (*e.g.* see Physicians' Cancer Chemotherapy Drug Manual 2014, Edward Chu, Vincent T. DeVita Jr., Jones & Bartlett Learning; Principles of Cancer Therapy, Chapter 85 in Harrison's Principles of Internal Medicine, 18th edition; Therapeutic Targeting of Cancer Cells: Era of Molecularly Targeted Agents and Cancer Pharmacology, Chs. 28-29 in Abeloff's Clinical Oncology, 2013 Elsevier; and Fischer D S (ed): The Cancer Chemotherapy Handbook, 4th ed. St. Louis, Mosby-Year Book, 2003).

In some embodiments of any of the aspects, the disclosure described herein does not concern a process for cloning human beings, processes for modifying the germ line genetic identity of human beings, uses of human embryos for industrial or commercial purposes or processes for modifying the genetic identity of animals which are likely to cause them suffering without any substantial medical benefit to man or animal, and also animals resulting from such processes.

Other terms are defined herein within the description of the various aspects of the invention.

All patents and other publications; including literature references, issued patents, published patent applications, and co-pending patent applications; cited throughout this application are expressly incorporated herein by reference for the purpose of describing and disclosing, for example, the methodologies described in such publications that might be used in connection with the technology described herein. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

The description of embodiments of the disclosure is not intended to be exhaustive or to limit the disclosure to the precise form disclosed. While specific embodiments of, and examples for, the disclosure are described herein for illustrative purposes, various equivalent modifications are possible within the scope of the disclosure, as those skilled in the relevant art will recognize. For example, while method steps or functions are presented in a given order, alternative embodiments may perform functions in a different order, or functions may be performed substantially concurrently. The teachings of the disclosure provided herein can be applied to other procedures or methods as appropriate. The various embodiments described herein can be combined to provide further embodiments. Aspects of the disclosure can be modified, if necessary, to employ the compositions, functions and concepts of the above references and application to provide yet further embodiments of the disclosure. Moreover, due to biological functional equivalency considerations, some changes can be made in protein structure without affecting the biological or chemical action in kind or amount. These and other changes can be made to the disclosure in light of the detailed description. All such modifications are intended to be included within the scope of the appended claims.

Specific elements of any of the foregoing embodiments can be combined or substituted for elements in other embodiments. Furthermore, while advantages associated with certain embodiments of the disclosure have been described in the context of these embodiments, other embodiments may also exhibit such advantages, and not all embodiments need necessarily exhibit such advantages to fall within the scope of the disclosure.

Some embodiments of the technology described herein can be defined according to any of the following numbered paragraphs:
1. A method for preparing a conditioned medium from cultured human hepatocytes, comprising:
   culturing human hepatocytes in a medium to generate a culture medium, and
   harvesting the culture medium having been incubated with the human hepatocytes for at least 1 hour, as the conditioned medium.
2. The method of paragraph 1, wherein the human hepatocytes comprise primary human hepatocytes (PHH), hepatocytes derived from a chimeric animal with humanized liver, or a combination thereof.
3. The method of paragraph 2, wherein the hepatocytes derived from the chimeric animal with humanized liver are humanized liver chimeric mice derived-human hepatocytes (HLCM-HH), which are obtained from a liver derived from a mouse injected with PHH or with previously isolated HLCM-HH into the spleen.
4. The method of paragraph 3, wherein the mouse has a replacement index of at least 10% before the HLCM-HH are obtained.
5. The method of paragraph 1, wherein the medium is a DMSO or DMSO2-supplemented hepatocyte clonal growth medium (dHCGM), and the dHCGM comprises, or consists essentially of, Dulbecco's Modified Eagle's Medium (DMEM), L-proline, insulin, dexamethasone, EGF, and L-ascorbic acid 2-phosphate (Asc-2P).
6. The method of paragraph 5, wherein the DMEM is DMEM-10, and the DMEM-10 comprises, or consists essentially of, DMEM, HEPES, penicillin-streptomycin, and fetal bovine serum (FBS).
7. The method of paragraph 5, wherein the L-proline is 15 µg/mL in the dHCGM, the insulin is 0.25 µg/mL in the dHCGM, the dexamethasone is 50 nM in the dHCGM, the EGF is 5 ng/mL in the dHCGM, the Asc-2P is 0.1 mM in the dHCGM, the DMSO or the DMSO2 is 2% in the dHCGM, the HEPES is 20 mM in the dHCGM, the penicillin is 100 IU/mL in the dHCGM, the streptomycin is 100 µg/mL in the dHCGM, and the FBS is heat-inactivated FBS at 10% in the dHCGM.
8. The method of paragraph 5, wherein the L-proline is 5-25 µg/mL in the dHCGM, the insulin is 0.1-0.5 µg/mL in the dHCGM, the dexamethasone is 10-100 nM in the dHCGM, the EGF is 1-10 ng/mL in the dHCGM, the Asc-2P is 0.01-1 mM in the dHCGM, the DMSO or the DMSO2 is 0.5%-5% in the dHCGM, the HEPES is 10-50 mM in the dHCGM, the penicillin is 10-300 IU/mL in the dHCGM, the streptomycin is 10-300 µg/mL in the dHCGM, and the FBS is heat-inactivated FBS at 2-20% in the dHCGM.
9. The method of any one of paragraphs 1-8, wherein medium is replaced with a fresh volume every 24 to 120 hours.
10. The method of paragraph 2, wherein the PHH, the hepatocytes derived from the chimeric animal with humanized liver, or the combination are cultured at a cell density from 0.5×10⁵/cm² to 5×10⁵/cm².
11. The method of any of paragraphs 1-3, wherein the human hepatocytes do not comprise HepG2 cells, Huh7 cells or murine hepatocytes.
12. The method of paragraph 1, wherein the human hepatocytes comprise hepatocytes derived from a chimeric animal with humanized liver, and the method further comprises obtaining the hepatocytes derived from the chimeric animal with humanized liver before the culturing step, wherein the obtaining of the hepatocytes derived from the chimeric animal with humanized liver comprises isolating hepatocytes derived from a liver of an animal injected with PHH or with previously isolated hepatocytes derived from a humanized liver chimeric animal into a spleen of the animal, thereby obtaining the hepatocytes derived from the chimeric animal with humanized liver.
13. The method of paragraph 12, wherein the hepatocytes derived from the chimeric animal are isolated via collagenase perfusion of the liver of the chimeric animal, and the chimeric animal has a replacement index of at least 10% before the hepatocytes are isolated.
14. A conditioned medium from cultured human hepatocytes (CMHH), prepared by a method according to any one of paragraphs 1-13.
15. A conditioned medium from cultured human hepatocytes (CMHH), comprising a hepatocyte clonal growth medium, wherein the hepatocyte clonal growth medium has been used to culture primary human hepatocytes (PHH), hepatocytes derived from a chimeric animal with humanized liver, or a combination thereof for at least 1 hour.
16. A conditioned medium from cultured human hepatocytes (CMHH), comprising one or more humoral factors secreted by human hepatocytes and a hepatocyte clonal growth medium.
17. The conditioned medium from the CMHH of paragraph 15 or paragraph 16, wherein the hepatocyte clonal growth medium comprises Dulbecco's Modified Eagle's Medium (DMEM) supplemented with L-proline, insulin, dexamethasone, EGF, L-ascorbic acid 2-phosphate (Asc-2P), and dimethyl sulfoxide (DMSO) or dimethyl sulfone (DMSO2).
18. A combination, comprising a conditioned medium from CMHH of any one of paragraphs 14-17 and extracellular matrices.
19. A combination, comprising a conditioned medium from CMHH of any one of paragraphs 14-17 and five chemicals of forskolin, SB431542, DAPT, IWP2, and LDN193189.
20. A conditioned medium from cultured human hepatocytes (CMHH) for use in cultivating terminally differentiated human hepatocytes, hepatocytes derived from a chimeric animal with humanized liver, and/or cryopreserved primary human hepatocytes.
21. A conditioned medium from cultured human hepatocytes (CMHH) for use in differentiating hepatic bipotential progenitor cells (iHep) and/or chemically induced liver progenitors into terminally differentiated hepatocytes or matured hepatocytes, respectively.
22. A conditioned medium from cultured human hepatocytes (CMHH) for use in co-culturing with nonparenchymal cells, and/or for use in suspending human hepatocytes in cryopreservation.
23. A method of culturing human hepatocytes (HH) for recovery after isolation and/or preservation and establishment of hepatic function, the method comprising:
   culturing a quantity of HH in a first phase with a first cell culture medium for a first period of time,
   removing the first cell culture medium from the quantity of HH, followed by
   culturing the quantity of HH in a second phase with a second cell culture medium for a second period of time,
   wherein the first cell culture medium comprises a dimethyl sulfoxide (DMSO)-supplemented cell culture medium, the second cell culture medium comprises a dimethyl sulfone (DMSO2)-supplemented cell culture medium or a tetramethylene sulfoxide (TMSO)-supplemented cell culture medium, and
   wherein the second cell culture medium does not comprise DMSO.
24. The method of paragraph 23, wherein the first period of time is at least 4 days and up to 2 months, and wherein the quantity of HH cultured in the first phase establishes cellular polarity, inter-cellular structure characterized by bile canaliculi, and/or a gene expression level equivalent to control HH exhibiting hepatic function.
25. The method of paragraph 24, wherein the first period of time is about 7 days, and the first cell culture medium is optionally refreshed every 3 or 4 days.
26. The method of paragraph 23, wherein the quantity of HH cultured in the second phase has a physiological level of expression of cytochrome P450 family 3 subfamily A member 4 (CYP3A4) and cytochrome P450 family 2 subfamily E member 1 (CYP2E1); or the quantity of HH cultured in the second phase metabolizes alcohol, xenobiotics, vitamin A, or a combination thereof.
27. The method of any one of paragraphs 23-25, wherein the first cell culture medium is a DMSO-supplemented hepatocyte culture medium, which includes DMSO, Dulbecco's Modified Eagle's Medium (DMEM), and one or more of L-proline, insulin, dexamethasone, epidermal growth factor (EGF), L-ascorbic acid 2-phosphate (Asc-2P), 4-(2-hydroxyethyl)-1-piperazineethanesulfonic acid (HEPES), penicillin-streptomycin, and heat-inactivated fetal bovine serum (FBS).
28. The method of paragraph 23 or paragraph 26, wherein the second cell culture medium is a DMSO2-supplemented hepatocyte culture medium, which includes DMSO2, DMEM, and one or more of L-proline, insulin, dexamethasone, EGF, Asc-2P, HEPES, penicillin-streptomycin, and heat-inactivated FBS.
29. The method of paragraph 23, wherein the second cell culture medium contains the DMSO2 at a final concentration of about 140 mM.
30. The method of paragraph 23, wherein the first cell culture medium comprises the DMSO at 2 (v/v) %, the DMEM, the L-proline at 15 µg/mL, the insulin at 0.25 µg/mL, the dexamethasone at 50 nM, the EGF at 5 ng/mL, the Asc-2P at 0.1 mM, the HEPES at 20 mM, the penicillin at 100 IU/mL, the streptomycin at 100 µg/mL, and the heat-inactivated FBS at 10%; and
   wherein the second cell culture medium comprises the DMSO2 at 140 mM, the DMEM, the L-proline at 15 µg/mL, the insulin at 0.25 µg/mL, the dexamethasone at 50 nM, the EGF at 5 ng/mL, the Asc-2P at 0.1 mM, the HEPES at 20 mM, the penicillin at 100 IU/mL, the streptomycin at 100 µg/mL, and the heat-inactivated FBS at 10%.
31. The method of paragraph 23, wherein the first cell culture medium comprises the DMSO at 0.5%-5% (v/v), the DMEM, the L-proline at 5-25 µg/mL, the insulin at 0.1-0.5 µg/mL, the dexamethasone at 10-100 nM, the EGF at 1-10 ng/mL, the Asc-2P at 0.01-1 mM, the HEPES at 10-50 mM, the penicillin at 10-300 IU/mL, the streptomycin at 10-300 µg/mL, and the heat-inactivated FBS at 2-20%; and
   wherein the second cell culture medium comprises the DMSO2 at 100-180 mM, the DMEM, the L-proline at 5-25 µg/mL, the insulin at 0.1-0.5 µg/mL, the dexamethasone at 10-100 nM, the EGF at 1-10 ng/mL, the Asc-2P at 0.01-1 mM, the HEPES at 10-50 mM, the penicillin at 10-300 IU/mL, the streptomycin at 10-300 µg/mL, and the heat-inactivated FBS at 2-20%.
32. The method of paragraph 23, for use in assaying performance of a candidate agent in promoting alcohol metabolism by HH or toxicity of the candidate agent to HH, by contacting the quantity of HH cultured in the second phase in the second cell culture medium with the candidate agent, measuring a first level of metabolizing an alcohol or excretion of the candidate agent by the quantity of HH before the contact with the candidate agent, and measuring a second level of metabolizing the alcohol or excretion of the candidate agent by the quantity of HH in the presence of or after the contact with the candidate agent.
33. A human hepatocyte culture medium for establishment and maintenance of hepatic functions of human hepatocytes (HH), comprising a dimethyl sulfone (DMSO2)-supplemented cell culture medium or a tetramethylene sulfoxide (TMSO)-supplemented cell culture medium, which does not include dimethyl sulfoxide (DMSO).
34. The human hepatocyte culture medium of paragraph 33, wherein the DMSO2 is supplemented to reach a concentration of about 140 mM or about 2 (v/v) % in DMEM, wherein the DMEM further includes one or more of L-proline, insulin, dexamethasone, EGF, Asc-2P, HEPES, penicillin-streptomycin, and heat-inactivated FBS.
35. The human hepatocyte culture medium of paragraph 34, which comprises the DMSO2 at 140 mM, the L-proline at 15 µg/mL, the insulin at 0.25 µg/mL, the dexamethasone at 50 nM, the EGF at 5 ng/mL, the Asc-2P at 0.1 mM, the HEPES at 20 mM, the penicillin at 100 IU/mL, the streptomycin at 100 µg/mL, and the heat-inactivated FBS at 10% in the DMEM.
36. The human hepatocyte culture medium of paragraph 34, which comprises the DMSO2 at 100-180 mM, the L-proline at 5-25 µg/mL, the insulin at 0.1-0.5 µg/mL, the dexamethasone at 10-100 nM, the EGF at 1-10 ng/mL, the Asc-2P at 0.01-1 mM, the HEPES at 10-50 mM, the penicillin at 10-300 IU/mL, the streptomycin at 10-300 µg/mL, and the heat-inactivated FBS at 2-20% in the DMEM.
37. A kit for culturing human hepatocytes, comprising:
   a first container storing a first cell culture medium, said first cell culture medium is supplemented with DMSO; and
   a second container storing a second cell culture medium, said second cell culture medium is supplemented with DMSO2 and/or TMSO and without DMSO.
38. A method of assaying toxicity of an agent to human hepatocytes (HH) or drug metabolism and pharmacokinetics (DMPK) of the agent, comprising:
   culturing a quantity of HH in a medium supplemented with DMSO2, without DMSO;
   contacting the agent with the quantity of HH cultured in the DMSO2-supplemented medium; and
   measuring a level of toxicity to the quantity of HH in the presence of, or after the contact with, the agent, or measuring a level of metabolism or excretion of the agent by the quantity of HH in the presence of, or after the contact with, the agent.
39. The method of paragraph 38, wherein the agent is an alcohol compound comprising ethanol, methanol, ethylene glycol, isopropanol, or a mixture thereof.
40. The method of paragraph 39, wherein the contacting and the measuring are performed in a tight-sealing apparatus.
41. The method of paragraph 38, wherein the level of toxicity is measured via quantifying expression or function of alcohol dehydrogenase (ADH) in the quantity of HH.
42. A method of assaying a candidate agent for promoting metabolism of alcohol or vitamin A, comprising:
   culturing a quantity of HH in a medium supplemented with DMSO2, without DMSO;
   contacting the quantity of HH cultured in the DMSO2-supplemented medium with the alcohol or the vitamin A in the presence of candidate agent; and
   measuring a level of metabolism of the alcohol or the vitamin A by the quantity of HH in the presence of, or after the contact with, the candidate agent.

Some embodiments of the technology described herein can be defined according to any of the following numbered paragraphs:
1. A method for preparing a conditioned medium from cultured human hepatocytes (HH), comprising:
   culturing HH in a medium to generate a culture medium, and
   harvesting the culture medium having been incubated with the HH for at least 1 hour, as the conditioned medium.
2. The method of paragraph 1, wherein the HH comprise primary human hepatocytes (PHH), hepatocytes derived from a chimeric animal with humanized liver, or a combination thereof.
3. The method of paragraph 2, wherein the hepatocytes derived from the chimeric animal with humanized liver are humanized liver chimeric mice derived-human hepatocytes (HLCM-HH), which are obtained from a liver derived from a mouse injected with PHH or with previously isolated HLCM-HH into the spleen.
4. The method of paragraph 3, wherein the mouse has a replacement index of at least 10% before the HLCM-HH are obtained.
5. The method of paragraph 1, wherein the medium is a dimethyl sulfoxide (DMSO), dimethyl sulfone (DMSO2), or tetramethylene sulfoxide (TMSO)-supplemented hepatocyte clonal growth medium (dHCGM, d2HCGM, or tHCGM), and the HCGM comprises, or consists essentially of, a standard cell culture base medium, L-proline, insulin, dexamethasone, EGF, and L-ascorbic acid 2-phosphate (Asc-2P).
6. The method of paragraph 1, wherein the medium is a DMSO, DMSO2, or TMSO-supplemented hepatocyte maintenance medium (dHMM, d2HMM, tHMM), and the HMM comprises, or consists essentially of, a standard cell culture base medium, L-proline, insulin, dexamethasone, and L-ascorbic acid 2-phosphate (Asc-2P).
7. The method of any of paragraphs 5 or 6, wherein the standard cell culture base medium is selected from the group including DMEM, MEM, RPMI-1640, IMDM, or William's E Medium.
8. The method of paragraph 7, wherein the standard cell culture base medium is supplemented with HEPES, penicillin-streptomycin, and serum.
9. The method of paragraph 7, wherein the standard cell culture base medium is DMEM, and the DMEM is DMEM-10, and the DMEM-10 comprises, or consists essentially of, DMEM, HEPES, penicillin-streptomycin, and serum.
10. The method of any of paragraphs 8 or 9, wherein the serum is fetal bovine serum (FBS) or human serum.
11. The method of paragraph 5, wherein dHCGM, d2HCGM, or tHCGM comprises, or consists essentially of L-proline at 15 µg/mL, insulin at 0.25 µg/mL, dexamethasone at 50 nM, EGF at 5 ng/mL, Asc-2P at 0.1 mM, DMSO, DMSO2, or TMSO at 281.6 mM, 140.8 mM, or 70.4 mM respectively, HEPES at 20 mM, penicillin at 100 IU/mL, streptomycin at 100 µg/mL, and the serum at 10% in the standard cell culture base medium.
12. The method of paragraph 5, wherein dHCGM, d2HCGM, tHCGM comprises, or consists essentially of L-proline at 5-25 µg/mL, insulin at 0.1-0.5 µg/mL, dexamethasone at 10-100 nM, EGF at 1-10 ng/mL, Asc-2P at 0.01-1 mM, DMSO, DMSO2, or TMSO at 50 - 300 mM, HEPES at 10-50 mM, penicillin at 10-300 IU/mL, streptomycin at 10-300 µg/mL, and the serum at 2-20% in the standard cell culture base medium.
13. The method of paragraph 6, wherein dHMM, d2HMM, tHMM comprises, or consists essentially of L-proline at 15 µg/mL, insulin at 0.25 µg/mL, dexamethasone at 50 nM, Asc-2P at 0.1 mM, DMSO, DMSO2, or TMSO at 281.6 mM, 140.8 mM, or 70.4 mM respectively, HEPES at 20 mM, penicillin at 100 IU/mL, streptomycin at 100 µg/mL, and the serum at 10% in the standard cell culture base medium.
14. The method of paragraph 6, wherein dHMM, d2HMM, tHMM comprises, or consists essentially of L-proline at 5-25 µg/mL, insulin at 0.1-0.5 µg/mL, dexamethasone at 10-100 nM, Asc-2P at 0.01-1 mM, DMSO, DMSO2, TMSO at 50 - 300 mM, HEPES at 10-50 mM, penicillin at 10-300 IU/mL, streptomycin at 10-300 µg/mL, and serum at 2-20% in the standard cell culture base medium.
15. The method of any one of paragraphs 1-14, wherein medium is replaced with a fresh volume every 24 to 120 hours.
16. The method of paragraph 2, wherein the PHH, the hepatocytes derived from the chimeric animal with humanized liver, or the combination are cultured at a cell density from 0.5×10⁵/cm² to 5×10⁵/cm².
17. The method of any of paragraph 1-3, wherein the HH do not comprise HepG2 cells, Huh7 cells or murine hepatocytes.
18. The method of paragraph 1, wherein the HH comprise hepatocytes derived from a chimeric animal with humanized liver, and the method further comprises obtaining the hepatocytes derived from the chimeric animal with humanized liver before the culturing step, wherein the obtaining of the hepatocytes derived from the chimeric animal with humanized liver comprises isolating hepatocytes derived from a liver of an animal injected with PHH or with previously isolated hepatocytes derived from a humanized liver chimeric animal into a spleen of the animal, thereby obtaining the hepatocytes derived from the chimeric animal with humanized liver.
19. The method of paragraph 18, wherein the hepatocytes derived from the chimeric animal are isolated via collagenase perfusion of the liver of the chimeric animal, and the chimeric animal has a replacement index of at least 10% before the HH are isolated.
20. A conditioned medium from cultured human hepatocytes (CMHH), prepared by a method according to any one of paragraphs 1-19.
21. A CMHH, wherein the CMHH comprises a hepatocyte clonal growth medium or hepatocyte maintenance medium, wherein the medium has been used to culture primary human hepatocytes (PHH), hepatocytes derived from a chimeric animal with humanized liver, or a combination thereof for at least 1 hour.
22. A CMHH of paragraph 21, wherein the CMHH comprises one or more humoral factors secreted by human hepatocytes and a hepatocyte clonal growth medium or a hepatocyte maintenance medium.
23. The CMHH of any of paragraphs 20-22, wherein the hepatocyte clonal growth medium comprises a standard cell culture base medium supplemented with L-proline, insulin, dexamethasone, EGF, Asc-2P, HEPES, penicillin, streptomycin, serum, and DMSO, DMSO2, or TMSO.
24. The CMHH of any of paragraphs 20-22, wherein the hepatocyte maintenance medium comprises a standard cell culture base medium supplemented with L-proline, insulin, dexamethasone, Asc-2P, HEPES, penicillin, streptomycin, serum, and DMSO, DMSO2, or TMSO.
25. A combination, comprising a CMHH of any one of paragraphs 20-24 and extracellular matrices.
26. A combination, comprising a CMHH of any one of paragraphs 20-24 and five chemicals of forskolin, SB431542, DAPT, IWP2, and LDN193189.
27. A CMHH of any one of paragraphs 20-24, for use in cultivating freshly isolated terminally differentiated HH, PHH, hepatocytes derived from a chimeric animal with humanized liver, or primary hepatocytes from other species of animals.
28. A CMHH of any of paragraphs 20-24, for use in cultivating cryopreserved terminally differentiated HH, PHH, hepatocytes derived from a chimeric animal with humanized liver, or primary hepatocytes from other species of animals.
29. A CMHH of any of paragraphs 20-24, for use in differentiating hepatic bipotential progenitor cells (iHep) and/or chemically induced liver progenitors into terminally differentiated hepatocytes, hepatoma cells such as HeparRG^{™}, or matured hepatocytes, respectively.
30. A CMHH of any of paragraphs 20-24, for use in co-culturing with nonparenchymal cells.
31. A CMHH of any of paragraphs 20-24, for use in suspending HH in cryopreservation.
32. A method of culturing HH for recovery after isolation and/or preservation and establishment of hepatic function, the method comprising:
   culturing a quantity of HH in a first phase with a first cell culture medium for a first period of time,
   removing the first cell culture medium from the quantity of HH, followed by culturing the quantity of HH in a second phase with a second cell culture medium for a second period of time,
   wherein the first cell culture medium comprises a DMSO-supplemented cell culture medium, and the second cell culture medium comprises a DMSO, DMSO2, or TMSO-supplemented cell culture medium.
33. A method of culturing HH for recovery after isolation and/or preservation and establishment of hepatic function, the method comprising:
   culturing a quantity of HH in a first phase with a first cell culture medium for a first period of time,
   removing the first cell culture medium from the quantity of HH, followed by culturing the quantity of HH in a second phase with a second cell culture medium for a second period of time,
   wherein the first cell culture medium comprises a dimethyl sulfoxide (DMSO)-supplemented cell culture medium, the second cell culture medium comprises a DMSO2 or TMSO-supplemented cell culture medium, and
   wherein the second cell culture medium does not comprise DMSO.
34. The method of paragraphs 32 or 33, wherein the first period of time is at least 4 days and up to 2 months, and wherein the quantity of HH cultured in the first phase establishes cellular polarity, inter-cellular structure characterized by bile canaliculi, and/or a gene expression level equivalent to control HH exhibiting hepatic function of matured hepatocytes or the liver tissue of healthy individuals.
35. The method of paragraphs 32 or 33, wherein the first period of time is about 7 days, and the first (recovery) phase cell culture medium is optionally refreshed every 3 or 4 days.
36. The method of paragraphs 32 or 33, wherein the quantity of HH cultured in the second phase has a physiological level of expression of hepatocyte metabolic genes such as, but not limited to, alcohol dehydrogenases (ADHs), aldehyde dehydrogenases (ALDHs), cytochrome P450 family 3 subfamily A member 4 (CYP3A4) and cytochrome P450 family 2 subfamily E member 1 (CYP2E1); or the quantity of HH cultured in the second phase metabolizes alcohol, xenobiotics, vitamin A, or a combination thereof.
37. The method of any one of paragraphs 32-36, wherein the first cell culture medium is a DMSO-supplemented hepatocyte culture medium or CMHH, which includes DMSO, a standard cell culture base medium, and one or more of L-proline, insulin, dexamethasone, EGF, Asc-2P, HEPES, penicillin-streptomycin, human serum and/or FBS.
38. The method of any of paragraphs 32-36, wherein the second cell culture medium is a DMSO, DMSO2, or TMSO-supplemented hepatocyte culture medium or CMHH, which includes DMSO, DMSO2, or TMSO, a standard cell culture base medium, and one or more of L-proline, insulin, dexamethasone, EGF, Asc-2P, HEPES, penicillin-streptomycin, human serum and/or heat-inactivated FBS.
39. The method of any of paragraphs 32-38, wherein the first or second cell culture medium contains the DMSO at a final concentration of about 2% v/v or 281.6 mM.
40. The method of any of paragraphs 32-38, wherein the second cell culture medium contains the DMSO2 at a final concentration of about 140.8 mM.
41. The method of any of paragraphs 32-38, wherein the second cell culture medium contains the TMSO at a final concentration of about 70 mM
42. The method of any of paragraphs 32-41, wherein the first cell culture medium comprises the DMSO at 2 (v/v) % or 286.1 mM, the L-proline at 15 µg/mL, the insulin at 0.25 µg/mL, the dexamethasone at 50 nM, the EGF at 5 ng/mL, the Asc-2P at 0.1 mM, the HEPES at 20 mM, the penicillin at 100 IU/mL, the streptomycin at 100 µg/mL, and the serum at 10% in a standard cell culture base medium; and
   wherein the second cell culture medium comprises the DMSO, DMSO2, or TMSO at 281.6 mM, 140.8 mM, or 70.4 mM, respectively, the L-proline at 15 µg/mL, the insulin at 0.25 µg/mL, the dexamethasone at 50 nM, the EGF at 5 ng/mL, the Asc-2P at 0.1 mM, the HEPES at 20 mM, the penicillin at 100 IU/mL, the streptomycin at 100 µg/mL, and the serum at 10% in a standard cell culture base medium.
43. The method of any of paragraphs 32-41, wherein the first cell culture medium comprises the DMSO at 2 (v/v) % or 286.1 mM, the L-proline at 15 µg/mL, the insulin at 0.25 µg/mL, the dexamethasone at 50 nM, the Asc-2P at 0.1 mM, the HEPES at 20 mM, the penicillin at 100 IU/mL, the streptomycin at 100 µg/mL, and the serum at 10% in a standard cell culture base medium; and
   wherein the second cell culture medium comprises the DMSO, DMSO2, or TMSO at 281.6 mM, 140.8 mM, or 70.4 mM, respectively, the L-proline at 15 µg/mL, the insulin at 0.25 µg/mL, the dexamethasone at 50 nM, the Asc-2P at 0.1 mM, the HEPES at 20 mM, the penicillin at 100 IU/mL, the streptomycin at 100 µg/mL, and the serum at 10% in a standard cell culture base medium.
44. The method of any of paragraphs 32-41, wherein the first cell culture medium comprises the DMSO at 0.5%-5% (v/v), the L-proline at 5-25 µg/mL, the insulin at 0.1-0.5 µg/mL, the dexamethasone at 10-100 nM, the EGF at 0-10 ng/mL, the Asc-2P at 0.01-1 mM, the HEPES at 10-50 mM, the penicillin at 10-300 IU/mL, the streptomycin at 10-300 µg/mL, and the serum at 2-20% in a standard cell culture medium; and
   wherein the second cell culture medium comprises the DMSO, DMSO2, or TMSO, at 50-300 mM, the L-proline at 5-25 µg/mL, the insulin at 0.1-0.5 µg/mL, the dexamethasone at 10-100 nM, the EGF at 0-10 ng/mL, the Asc-2P at 0.01-1 mM, the HEPES at 10-50 mM, the penicillin at 10-300 IU/mL, the streptomycin at 10-300 µg/mL, and the serum at 2-20% in a standard cell culture base medium.
45. The method of any of paragraphs 32-44, for use in assaying performance of a candidate agent in promoting alcohol metabolism by HH or toxicity of the candidate agent to HH, by contacting the quantity of HH cultured in the second phase in the second cell culture medium with the candidate agent, measuring a first level of metabolizing an alcohol or excretion of the candidate agent by the quantity of HH before the contact with the candidate agent, and measuring a second level of metabolizing the alcohol or excretion of the candidate agent by the quantity of HH in the presence of or after the contact with the candidate agent.
46. A HH culture medium for establishment and maintenance of hepatic functions of HH, comprising a DMSO2 or TMSO-supplemented cell culture medium, which does not include DMSO.
47. The HH culture medium of paragraph 46, wherein the DMSO2 or TMSO is supplemented to reach a concentration of about 140.8 mM or 70.4 mM, respectively, in a standard cell culture base medium, wherein the base medium further includes one or more of L-proline, insulin, dexamethasone, EGF, Asc-2P, HEPES, penicillin-streptomycin, human serum, and/or heat-inactivated FBS.
48. The HH culture medium of paragraphs 46 or 47, comprising the DMSO2 at 140.8 mM, the L-proline at 15 µg/mL, the insulin at 0.25 µg/mL, the dexamethasone at 50 nM, the EGF at 5 ng/mL, the Asc-2P at 0.1 mM, the HEPES at 20 mM, the penicillin at 100 IU/mL, the streptomycin at 100 µg/mL, and the serum at 10% in a standard cell culture base medium.
49. The HH culture medium of paragraphs 46 or 47, comprising the TMSO at 70.4 mM, the L-proline at 15 µg/mL, the insulin at 0.25 µg/mL, the dexamethasone at 50 nM, the EGF at 5 ng/mL, the Asc-2P at 0.1 mM, the HEPES at 20 mM, the penicillin at 100 IU/mL, the streptomycin at 100 µg/mL, and the serum 10% in a standard cell culture base medium.
50. The HH culture medium of any of paragraphs 46-49, comprising the DMSO2 or TMSO at 50-200 mM, the L-proline at 5-25 µg/mL, the insulin at 0.1-0.5 µg/mL, the dexamethasone at 10-100 nM, the EGF at 1-10 ng/mL, the Asc-2P at 0.01-1 mM, the HEPES at 10-50 mM, the penicillin at 10-300 IU/mL, the streptomycin at 10-300 µg/mL, and serum at 2-20% in a standard cell culture base medium.
51. A kit for culturing HH, comprising:
   a first container storing a first cell culture medium, said first cell culture medium is supplemented with DMSO; and
   a second container storing a second cell culture medium, said second cell culture medium is supplemented with DMSO, DMSO2, and/or TMSO.
52. A kit for culturing human hepatocytes, comprising:
   a first container storing a first cell culture medium, said first cell culture medium is supplemented with DMSO; and
   a second container storing a second cell culture medium, said second cell culture medium is supplemented with DMSO2 and/or TMSO, without DMSO.
53. A method of assaying toxicity of an agent to HH or drug metabolism and pharmacokinetics (DMPK) of the agent, comprising:
   culturing a quantity of HH in a medium supplemented with DMSO2, without DMSO;
   contacting the agent with the quantity of HH cultured in the DMSO2-supplemented medium; and
   measuring a level of toxicity to the quantity of HH in the presence of, or after the contact with, the agent, or measuring a level of metabolism or excretion of the agent by the quantity of HH in the presence of, or after the contact with, the agent.
54. The method of paragraph 53, wherein the agent is an alcohol compound comprising ethanol, methanol, ethylene glycol, isopropanol, or a mixture thereof.
55. The method of paragraphs 53 or 54, wherein the contacting and the measuring are performed in a tight-sealing apparatus.
56. The method of any of paragraphs 53-55, wherein the level of toxicity is measured via quantifying expression or function of xenobiotics-metabolizing enzymes such as, but not limited to Cytochrome P450 (CYP) enzymes in the quantity of HH.
57. The method of any of paragraphs 53-55, wherein the level of toxicity is measured via quantifying the toxic metabolites of alcohol such as acetaldehyde, or reduction of glutathione, the activation status of cell death pathways in the quantity of HH.
58. A method of assaying a candidate agent for promoting the metabolism of alcohol or vitamin A, comprising:
   culturing a quantity of HH in a medium supplemented with DMSO2, without DMSO;
   contacting the quantity of HH cultured in the DMSO2-supplemented medium with the alcohol or the vitamin A in the presence of a candidate agent; and
   measuring a level of metabolism of the alcohol or the vitamin A by the quantity of HH in the presence of, or after the contact with, the candidate agent.
59. The method of any of paragraphs 32-58, wherein the medium used is a conditioned medium from HH from any of paragraphs 20-24.

### EXAMPLES

The following examples are provided to better illustrate the claimed invention and are not to be interpreted as limiting the scope of the invention. To the extent that specific materials are mentioned, it is merely for purposes of illustration and is not intended to limit the invention. One skilled in the art may develop equivalent means or reactants without the exercise of inventive capacity and without departing from the scope of the invention.

### Example 1. Compositions and regimen for a two-step culturing method of HH.

We have discovered that the maintenance hepatocytes' cell fate and hepatic function requires a two-step culture method, in which Phase 1 is the term to promote the recovery from the cell injury taking place during the cell isolation procedure and freeze-thaw process. This Phase requires at least 4 days, preferably 7 days of in vitro culture. During Phase 1, with our method using an "HH recovery medium" (Tables 1-1 and 1-3), HH reestablished the cellular polarity and inter-cellular structures such as but not limited to the bile canaliculi. In addition to the morphological recovery, Phase 1 also allowed HH to regain the hepatocyte characteristics at the level of gene transcription and protein expression. We stringently compared the robustness of our Phase 1 culture medium with other publicly available culture media of HH and found that our method is substantially superior to these conditions (commercially available hepatocytes culture medium, such as HCM^{™} hepatocyte culture medium bulletkit or HMM^{™} hepatocyte basal medium, both from LONZA, or CELLARTIS^{®} POWER^{™} primary hepatocyte medium from TAKARA) (Figure 1). We also found that the culture condition for Phase 1 allows the cell fate maintenance of in vitro cultured HH up to 2 months. However, we also discovered that our Phase I culture condition substantially prohibits metabolic functions of hepatocytes, such as but not limited to the alcohol metabolism and drug metabolism (Figure 2 and 3). This notation indicates that the culturing condition that allows for the recovery and cell fate maintenance compromises the versatility of HH as an experimental tool.

**Table 1-1. Composition of Phase 1 (recovery phase), Hepatocyte Clonal Growth Medium (DMSO-containing).**

| | | Volume. | Final Conc. |
|---|---|---|---|
| **1.** | DMEM10 or a similar or equivalent thereof | 500 mL | |
| **2.** | L-Proline Stock Solution (30 mg/mL) | 250 µL | 15 µg/mL |
| **3.** | Insulin Stock Solution (10 mg/ml) | 12.5 µL | 0.25 µg/mL |
| **4** | Dexamethasone (Dex) Stock Solution (10 mM) | 2.5 µL | 50 nM |
| **5.** | EGF Stock Solution (10 ug/mL) | 250 µL | 5 ng/mL |
| **6.** | L-ascorbic Acid 2-Phosphate (Asc-2P) Stock Solution (0.1 M) | 500 µL | 0.1 mM |
| **7.** | DMSO | 10.2 mL | 2% |

**Table 1-2. Composition of Phase 1 (recovery phase), Hepatocyte Maintenance Medium (DMSO-containing).**

| | | Volume. | Final Conc. |
|---|---|---|---|
| **1.** | DMEM10 or a similar or equivalent thereof | 500 mL | |
| **2.** | L-Proline Stock Solution (30 mg/mL) | 250 µL | 15 µg/mL |
| **3.** | Insulin Stock Solution (10 mg/ml) | 12.5 µL | 0.25 µg/mL |
| **4** | Dexamethasone (Dex) Stock Solution (10 mM) | 2.5 µL | 50 nM |
| **5.** | L-ascorbic Acid 2-Phosphate (Asc-2P) Stock Solution (0.1 M) | 500 µL | 0.1 mM |
| **6.** | DMSO | 10.2 mL | 2% |

**Table 1-3. DMEM10 Media**

| | | Volume. | Final Conc. |
|---|---|---|---|
| **1.** | DMEM (without phenol red) | 450 mL | |
| **2.** | HEPES 1M solution | 10 mL | 20 mM |
| **3.** | Penicillin-Streptomycin | 5 mL | 100 IU/mL, 100 µg/mL |
| **4** | Heat inactivated*¹ FBS Stock Solution*² or Human serum | 50 mL | 10% |

In search of the mechanism by which the Phase 1 culture condition prohibits the fundamental metabolic function of the HH, we identified that DMSO contained in the culture medium is the culprit (Figure 2, 3, and 4). Of important note, we discovered that the withdrawal of DMSO from the culture medium is not feasible as it results in a rapid deterioration of the hepatocytic function and morphology (Figure 5). We also uncovered that culturing HH without DMSO during Phase 1 would not promote the recovery from the cell injury and stress that result from the cell isolation/preservation procedures (Figure 6).

We sought for a substitution for DMSO (i.e., replacing DMSO), and the substitution would be capable of maintaining the cell fate of terminally differentiated HH while not being prohibitive to metabolic functions of hepatocytes. Among numerous organic compounds tested (Table 3; Figure 7 and 8), we identified DMSO2, an oxidized form of DMSO, as a desirable substitution for DMSO. The Phase 2 culture (day 7 and thereafter) with medium containing DMSO2, but not DMSO, allowed HH to metabolize alcohol at a level comparable to that of the human liver (Figure 2 and 4), indicating that our Phase 2 culture medium (containing DMSO2) could serve as a platform for the *in vitro* study of alcoholic liver diseases.

Accordingly, we have established a culturing condition that allows HH to exhibit the fundamental functionality of hepatocytes for Phase 2 (e.g., the study of DMPK and various liver diseases), which does not compromise the cell fate of HH that fully have recovered during Phase 1 (day 0-day 7). See Tables 2-1 and 2-3.

**Table 2-1. Composition of Phase 2 (application phase), Hepatocyte Clonal Growth Medium (DMSO2-containing).**

| | | | Final Conc. |
|---|---|---|---|
| **1.** | DMEM10 or a similar or equivalent thereof | 500 mL | |
| **2.** | L-Proline Stock Solution (30 mg/mL) | 250 µL | 15 µg/mL |
| **3.** | Insulin Stock Solution (10 mg/ml) | 12.5 µL | 0.25 µg/mL |
| **4** | Dexamethasone (Dex) Stock Solution (10 mM) | 2.5 µL | 50 nM |
| **5.** | EGF Stock Solution (10 ug/mL) | 250 µL | 5 ng/mL |
| **6.** | L-ascorbic Acid 2-Phosphate (Asc-2P) Stock Solution (0.1 M) | 500 µL | 0.1 mM |
| **7.** | DMSO₂ | 6.38 g | 140.8 mM |

**Table 2-2. Composition of Phase 2 (application phase), Hepatocyte Maintenance Medium (DMSO2-containing).**

| | | | Final Conc. |
|---|---|---|---|
| **1.** | DMEM10 or a similar or equivalent thereof | 500 mL | |
| **2.** | L-Proline Stock Solution (30 mg/mL) | 250 µL | 15 µg/mL |
| **3.** | Insulin Stock Solution (10 mg/ml) | 12.5 µL | 0.25 µg/mL |
| **4** | Dexamethasone (Dex) Stock Solution (10 mM) | 2.5 µL | 50 nM |
| **5.** | L-ascorbic Acid 2-Phosphate (Asc-2P) Stock Solution (0.1 M) | 500 µL | 0.1 mM |
| **6.** | DMSO₂ | 6.38 g | 140.8 mM |

**Table 2-3. DMEM10 Media**

| | | Volume. | Final Conc. |
|---|---|---|---|
| **1.** | DMEM (without phenol red) | 450 mL | |
| **2.** | HEPES 1M solution | 10 mL | 20 mM |
| **3.** | Penicillin-Streptomycin | 5 mL | 100 IU/mL, 100 µg/mL |
| **4** | Heat inactivated*¹ fetal bovine serum (FBS) Stock Solution*² or Human serum | 50 mL | 10% |

In addition, we found that HH cultured with the phase 1 culture medium (containing DMSO) promoted the expression of CYP3A4 and CYP2E1 to a supraphysiological level, and that HH cultured with a DMSO-containing medium strongly prohibited the metabolic functions of these enzymes (Figure 6 and 9). As these enzymes are central players in drug metabolism and also drug toxicity studies, the DMSO-containing medium abolishes the versatility of *in vitro* cultured HH as a tool for the study of DMPK. In contrast, HH cultured with the DMSO₂-containing medium showed a much superior capacity in metabolizing xenobiotics to that of HH cultured with DMSO (Figure 3). Therefore, the DMSO₂-containing medium allows researchers to apply *in vitro* cultured HH for the study of DMPK. These results collectively indicated that the cell culture medium with DMSO₂ has a great potential for commercialization.

Overall, the culture condition suitable for the recovery of HH (the phase 1) helps regain and maintain the characteristics of terminally differentiated HH, but limits the versatility of HH as a research tool due to the potent prohibitory effect of DMSO on numerous enzymatic activities of HH. The affected metabolic pathways include, but not limited to, the alcohol metabolic pathway and xenobiotics metabolism pathways, and thereby hampering the utility of HH in the studies of alcoholic liver disease (ALD) and DMPK, respectively (Figure 2, 3, 4, and 9).

**Table 3. List of chemicals/organic compound tested in identifying substitution for DMSO.**

| Chemicals/Products name | Abbreviations | Manufacture | Cat No. | Conc. |
|---|---|---|---|---|
| | DMSO | Sigma | D2650 | 281.6 mM |
| dimethyl sulfone | DMSO2 | Sigma | M81705 | 140.8 mM |
| dimethyl sulfide | DMS | Sigma | 471577 | 281.6 mM |
| N,N-Dimethylformamide | DMF | Sigma | D4551 | 281.6 mM |
| Dimethylacetamide | DMA | Sigma | 271012 | 70.4 mM |
| tert-Butanol | TBA | Sigma | 471712 | 281.6 mM |
| N-Acetyl-L-cysteine | NAC | Sigma | A9165 | 10 mM |
| Ascorbic acid | VtC | Sigma | A8960 | 10 mM |
| a-tocopherol | VtE | Sigma | T1157 | 1 mM |
| Antioxidant Supplement | Supplement | Sigma | A1345 | x1000 dilution |
| Diethylene glycol dimethyl ether | DEGDME | Sigma | 04143 | 56.32 mM |
| 1,2-Dimethoxyethane, inhibitor-free | DME | Sigma | 259527 | 281.6 mM |
| Diethylene glycol | DEG | Sigma | 93171 | 140.8 mM |
| Pyridine | PY | Sigma | 270970 | 56.32 mM |
| Pyridine hydrochloride | PYH | Sigma | 307475 | 28.16 mM |
| Acetonitrile | ACN | Sigma | 271004 | 281.6 mM |
| 1,4-dioxane | DEO | Sigma | 296309 | 281.6 mM |
| Glyceryl triacetate | GTA | Sigma | 90240 | 5.632 mM |
| Poly(ethylene glycol) large PEG 20000 | Small-PEG | Sigma | 95172 | 2.5 mM |
| Poly(ethylene glycol) small PEG 400 | Large-PEG | Sigma | 91893 | 62.5 mM |
| Poly(vinyl alcohol) | PVA | Sigma | P8136 | 0.5 mM |
| Sulfolane | SL | Sigma | T22209 | 70.4 mM |
| Taurine | Tau | Sigma | T0625 | 140.8 mM |
| Tetramethylene sulfoxide 97% | TMSO | Alfa Aesar | A17502 | 140.8 mM |
| L-Methionine sulfoxide | MetO | Alfa Aesar | J62873-03 | 281.6 mM |
| 1,4-Thioxane 1,1-dioxide | DiO | TCI America | D2108 | 70.4 mM |
| Dimethylsulfoximine | DMSOM | TCI America | D4971 | 281.6 mM |

### Example 2. The use of DMSO2-containing medium for the study of ALD.

The phase 1 medium with DMSO significantly prohibits two major alcohol metabolic pathways, i.e., ADH pathway and CYP2E1 pathway. There has not been a method (culture condition) that allows researchers to utilize the recovered HH for the study of ALD, a most significant cause of chronic liver disease (CLD) (nearly 50% of CLD in the US is due to ALD) with no effective therapeutic interventions. Therefore, the establishment of a culture condition suitable for the study of ALD is a technical breakthrough with tremendous commercialization potential.

In search of the DMSO substitution, we screened numerous organic compounds. The list of chemicals (Table 3) is selected based on the shared characteristics with DMSO, such as but not limited to the molecular size, solubility to the cell culture medium, stability at the standard temperature (melting and boiling temperatures), feasibility in handling (no significant chemical hazardous concern), and the cost. These chemicals were added to the cell culture medium as a substitution for DMSO, and first assayed for a highest concentration that does not elicit a direct toxicity to the recovered HH. With the highest possible concentration, recovered HH (7 days old) were cultured for additional 7 and 14 days with refreshing of the medium at a frequency of every 3-4 days. On day 14 and 21, cells were subjected to the morphological and cell fate assessment along with the functional assessment (Figure 7 and 8).

Of those tested, TMSO and DMSO2 are only two conditions that are capable of maintaining the morphology and the marker genes expression at a level comparable to that of DMSO-containing medium (Figure 7 and 8). Our follow up experiment revealed that the TMSO-containing medium is as equally prohibitive to the alcohol metabolizing pathways as the DMSO-containing medium (Figure 10), whereas the DMSO2-containing medium allows the HH to consume ethanol at a level resembling the capacity of the human liver (Figure 4).

Our research also discovered that it is important to perform the *in vitro* alcohol treatment on HH with tight-sealing apparatus (Figure 11). Due to the high volatility of ethanol, it evaporates rapidly at a much higher rate that the rate of hepatocytes metabolizes. Consequently, ethanol treated HH cultured with DMSO2-containing medium develops accumulation of lipid droplet (LD), the hallmark phenotype of ALD, only when the tight-sealing apparatus is applied. The use of the tight-sealing apparatus for the study of ALD is another aspect of technical innovation.

The substitution of DMSO2 for DMSO (i.e., replacement of DMSO with DMSO2) for culturing HH, once the cells have achieved a full recovery (with a DMSO-containing medium for at least ~4 days, but preferably for 7 days), allows HH to metabolize alcohol (ethanol) (Figure 2, 3, and 4). The DMSO2-containing medium also allows HH to test the metabolism and toxicities of other types of alcohol, such as methanol, ethylene glycol, and isopropanol, which are highly relevant for the human toxicology. The DMSO2-containing medium allows HH to metabolize vitamin A (these are also metabolized by ADH), thus allowing HH to be used for the studies of vitamin A biology. The application of the tight-sealing apparatus will be the default experimental condition for the study of ALD (or any metabolic studies with high volatility chemicals) (Figure 12).

### Example 3. Use of DMSO2-containing medium for the study of drug toxicity and DMPK.

DMPK study is one of the most application of in vitro cultured HH as it serves as the critical milestone in the process of drug development. FDA and regulatory authorities in other countries mandates the utilization of HH for the preclinical screening of newly developed compound prior to the clinical trial. Thus, HH is required to have fully functioning drug-metabolic pathways at a level comparable to that of the human liver in order to fulfill its role as the preclinical trial screening tool. In our studies investigating the similarities and differences between HH cultured with DMSO- and DMSO2-containing medium, the results collectively revealed that DMSO-containing medium upregulates genes that are involved in the uptake, metabolism, and excretions of xenobiotics (Figure 1, 6, and 9). For example, our studies showed a marked upregulation of CYP3A4, a most important drug metabolizing enzyme responsible for the metabolism of more than 50% of medicines. In addition, our studies found that DMSO potently prohibits the metabolic activity of CYP3A4, indicating that the supraphysiological induction of drug-metabolizing enzymes might reflect the degree of inhibition (Figure 3 and 9). Such reciprocal changes were also seen in CYP2E1 (Figure 3 and 9), further indicating that the condition with the DMSO-containing medium would not be suitable for the study of DMPK.

Accordingly, our studies demonstrated that cells cultured with DMSO2-containing medium exhibit the expression abundance of CYP3A4 and CYP2E1 at a physiological level, indicating that the HH cultured with DMSO2 maintains the physiological relevance in terms of the drug-metabolizing pathways (Figure 3 and 9). Moreover, the impact of DMSO2 on the xenobiotics metabolic activities of these enzymes are substantially less than that of DMSO-containing medium (Figure 3 and 9).

Our study also revealed that cells cultured with DMOS2 are suitable for the studies of drug-drug interaction and drug-metabolizing enzyme inductions. For example, Carbamazepine, one of the classic anti-seizures (epilepsy) medication, is known to induce CYP3A4 and also is metabolized by CYP3A4. In our observations, on HH cultured with DMSO2, but not with DMSO, recapitulated the CYP3A4 induction in a dose dependent manner over the course of a 7-day carbamazepine treatment. Similar results are noted with Rifampicin. In addition, we observed that HH maintained with DMSO-containing medium, but not DMSO2-containing medium, showed a supraphysiological expression level of CYP2E1 expression, as well as the lack of CYP2E1 induction by their inducers (Ethanol and Isoniazid) (Figure 9).

Taken together, our discovery shows that DMSO2-containing medium greatly expand the versatility of in vitro cultured HH for the study of DMPK. DMSO2- but not DMSO-containing medium serves as an improved platform for the in vitro studies of xenobiotics metabolism. DMSO2-containing medium culture condition allows the detection of xenobiotic metabolizing enzyme induction at a much greater sensitivity over that of DMSO-based medium. DMSO2-containing medium culture condition serves as the experimental platform for the detection of drug toxicities that are developed over the long-term culture. DMSO2-containing medium culture condition offers an improved experiment condition for the detection toxicities that result from drug-drug interaction (poly-pharmacy).

### Example 4. Production of conditioned medium of in vitro cultured human hepatocytes and its unique biological effect

The discovery of the CMHH was made possible with our techniques in the production of HLCM. In brief, the production of one HLCM requires ~10⁵ PHH and/or HLCM-HH, which are injected to the spleen of the host mouse (such as uPA-SCID strain). This results in the robust proliferation of PHH in the liver of host mouse in which endogenous murine hepatocytes with toxic transgene (uPA) overexpression do not grow as a result of the competition with the transplanted human hepatocytes. (After PHH transplantation, endogenous murine hepatocytes diminish; whereas in the host mice without PHH transplant, the murine hepatocytes could grow, not diminishing.) After 8 weeks post-PHH transplant, the liver of the host mouse will be replaced by human hepatocytes up to 95%. Consequently, the liver of one HLCM offers 2-3x10⁸ human hepatocytes, thereby enabling the mass production of HLCM-HH. Thus, our technique provides the stable supply of HLCM-HH that will be used for the mass production of HLCM-HH. In addition, the advantage of HLCM-HH over PHH for the production of CMHH is the viability and the platability (Sugahara G. et al., Semin Liver Dis. 2020 May;40(2):189-212). Accordingly, the use of HLCM-HH has economical advantage over the use of PHH.

**Material and Method:** HLCM is subjected to the quality assessment of the degree of human hepatocytes proliferation in the liver, namely replacement index (R.I.). The serum/blood human albumin level has been shown to well correspond with the R.I. HLCM with blood albumin level greater than 10 mg/ml are considered high-R.I. (>70%). The HLCM, preferentially with high R.I., are subjected to the isolation of human hepatocytes via a collagenase perfusion-based method. Upon the assessment of cell quality (yield and viability), the HLCM-derived human hepatocyte (HLCM-HH) are plated onto a cell culture flask/dish that are coated with collagen (with/without other types of extracellular matrix (ECM)) at the cell density ranging 0.5-5×10⁵ cells/cm² (or in some implementations, 2.1-4.2 x 10⁵ cell/cm²). Similarly, PHH, or HepaRG cells are plated onto the collagen coated cell culture flask/dish. (The HEPARG^{™} cell line is an immortalized hepatic cell line that retains many characteristics of primary human hepatocytes. HEPARG^{™} cells are terminally differentiated and provided in a convenient cryopreserved format.) These cells are cultured with DMSO or DMSO2 (or similar or equivalents thereof)-supplemented HCGM (i.e., dHCGM, d2HCGM, dHMM, or d2HMM) medium (Tables 4-6) (such as but not limited to dHCGM containing DMSO2) for 6 months (e.g., at least 21 days, at least 4, 5, 6, 7, 8 weeks, or about 2, 3, 4, 5, or 6 months). After 4 days (preferably 7 days) cell seeding, the culture medium is replaced every 24-48 hours and the medium incubated with the cells is harvested and stored as conditioned medium of human hepatocytes (CMHH) (Figure 12). The biological effect of CMHH is unique and robust as mentioned in the following sections.

**Table 4. Composition of dHCGM/d2HCGM Cell Culture Medium**

| | | | Final Conc. |
|---|---|---|---|
| **1.** | DMEM10 or a similar or equivalent thereof | 500 mL | |
| **2.** | L-Proline Stock Solution (30 mg/mL) | 250 µL | 15 µg/mL |
| **3.** | Insulin Stock Solution (10 mg/ml) | 12.5 µL | 0.25 µg/mL |
| **4** | Dexamethasone (Dex) Stock Solution (10 mM) | 2.5 µL | 50 nM |
| **5.** | EGF Stock Solution (10 ug/mL) | 250 µL | 5 ng/mL |
| **6.** | L-ascorbic Acid 2-Phosphate (Asc-2P) Stock Solution (0.1 M) | 500 µL | 0.1 mM |
| **7.** | DMSO or DMSO2 or a similar or equivalent thereof | 10.2 mL or 6.38 g, respectively | 2% (DMSO) or 140.8 mM (DMSO2) |

**Table 5. Composition of dHMM/d2HMM Cell Culture Medium**

| | | | Final Conc. |
|---|---|---|---|
| **1.** | DMEM10 or a similar or equivalent thereof | 500 mL | |
| **2.** | L-Proline Stock Solution (30 mg/mL) | 250 µL | 15 µg/mL |
| **3.** | Insulin Stock Solution (10 mg/ml) | 12.5 µL | 0.25 µg/mL |
| **4** | Dexamethasone (Dex) Stock Solution (10 mM) | 2.5 µL | 50 nM |
| **6.** | L-ascorbic Acid 2-Phosphate (Asc-2P) Stock Solution (0.1 M) | 500 µL | 0.1 mM |
| **7.** | DMSO or DMSO2 or a similar or equivalent thereof | 10.2 mL or 6.38 g, respectively | 2% (DMSO) or 140.8 mM (DMSO2) |

**Table 6. DMEM10 Media**

| | | Volume. | Final Conc. |
|---|---|---|---|
| **1.** | DMEM (without phenol red) | 450 mL | |
| **2.** | HEPES 1M solution | 10 mL | 20 mM |
| **3.** | Penicillin-Streptomycin | 5 mL | 100 IU/mL, 100 µg/mL |
| **4** | Heat inactivated*¹ fetal bovine serum (FBS) Stock Solution*² or Human serum | 50 mL | 10% |

| | | | |
|---|---|---|---|
| *1. After thawing FBS, heat it for 30 min at 56°C (to deactivate the complement) *2. Added when use. We recently found that without FBS, or with human serum could result in a comparable or even better biological effect. | | | |

The biological effect of CMHH was assessed using HLCM-HH, which demonstrates that the cellular architecture of HH incubated with CMHH well recapitulates that of terminally differentiated human hepatocytes in the liver tissue of healthy individuals evidenced by reestablishment of the polygonal-cuboidal cellular morphology, and the formation of the bile canaliculi between the tight cell-to-cell contact (Figure 13). In addition, we confirmed the functionality of the bile canaliculi developed in CMHH-treated HH using the fluorescent substrate of MRP2, one of the transporters involved in the bile secretion machinery (Figure 14). Furthermore, HH treated with CMHH revealed the significant upregulation of hepatocyte marker genes, the downregulation of cholangiocytes/hepatic progenitor cell marker genes, and the inhibition of TGF-β/Yap signaling, the pathway known to compromise the cell fate of HH (Figure 15). Moreover, as we expected, HH cultured with CMHH, but not with fresh dHCGM, showed a susceptibility to one of the hepatotropic viral pathogens, hepatitis B virus (HBV) and the response to antiviral drug against HBV (Figure 16 and 17) as well as the enhanced functionality of xenobiotics metabolizing pathways (Figure 18); further indicating that the HH treated with CMHH exhibits the cell fate of bona fide human terminally differentiated human hepatocytes. Lastly, we found that CMHH propagated with DMSO2-contaiming hepatocyte culture medium (dHCGM) possess a comparable level of biological effect compared to that prepared with DMSO-containing medium (Figure 15).

We have also tested the conditioned medium obtained from in vitro cultured conventional hepatoma cell lines (HepG2, Huh7 cells) as well as 293 cells, and mouse primary hepatocyte, all of which lack the biological effect seen in our CMHH (Figure 19 and 20). In contrast, CM propagated with HLCM-HH (two different donors) or PHH (three different donors) were all capable of maintaining the appearance characteristics of matured, terminally differentiated human hepatocytes including the polygonal-cuboidal appearance of matured mono- or binucleated cells with well-established tight cell-cell contact wherein promoting bile canaliculi formation (Figure 21). In addition, CMHH propagated with HLCM-HH (two different donors) or PHH (three different donors) were all capable of supporting the upregulation/maintenance of "hepatocyte marker" genes (CYP2C9, CYP2D6, OATP1B1, and ALDH2) as well as the suppression of dedifferentiation/transdifferentiation genes (TGFβ1, TGFβ2, Cyr61, CTGF, and CK19) (Figure 22).

There have been a few methodologies proposed to enhance the maturation and function of in vitro cultured hepatocytes such as Matrigel^{®} and five chemicals combination (namely, 5C) (Xiang C., Science. 2019 Apr 26;364(6438):399-402). In comparison, our product (CMHH) exhibited substantially more robust effects on the maintenance of the appearance characteristics of matured HH (Figure 23). Furthermore, our gene expression analysis revealed that the effect of CMHH is highly distinctive from that of Matrigel^{®} or 5C (five chemicals: forskolin (FSK; an adenylate cyclase activator), SB431542 (SB43; TGF-β inhibitor), DAPT (Notch inhibitor), IWP2 (Wnt inhibitor), and LDN193189 (BMP inhibitor)) (Figure 24). Our analysis revealed that the biological effect of Matrigel^{®} and 5C are distinctive and weak from that of CMHH, especially in the prevention of dedifferentiation/transdifferentiation of HH.

We have also analyzed the humoral factor(s) present in the CMHH. First the analysis of CM filtered through size exclusion columns indicates that the key biologically active composition of CMHH responsible for the cell fate and functional maintenance of HH largely mediated through protein molecules secreted from HH (Figure 25). The assessment of protein molecules contained in CMHH reveal that high abundance of LAMC2, FGA, SERPINA3, and TGM2, which is a sharp contrast to that of Matrigel^{®} (Figure 26). In addition, CMHH contains secretory proteins unique to hepatocytes such as but not limited to A2MG, A1AT, albumin, most of which are equipped with hepatocyte-protective properties.

Taken together, the hepatokines in the CMHH, which is comprised of the combination of extracellular matrixes and secretory proteins unique to HH, exhibit the specialized biological function to the in vitro cultured human hepatocytes.

### Example 5. Application of CMHH for the recovery and maintenance of primary hepatocytes.

We tested the biological effect of CMHH on the recovery and maintenance of primary human hepatocytes (PHH) in vitro. In general, the viability, platability, and longevity primary hepatocytes substantially differ lot-by-lot and donor-by-donor; thereby it hardly attains the cell seeding at the optimal cell density, which results in the loss of their genuine characteristics. Thus, PHH has been perceived as a rapidly expiring study tool with limited versatility. We tested the biological effect of CMHH on the recovery and the cell fate maintenance of cryopreserved human hepatocytes. To this end, cryopreserved HLCM-HH were thawed and cultured with either CMHH or fresh dHCGM for 7 days followed by the assessment of the cellular architecture via light microscope, the bile secretion machinery via fluorescent microscopic analysis, as well as the expression abundance of hepatocyte marker genes (Figure 27, 28, and 29). We observed the robust recovery of human hepatocytes from the stress and the injury associated with freeze-thaw processes; the incubation with CMHH supports the reestablishment of proper architectural structures, functions, and marker genes expression abundance characteristics of terminally differentiated human hepatocytes, which is a sharp contrast to that with fresh dHCGM as well as the culture medium supplied by the manufactures.

We tested the biological effect of CMHH on the recovery and the maintenance primary hepatocytes of mouse, rat, and dog in vitro. The viability, platability, and longevity primary hepatocytes of these species substantially lower than that of HLCM-HH; thereby it hardly attains the cell seeding at the optimal cell density, which results in the loss of their genuine characteristics. Similar to that with PHH, the incubation of primary mouse, rat, and dog hepatocytes with CMHH demonstrates the robust biological effect in the maintenance of the morphological appearance and functionality characteristics of primary hepatocytes (Figure 30).

The robust effect of CMHH might not be solely for the maintenance of cell fate and function of HH, and it may offer the environment necessary for the further differentiation of stem cell derived hepatocytes-like cells, namely iHeps (induced hepatocyte-like cells). Consequently, we tested the effect of CMHH on the morphological architectures. Similar to the effect on PHH and HLCM-HH, CMHH treatment promote the formation of bile canaliculi between the polygonal-cuboidal appearing human hepatocyte-like cells (Figure 31). We also assessed the effect of CMHH on the characteristics of iHeps via single-cell RNA sequencing approach (Figure 32 and 33). The principal component analysis (PCA), a classic dimension reduction approach, demonstrates a much lower dimensionality between HLCM-HH and iHep treated with CMHH than that treated with iHeps culture medium.

We also teste whether CMHH promote the re-differentiation of Clip cells. The incubation of Clip cells with CMHH promotes a significant upregulation of hepatocyte marker genes compare with that cultured with fresh dHCGM (Figure 34), indicating that CMHH has not only cell fate maintenance properties but also hepatocyte-directed differentiation properties.

To confirm the favorable effect and the robustness of CMHH on the cell fate maintenance of human hepatocytes, we conducted an in vivo study. Human hepatocytes cultured with either CMHH or dHCGM (control medium) are transplanted into the host mouse of humanized liver chimeric mice and monitor the degree of the engraftment and proliferation capacity. Both CMHH- and dHCGM-treated human hepatocytes reveal a comparable degree of engraftment according to the blood human albumin levels on day 2 post implantation (Figure 35). However, at later timepoint, CMHH-treated HH exhibit the robust proliferation capacity than dHCGM-treated HH based on the blood human albumin levels on day 14 and 21 post implantation. Since the potential to grow in the liver of HLCM host mice serves as the surrogate of primary hepatocyte quality, this in vivo study results provides an additional layer of evidence on the biological effect of CMHH.

Lastly, we also tested the utility of CMHH on the recovery and fate maintenance of cryopreserved HLCM-HH. To this end, cryopreserved HLCM-HH were thawed and seeded at the optimized condition noted in the sections above and cultured in the presence of CMHH or fresh-dHCGM. As expected, CMHH treatment greatly improved the morphology and the expression abundance of hepatocyte marker genes at levels comparable to that of terminally differentiated hepatocytes (Figure 36 and 37).

In summary, our studies indicate that CMHH represents a culture supplement that robustly supports the recovery and maintenance of primary hepatocytes. These effects are not unique to primary human hepatocytes but also HLCM-HH, primary hepatocytes of mouse, rat, and dog whether these are freshly isolated or cryopreserved. In addition, CMHH promotes the further maturation of immature hepatocytes (e.g., iHep, CLiPs), which will likely contribute to the future development of stem-cell derived hepatocyte transplantation therapy as a replacement for liver transplantation.

### ASPECTS

1. A method for preparing a conditioned medium from cultured human hepatocytes (HH), comprising:
   culturing HH in a medium to generate a culture medium, and
   harvesting the culture medium having been incubated with the HH for at least 1 hour, as the conditioned medium.
2. The method of aspect 1, wherein the HH comprise primary human hepatocytes (PHH), hepatocytes derived from a chimeric animal with humanized liver, or a combination thereof.
3. The method of aspect 2, wherein the hepatocytes derived from the chimeric animal with humanized liver are humanized liver chimeric mice derived-human hepatocytes (HLCM-HH), which are obtained from a liver derived from a mouse injected with PHH or with previously isolated HLCM-HH into the spleen.
4. The method of aspect 3, wherein the mouse has a replacement index of at least 10% before the HLCM-HH are obtained.
5. The method of aspect 1, wherein the medium is a dimethyl sulfoxide (DMSO), dimethyl sulfone (DMSO2), or tetramethylene sulfoxide (TMSO)-supplemented hepatocyte clonal growth medium (dHCGM, d2HCGM, or tHCGM), and the HCGM comprises, or consists essentially of, a standard cell culture base medium, L-proline, insulin, dexamethasone, EGF, and L-ascorbic acid 2-phosphate (Asc-2P).
6. The method of aspect 1, wherein the medium is a DMSO, DMSO2, or TMSO-supplemented hepatocyte maintenance medium (dHMM, d2HMM, tHMM), and the HMM comprises, or consists essentially of, a standard cell culture base medium, L-proline, insulin, dexamethasone, and L-ascorbic acid 2-phosphate (Asc-2P).
7. The method of aspect 5 or 6, wherein the standard cell culture base medium is selected from the group including Dulbecco's Modified Eagle's Medium (DMEM), Minimum Essential Media (MEM), RPMI-1640, Iscove's Modified Dulbecco's Medium (IMDM), or William's E Medium.
8. The method of aspect 7, wherein the standard cell culture base medium is supplemented with HEPES, penicillin-streptomycin, and serum.
9. The method of aspect 7, wherein the standard cell culture base medium is DMEM, and the DMEM is DMEM-10, and the DMEM-10 comprises, or consists essentially of, DMEM, HEPES, penicillin-streptomycin, and serum.
10. The method of any of aspects 8 or 9, wherein the serum is fetal bovine serum (FBS) or human serum.
11. The method of aspect 5, wherein dHCGM, d2HCGM, or tHCGM comprises, or consists essentially of L-proline at 15 µg/mL, insulin at 0.25 µg/mL, dexamethasone at 50 nM, EGF at 5 ng/mL, Asc-2P at 0.1 mM, DMSO, DMSO2, or TMSO at 281.6 mM, 140.8 mM, or 70.4 mM respectively, HEPES at 20 mM, penicillin at 100 IU/mL, streptomycin at 100 µg/mL, and the serum at 10% in the standard cell culture base medium.
12. The method of aspect 5, wherein dHCGM, d2HCGM, tHCGM comprises, or consists essentially of L-proline at 5-25 µg/mL, insulin at 0.1-0.5 µg/mL, dexamethasone at 10-100 nM, EGF at 1-10 ng/mL, Asc-2P at 0.01-1 mM, DMSO, DMSO2, or TMSO at 50 - 300 mM, HEPES at 10-50 mM, penicillin at 10-300 IU/mL, streptomycin at 10-300 µg/mL, and the serum at 2-20% in the standard cell culture base medium.
13. The method of aspect 6, wherein dHMM, d2HMM, tHMM comprises, or consists essentially of L-proline at 15 µg/mL, insulin at 0.25 µg/mL, dexamethasone at 50 nM, Asc-2P at 0.1 mM, DMSO, DMSO2, or TMSO at 281.6 mM, 140.8 mM, or 70.4 mM respectively, HEPES at 20 mM, penicillin at 100 IU/mL, streptomycin at 100 µg/mL, and the serum at 10% in the standard cell culture base medium.
14. The method of aspect 6, wherein dHMM, d2HMM, tHMM comprises, or consists essentially of L-proline at 5-25 µg/mL, insulin at 0.1-0.5 µg/mL, dexamethasone at 10-100 nM, Asc-2P at 0.01-1 mM, DMSO, DMSO2, TMSO at 50 - 300 mM, HEPES at 10-50 mM, penicillin at 10-300 IU/mL, streptomycin at 10-300 µg/mL, and serum at 2-20% in the standard cell culture base medium.
15. The method of any one of aspects 1-14, wherein medium is replaced with a fresh volume every 24 to 120 hours.
16. The method of aspect 2, wherein the PHH, the hepatocytes derived from the chimeric animal with humanized liver, or the combination are cultured at a cell density from 0.5×10⁵/cm² to 5×10⁵/cm².
17. The method of any of aspects 1-3, wherein the HH do not comprise HepG2 cells, Huh7 cells or murine hepatocytes.
18. The method of aspect 1, wherein the HH comprise hepatocytes derived from a chimeric animal with humanized liver, and the method further comprises obtaining the hepatocytes derived from the chimeric animal with humanized liver before the culturing step, wherein the obtaining of the hepatocytes derived from the chimeric animal with humanized liver comprises isolating hepatocytes derived from a liver of an animal injected with PHH or with previously isolated hepatocytes derived from a humanized liver chimeric animal into a spleen of the animal, thereby obtaining the hepatocytes derived from the chimeric animal with humanized liver.
19. The method of aspect 18, wherein the hepatocytes derived from the chimeric animal are isolated via collagenase perfusion of the liver of the chimeric animal, and the chimeric animal has a replacement index of at least 10% before the HH are isolated.
20. A conditioned medium from cultured human hepatocytes (CMHH), prepared by a method according to any one of aspects 1-19.
21. A CMHH, wherein the CMHH comprises a hepatocyte clonal growth medium or hepatocyte maintenance medium, wherein the medium has been used to culture primary human hepatocytes (PHH), hepatocytes derived from a chimeric animal with humanized liver, or a combination thereof for at least 1 hour.
22. A CMHH of aspect 21, wherein the CMHH comprises one or more humoral factors secreted by human hepatocytes and a hepatocyte clonal growth medium or a hepatocyte maintenance medium.
23. The CMHH of any of aspects 20-22, wherein the hepatocyte clonal growth medium comprises a standard cell culture base medium supplemented with L-proline, insulin, dexamethasone, EGF, Asc-2P, HEPES, penicillin, streptomycin, serum, and DMSO, DMSO2, or TMSO.
24. The CMHH of any of aspects 20-22, wherein the hepatocyte maintenance medium comprises a standard cell culture base medium supplemented with L-proline, insulin, dexamethasone, Asc-2P, HEPES, penicillin, streptomycin, serum, and DMSO, DMSO2, or TMSO.
25. A combination, comprising a CMHH of any one of aspects 20-24 and extracellular matrices.
26. A combination, comprising a CMHH of any one of aspects 20-24 and five chemicals of forskolin, SB431542, DAPT, IWP2, and LDN193189.
27. A CMHH of any one of aspects 20-24, for use in cultivating freshly isolated terminally differentiated HH, PHH, hepatocytes derived from a chimeric animal with humanized liver, or primary hepatocytes from other species of animals.
28. A CMHH of any of aspects 20-24, for use in cultivating cryopreserved terminally differentiated HH, PHH, hepatocytes derived from a chimeric animal with humanized liver, or primary hepatocytes from other species of animals.
29. A CMHH of any of aspects 20-24, for use in differentiating hepatic bipotential progenitor cells (iHep) and/or chemically induced liver progenitors into terminally differentiated hepatocytes, hepatoma cells such as HeparRG^{™}, or matured hepatocytes, respectively.
30. A CMHH of any of aspects 20-24, for use in co-culturing with nonparenchymal cells.
31. A CMHH of any of aspects 20-24, for use in suspending HH in cryopreservation.
32. A method of culturing HH for recovery after isolation and/or preservation and establishment of hepatic function, the method comprising:
   culturing a quantity of HH in a first phase with a first cell culture medium for a first period of time,
   removing the first cell culture medium from the quantity of HH, followed by
   culturing the quantity of HH in a second phase with a second cell culture medium for a second period of time,
   wherein the first cell culture medium comprises a DMSO-supplemented cell culture medium, and the second cell culture medium comprises a DMSO, DMSO2, or TMSO-supplemented cell culture medium.
33. A method of culturing HH for recovery after isolation and/or preservation and establishment of hepatic function, the method comprising:
   culturing a quantity of HH in a first phase with a first cell culture medium for a first period of time,
   removing the first cell culture medium from the quantity of HH, followed by
   culturing the quantity of HH in a second phase with a second cell culture medium for a second period of time,
   wherein the first cell culture medium comprises a dimethyl sulfoxide(DMSO)-supplemented cell culture medium, the second cell culture medium comprises a DMSO2 or TMSO-supplemented cell culture medium, and
   wherein the second cell culture medium does not comprise DMSO.
34. The method of aspect 32 or 33, wherein the first period of time is at least 4 days and up to 2 months, and wherein the quantity of HH cultured in the first phase establishes cellular polarity, inter-cellular structure characterized by bile canaliculi, and/or a gene expression level equivalent to control HH exhibiting hepatic function of matured hepatocytes or the liver tissue of healthy individuals.
35. The method of aspect 32 or 33, wherein the first period of time is about 7 days, and the first (recovery) phase cell culture medium is optionally refreshed every 3 or 4 days.
36. The method of aspect 32 or 33, wherein the quantity of HH cultured in the second phase has a physiological level of expression of hepatocyte metabolic genes such as, but not limited to, alcohol dehydrogenases (ADHs), aldehyde dehydrogenases (ALDHs), cytochrome P450 family 3 subfamily A member 4 (CYP3A4) and cytochrome P450 family 2 subfamily E member 1 (CYP2E1); or the quantity of HH cultured in the second phase metabolizes alcohol, xenobiotics, vitamin A, or a combination thereof.
37. The method of any one of aspects 32-36, wherein the first cell culture medium is a DMSO-supplemented hepatocyte culture medium or CMHH, which includes DMSO, a standard cell culture base medium, and one or more of L-proline, insulin, dexamethasone, EGF, Asc-2P, HEPES, penicillin-streptomycin, human serum and/or FBS.
38. The method of any of aspects 32-36, wherein the second cell culture medium is a DMSO, DMSO2, or TMSO-supplemented hepatocyte culture medium or CMHH, which includes DMSO, DMSO2, or TMSO, a standard cell culture base medium, and one or more of L-proline, insulin, dexamethasone, EGF, Asc-2P, HEPES, penicillin-streptomycin, human serum and/or heat-inactivated FBS.
39. The method of any of aspects 32-38, wherein the first or second cell culture medium contains the DMSO at a final concentration of about 2% v/v or 281.6 mM.
40. The method of any of aspects 32-38, wherein the second cell culture medium contains the DMSO2 at a final concentration of about 140.8 mM.
41. The method of any of aspects 32-38, wherein the second cell culture medium contains the TMSO at a final concentration of about 70 mM
42. The method of any of aspects 32-41, wherein the first cell culture medium comprises the DMSO at 2 (v/v) % or 286.1 mM, the L-proline at 15 µg/mL, the insulin at 0.25 µg/mL, the dexamethasone at 50 nM, the EGF at 5 ng/mL, the Asc-2P at 0.1 mM, the HEPES at 20 mM, the penicillin at 100 IU/mL, the streptomycin at 100 µg/mL, and the serum at 10% in a standard cell culture base medium; and
   wherein the second cell culture medium comprises the DMSO, DMSO2, or TMSO at 281.6 mM, 140.8 mM, or 70.4 mM, respectively, the L-proline at 15 µg/mL, the insulin at 0.25 µg/mL, the dexamethasone at 50 nM, the EGF at 5 ng/mL, the Asc-2P at 0.1 mM, the HEPES at 20 mM, the penicillin at 100 IU/mL, the streptomycin at 100 µg/mL, and the serum at 10% in a standard cell culture base medium.
43. The method of any of aspects 32-41, wherein the first cell culture medium comprises the DMSO at 2 (v/v) % or 286.1 mM, the L-proline at 15 µg/mL, the insulin at 0.25 µg/mL, the dexamethasone at 50 nM, the Asc-2P at 0.1 mM, the HEPES at 20 mM, the penicillin at 100 IU/mL, the streptomycin at 100 µg/mL, and the serum at 10% in a standard cell culture base medium; and
   wherein the second cell culture medium comprises the DMSO, DMSO2, or TMSO at 281.6 mM, 140.8 mM, or 70.4 mM, respectively, the L-proline at 15 µg/mL, the insulin at 0.25 µg/mL, the dexamethasone at 50 nM, the Asc-2P at 0.1 mM, the HEPES at 20 mM, the penicillin at 100 IU/mL, the streptomycin at 100 µg/mL, and the serum at 10% in a standard cell culture base medium.
44. The method of any of aspects 32-41, wherein the first cell culture medium comprises the DMSO at 0.5%-5% (v/v), the L-proline at 5-25 µg/mL, the insulin at 0.1-0.5 µg/mL, the dexamethasone at 10-100 nM, the EGF at 0-10 ng/mL, the Asc-2P at 0.01-1 mM, the HEPES at 10-50 mM, the penicillin at 10-300 IU/mL, the streptomycin at 10-300 µg/mL, and the serum at 2-20% in a standard cell culture medium; and
   wherein the second cell culture medium comprises the DMSO, DMSO2, or TMSO, at 50-300 mM, the L-proline at 5-25 µg/mL, the insulin at 0.1-0.5 µg/mL, the dexamethasone at 10-100 nM, the EGF at 0-10 ng/mL, the Asc-2P at 0.01-1 mM, the HEPES at 10-50 mM, the penicillin at 10-300 IU/mL, the streptomycin at 10-300 µg/mL, and the serum at 2-20% in a standard cell culture base medium.
45. The method of any of aspects 32-44, for use in assaying performance of a candidate agent in promoting alcohol metabolism by HH or toxicity of the candidate agent to HH, by contacting the quantity of HH cultured in the second phase in the second cell culture medium with the candidate agent, measuring a first level of metabolizing an alcohol or excretion of the candidate agent by the quantity of HH before the contact with the candidate agent, and measuring a second level of metabolizing the alcohol or excretion of the candidate agent by the quantity of HH in the presence of or after the contact with the candidate agent.
46. A HH culture medium for establishment and maintenance of hepatic functions of HH, comprising a DMSO2 or TMSO-supplemented cell culture medium, which does not include DMSO.
47. The HH culture medium of aspect 46, wherein the DMSO2 or TMSO is supplemented to reach a concentration of about 140.8 mM or 70.4 mM, respectively, in a standard cell culture base medium, wherein the base medium further includes one or more of L-proline, insulin, dexamethasone, EGF, Asc-2P, HEPES, penicillin-streptomycin, human serum, and/or heat-inactivated FBS.
48. The HH culture medium of aspect 46 or 47, comprising the DMSO2 at 140.8 mM, the L-proline at 15 µg/mL, the insulin at 0.25 µg/mL, the dexamethasone at 50 nM, the EGF at 5 ng/mL, the Asc-2P at 0.1 mM, the HEPES at 20 mM, the penicillin at 100 IU/mL, the streptomycin at 100 µg/mL, and the serum at 10% in a standard cell culture base medium.
49. The HH culture medium of aspect 46 or 47, comprising the TMSO at 70.4 mM, the L-proline at 15 µg/mL, the insulin at 0.25 µg/mL, the dexamethasone at 50 nM, the EGF at 5 ng/mL, the Asc-2P at 0.1 mM, the HEPES at 20 mM, the penicillin at 100 IU/mL, the streptomycin at 100 µg/mL, and the serum 10% in a standard cell culture base medium.
50. The HH culture medium of any of aspects 46-49, comprising the DMSO2 or TMSO at 50-200 mM, the L-proline at 5-25 µg/mL, the insulin at 0.1-0.5 µg/mL, the dexamethasone at 10-100 nM, the EGF at 1-10 ng/mL, the Asc-2P at 0.01-1 mM, the HEPES at 10-50 mM, the penicillin at 10-300 IU/mL, the streptomycin at 10-300 µg/mL, and serum at 2-20% in a standard cell culture base medium.
51. A kit for culturing HH, comprising:
   a first container storing a first cell culture medium, said first cell culture medium is supplemented with DMSO; and
   a second container storing a second cell culture medium, said second cell culture medium is supplemented with DMSO, DMSO2, and/or TMSO.
52. A kit for culturing human hepatocytes, comprising:
   a first container storing a first cell culture medium, said first cell culture medium is supplemented with DMSO; and
   a second container storing a second cell culture medium, said second cell culture medium is supplemented with DMSO2 and/or TMSO, without DMSO.
53. A method of assaying toxicity of an agent to HH or drug metabolism and pharmacokinetics (DMPK) of the agent, comprising:
   culturing a quantity of HH in a medium supplemented with DMSO2, without DMSO;
   contacting the agent with the quantity of HH cultured in the DMSO2-supplemented medium; and
   measuring a level of toxicity to the quantity of HH in the presence of, or after the contact with, the agent, or measuring a level of metabolism or excretion of the agent by the quantity of HH in the presence of, or after the contact with, the agent.
54. The method of aspect 53, wherein the agent is an alcohol compound comprising ethanol, methanol, ethylene glycol, isopropanol, or a mixture thereof.
55. The method of aspect 53 or 54, wherein the contacting and the measuring are performed in a tight-sealing apparatus.
56. The method of any of aspects 53-55, wherein the level of toxicity is measured via quantifying expression or function of xenobiotics-metabolizing enzymes such as, but not limited to Cytochrome P450 (CYP) enzymes in the quantity of HH.
57. The method of any of aspects 53-55, wherein the level of toxicity is measured via quantifying the toxic metabolites of alcohol such as acetaldehyde, or reduction of glutathione, the activation status of cell death pathways in the quantity of HH.
58. A method of assaying a candidate agent for promoting the metabolism of alcohol or vitamin A, comprising:
   culturing a quantity of HH in a medium supplemented with DMSO2, without DMSO;
   contacting the quantity of HH cultured in the DMSO2-supplemented medium with the alcohol or the vitamin A in the presence of a candidate agent; and
   measuring a level of metabolism of the alcohol or the vitamin A by the quantity of HH in the presence of, or after the contact with, the candidate agent.
59. The method of any of aspects 32-58, wherein the medium used is a conditioned medium from HH from any of aspects 20-24.

## Claims

1. A method for culturing, differentiating, or suspending hepatocytes, iPS cell-derived hepatocyte-like cells (iHep), or chemically induced liver progenitor (CLiP) cells for recovery after isolation and/or preservation and for establishing liver function, the method comprising:
culturing a quantity of hepatocytes, iHep, or CLiP cells in a first phase with a first cell culture medium for a first period of time, and
removing the first cell culture medium from the quantity of hepatocytes, iHep, or CLiP cells, followed by culturing the quantity of hepatocytes, iHep, or CLiP cells in a second phase with a second cell culture medium for a second period of time,
wherein the first cell culture medium comprises a DMSO-supplemented cell culture medium, and the second cell culture medium comprises a DMSO, DMSO2, or TMSO-supplemented cell culture medium.

2. The method according to claim 1, wherein the first period of time is at least 4 days and up to 2 months, and wherein the quantity of hepatocytes, iHep, or CLiP cells cultured in the first phase establishes cellular polarity, inter-cellular structure **characterized by** bile canaliculi, and/or a gene expression level equivalent to control hepatocytes, iHep, or CLiP cells exhibiting hepatic function of matured hepatocytes or the liver tissue of healthy individuals.

3. The method according to claim 1 or 2, wherein the first period of time is about 7 days, and the first (recovery) phase cell culture medium is optionally refreshed every 3 or 4 days.

4. The method according to any one of the preceding claims, wherein the quantity of hepatocytes, iHep, or CLiP cells cultured in the second phase has a physiological level of expression of hepatocyte metabolic genes such as, but not limited to, alcohol dehydrogenases (ADHs), aldehyde dehydrogenases (ALDHs), cytochrome P450 family 3 subfamily A member 4 (CYP3A4) and cytochrome P450 family 2 subfamily E member 1 (CYP2E1); or the quantity of hepatocytes, iHep, or CLiP cells cultured in the second phase metabolizes alcohol, xenobiotics, vitamin A,
or a combination thereof.

5. The method according to any one of the preceding claims, wherein the first cell culture medium is a DMSO-supplemented hepatocyte culture medium or conditioned medium from cultured human hepatocytes (CMHH), which includes DMSO, a standard cell culture base medium, and one or more of L-proline, insulin, dexamethasone, EGF, Asc-2P, HEPES, penicillin-streptomycin, human serum and/or FBS.

6. The method according to any one of the preceding claims, wherein the second cell culture medium is a DMSO, DMSO2, or TMSO-supplemented hepatocyte culture medium or CMHH, which includes DMSO, DMSO2, or TMSO, a standard cell culture base medium, and one or more of L-proline, insulin, dexamethasone, EGF, Asc-2P, HEPES, penicillin- streptomycin, human serum and/or heat-inactivated FBS, optionally wherein:
a) the first or second cell culture medium contains the DMSO at a final concentration of about 2% v/v or 281.6 mM;
the second cell culture medium contains the DMSO2 at a final concentration of about 140.8 mM; or
the second cell culture medium contains the TMSO at a final concentration of about 70 mM;
b) the first cell culture medium comprises the DMSO at 0.5%-5% (v/v), the L-proline at 5-25 µg/mL, the insulin at 0.1-0.5 µg/mL, the dexamethasone at 10-100 nM, the EGF at 0-10 ng/mL, the Asc-2P at 0.01-1 mM, the HEPES at 10-50 mM, the penicillin at 10-300 IU/mL, the streptomycin at 10-300 µg/mL, and the serum at 2-20% in a standard cell culture medium; and
the second cell culture medium comprises the DMSO, DMSO2, or TMSO, at 50-300 mM, the L-proline at 5-25 µg/mL, the insulin at 0.1-0.5 µg/mL, the dexamethasone at 10-100 nM, the EGF at 0-10 ng/mL, the Asc-2P at 0.01-1 mM, the HEPES at 10-50 mM, the penicillin at 10-300 IU/mL, the streptomycin at 10-300 µg/mL, and the serum at 2-20% in a standard cell culture base medium;
c) the first cell culture medium comprises the DMSO at 2 (v/v) % or 286.1 mM, the L-proline at 15 µg/mL, the insulin at 0.25 µg/mL, the dexamethasone at 50 nM, the EGF at 5 ng/mL, the Asc-2P at 0.1 mM, the HEPES at 20 mM, the penicillin at 100 IU/mL, the streptomycin at 100 µg/mL, and the serum at 10% in a standard cell culture base medium; and
the second cell culture medium comprises the DMSO, DMSO2, or TMSO at 281.6 mM, 140.8 mM, or 70.4 mM, respectively, the L-proline at 15 µg/mL, the insulin at 0.25 µg/mL, the dexamethasone at 50 nM, the EGF at 5 ng/mL, the Asc-2P at 0.1 mM, the HEPES at 20 mM, the penicillin at 100 IU/mL, the streptomycin at 100 µg/mL, and the serum at 10% in a standard cell culture base medium; or
d) the first cell culture medium comprises the DMSO at 2 (v/v) % or 286.1 mM, the L-proline at 15 µg/mL, the insulin at 0.25 µg/mL, the dexamethasone at 50 nM, the Asc-2P at 0.1 mM, the HEPES at 20 mM, the penicillin at 100 IU/mL, the streptomycin at 100 µg/mL, and the serum at 10% in a standard cell culture base medium; and
the second cell culture medium comprises the DMSO, DMSO2, or TMSO at 281.6 mM, 140.8 mM, or 70.4 mM, respectively, the L-proline at 15 µg/mL, the insulin at 0.25 µg/mL, the dexamethasone at 50 nM, the Asc-2P at 0.1 mM, the HEPES at 20 mM, the penicillin at 100 IU/mL, the streptomycin at 100 µg/mL, and the serum at 10% in a standard cell culture base medium.

7. The method according to any one of the preceding claims, wherein the method further comprises assaying performance of a candidate agent in promoting alcohol metabolism by hepatocytes, iHep, or CLiP cells or toxicity of the candidate agent to hepatocytes, iHep, or CLiP cells, by contacting the quantity of hepatocytes, iHep, or CLiP cells cultured in the second phase in the second cell culture medium with the candidate agent, measuring a first level of metabolizing an alcohol or excretion of the candidate agent by the quantity of hepatocytes, iHep, or CLiP cells before the contact with the candidate agent, and measuring a second level of metabolizing the alcohol or excretion of the candidate agent by the quantity of hepatocytes, iHep, or CLiP cells in the presence of or after the contact with the candidate agent.

8. A kit for culturing hepatocytes, iHep, or CLiP cells, comprising:
a first container storing a first cell culture medium, said first cell culture medium is supplemented with DMSO; and
a second container storing a second cell culture medium, said second cell culture medium is supplemented with DMSO, DMSO2, and/or TMSO.

9. The kit according to claim 8, wherein the hepatocytes are human hepatocytes.

10. A method of assaying toxicity of an agent to hepatocytes, iHep, or CLiP cells or drug metabolism and pharmacokinetics (DMPK) of the agent, comprising:
culturing a quantity of hepatocytes, iHep, or CLiP cells by the method according to claim 1;
contacting the agent with the quantity of hepatocytes, iHep, or CLiP cells cultured in the second cell culture medium; and
measuring a level of toxicity to the quantity of hepatocytes, iHep, or CLiP cells in the presence of, or after the contact with, the agent, or measuring a level of metabolism or excretion of the agent by the quantity of hepatocytes, iHep, or CLiP cells in the presence of, or after the contact with, the agent.

11. The method according to claim 10, wherein the agent is an alcohol compound comprising ethanol, methanol, ethylene glycol, isopropanol, or a mixture thereof.

12. The method according to claim 10 or 11, wherein the contacting and the measuring are performed in a tight-sealing apparatus.

13. The method according to any one of claims 10-12, wherein:
a) the level of toxicity is measured via quantifying expression or function of xenobiotics-metabolizing enzymes such as, but not limited to Cytochrome P450 (CYP) enzymes in the quantity of hepatocytes, iHep, or CLiP cells; or
b) the level of toxicity is measured via quantifying the toxic metabolites of alcohol such as acetaldehyde, or reduction of glutathione, the activation status of cell death pathways in the quantity of hepatocytes, iHep, or CLiP cells.

14. A method of assaying a candidate agent for promoting the metabolism of alcohol or vitamin A, comprising:
culturing a quantity of hepatocytes, iHep, or CLiP cells by the method according to claim 1;
contacting hepatocytes, iHep, or CLiP cells cultured in the second cell culture medium with the alcohol or the vitamin A in the presence of a candidate agent; and
measuring a level of metabolism of the alcohol or the vitamin A by the quantity of hepatocytes, iHep, or CLiP cells in the presence of, or after the contact with, the candidate agent.

15. The method according to any one of claims 10-14, wherein the medium used is a conditioned medium from human hepatocytes (CMHH).
